(19) Europäisches Patentamt
European Patent Office
Office européen des brevets

(11) Veröffentlichungsnummer: **0 012 170**
**B1**

(12) # EUROPÄISCHE PATENTSCHRIFT

(45) Veröffentlichungstag der Patentschrift: **21.04.82**

(51) Int. Cl.³: **C 07 D 455/02,** A 61 K 31/435 // (C07D455/02, 221/00, 235/00)

(21) Anmeldenummer: **79103907.6**

(22) Anmeldetag: **11.10.79**

(54) **Neue 7-Phenyl-chinolizidine (I), ihre Herstellung, sie enthaltende Arzneimittel und (I) zur Verwendung bei der Bekämpfung von Krankheiten.**

(30) Priorität: **13.10.78 CH 10654/78**
**03.08.79 CH 7156/79**

(43) Veröffentlichungstag der Anmeldung: **25.06.80 Patentblatt 80/13**

(45) Bekanntmachung des Hinweises auf die Patenterteilung: **21.04.82 Patentblatt 82/16**

(84) Benannte Vertragsstaaten: **AT BE CH DE FR GB IT LU NL SE**

(56) Entgegenhaltungen:
**US-A-3 429 884**
**Chemical Abstracts, vol. 62, no. 3, April 12—May 10, 1965, Columbus Ohio, USA F. Bohlmann et al.: »Dehydrogenation of phenyl-substituted quinolizidines«, column 9103e**
**Chemical Abstracts, vol. 64, no. 4, May 9—May 23, 1966, Columbus Ohio, USA J. Wrobel et al.: »New synthesis of quinolizidine compounds«, column 15936g**

(73) Patentinhaber: **F. HOFFMANN-LA ROCHE & CO. Aktiengesellschaft, CH-4002 Basel (CH)**

(72) Erfinder: **Imhof, René, Dr., In der Kehr, CH-5265 Wittnau (CH)**
Erfinder: **Kyburz, Emilio, Dr., Unterer Rebbergweg 127, CH-4153 Reinach (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 012 170 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Neue 7-Phenyl-chinolizidine (I), ihre Herstellung, sie enthaltende Arzneimittel und (I) zur Verwendung bei der Bekämpfung von Krankheiten

Die vorliegende Erfindung betrifft neue pharmakologisch aktive Phenyl-chinolizidine, ihre Herstellung und Verwendung und Arzneimittel enthaltend solche Chinolizidine.

Die erfindungsgemäßen Phenyl-chinolizidine sind Verbindungen der allgemeinen Formel

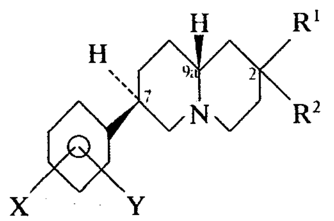

(I)

worin X, Y, $R^1$ und $R^2$ folgendes bedeuten:

X: Wasserstoff, Fluor, Chlor, $C_{1-6}$-Alkoxy, $C_{1-6}$-Alkyl oder Trifluormethyl,
Y: Wasserstoff, Fluor, Chlor, $C_{1-6}$-Alkoxy oder $C_{1-6}$-Alkyl, vorzugsweise Wasserstoff,
R: Hydroxy, $C_{1-6}$-Alkoxy, Acyloxy oder, falls $R^2$ die nachstehend definierte Gruppe A darstellt, Wasserstoff,
$R^2$: $C_{1-6}$-Alkyl, gegebenenfalls durch Halogen, $C_{1-6}$-Alkoxy oder Trifluormethyl substituiertes Phenyl, oder (falls $R^1$ = Wasserstoff) eine Gruppe A mit der Bedeutung

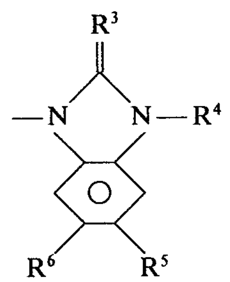

oder

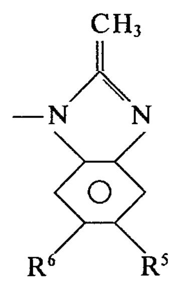

A

mit folgender Bedeutung für $R^3 - R^6$:

$R^3$: Sauerstoff oder Schwefel,
$R^4$: Wasserstoff oder $C_{1-6}$-Alkyl,
$R^5$ und $R^6$ unabhängig voneinander: Wasserstoff, Methyl, Fluor, Chlor, Methoxy, Trifluormethyl oder zusammen $-(CH_2)_4$,

in Form des Racemats oder der Enantiomeren, sowie in Form der freien Basen wie von Säureadditionssalzen.

Wie in der Formel I und in verschiedenen der nachfolgenden Formeln angegeben, befinden sich die Wasserstoffatome in den Stellungen 7 und 9a transständig zueinander, wobei in den Formeln das 9a-Wasserstoffatom willkürlich in $\beta$-Stellung und das 7-Wasserstoffatom dementsprechend in $\alpha$-Stellung angegeben ist. Die erfindungsgemäßen Chinolizidine wie auch die entsprechenden Zwischenprodukte und Ausgangsstoffe sind jedoch nicht auf diese absolute Konfiguration beschränkt, sondern umfassen auch die entsprechenden enantiomeren Formen, also Verbindungen mit Wasserstoff in 9a$\alpha$ und 7$\beta$, sowie die Racemate dieser beiden enantiomeren Formen. Dasselbe gilt mit Bezug auf die Konfiguration in 2-Stellung des Chinolizidins. Sofern, wie beispielsweise in der nachfolgenden Formel III die 2- und 9a-Wasserstoffatome in $\beta$- Stellung und das 7-Wasserstoffatom in $\alpha$-Stellung angegeben ist, so soll damit lediglich die relative Konfiguration an diesen 3 Asymmetriezentren festgelegt werden (9aH zu 7H: trans, 9aH zu 2H: cis), jedoch keine Beschränkung auf das eine Enantiomere ausgesprochen sein.

Unter $C_{1-6}$-Alkyl- bzw. $C_{1-6}$-Alkoxygruppen sind im vorliegenden Rahmen solche Gruppen zu verstehen, die 1–6, insbesondere 1–4 Atome enthalten, wobei die Gruppen mit 3 und mehr C-Atomen geradkettig oder verzweigt sein können. Die als möglicher $R^1$-Substituent genannte Acyloxy-Gruppe kann sich von organischen, gesättigten, gegebenenfalls substituierten Carbonsäuren, insbesondere niedern, gegebenenfalls halogensubstituierten Alkancarbonsäuren, wie der Essigsäure oder der Trifluoressigsäure, ableiten.

Als Säureadditionssalze der erfindungsgemäßen Phenylchinolizidine kommen die pharmakologisch verträglichen Salze mit den üblicherweise zur Salzbildung verwendeten organischen und anorganischen Säuren in Betracht. Beispiele solcher Säuren sind: Mineralsäuren, wie Schwefelsäure,

Salpetersäure, Phosphorsäure, Halogenwasserstoffsäuren (z. B. Salzsäure, Bromwasserstoffsäure); organische Säuren, wie Weinsäure, Citronensäure, aliphatische oder aromatische Sulfonsäuren, Maleinsäure, Mandelsäure etc. Die Salzbildung kann auf an sich bekannte Art erfolgen.

Die Formel I umfaßt folgende Untergruppen:

a) die Alkyl- resp. Aryl-carbinole der Formel

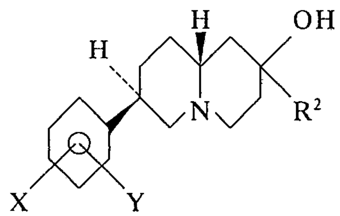

(II)

bevorzugt diejenigen der Formel

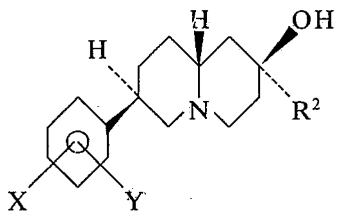

(II-1)

wobei X, Y und $R^2$ dasselbe wie oben bedeuten,

in racemischer Form und in Form der Enantiomeren, sowie gegebenenfalls in Form von Säureadditionssalzen,

b) die Ester und Äther der sub a) genannten Carbinole, d. h. die Verbindungen der Formel

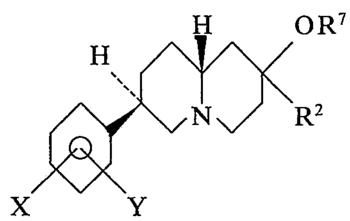

(IV)

worin X, Y und $R^2$ dasselbe wie oben bedeuten und $R^7$ $C_{1-6}$-Alkyl oder Acyl darstellt,

in racemischer Form und in Form der Enantiomeren, sowie gegebenenfalls in Form von Säureadditionssalzen,

c) die Benzimidazolinon(und -thion)-Derivate der Formel

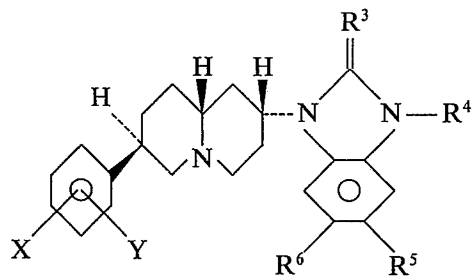

(III-1)

worin X, y sowie $R^3-R^6$ dasselbe wie oben bedeuten,

in racemischer Form und in Form der Enantiomeren, sowie gegebenenfalls in Form von Säureadditionssalzen.

d) die Methylbenzimidazol-Derivate der Formel

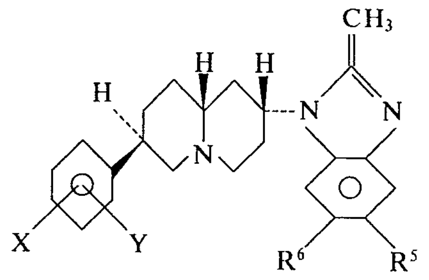

(III-2)

mit obiger Bedeutung für X, Y, $R^5$ und $R^6$

in racemischer Form und in Form der Enantiomeren, sowie gegebenenfalls in Form von Säureadditionssalzen.

Bevorzugte Verbindungen der Formel II-1 sind diejenigen, in denen $R^2$ verzweigtes $C_{1-6}$-Alkyl, X Fluor oder Chlor und Y Wasserstoff, Fluor oder Chlor, wobei X und Y in ortho- und/oder para-Stellung stehen, dies vorwiegend wegen ihrer neuroleptischen Wirkung. Besonders bevorzugte Verbindungen der Formel II-1 sind diejenigen, in denen $R^2$ tert. Butyl, X o-Chlor und Y p-Chlor oder $R^2$ tert. Butyl, X p-Trifluormethyl und Y Wasserstoff bedeuten.

Die Verbindungen der Formel IV sind vorwiegend wegen ihrer analgetischen Wirkung bevorzugt. Darunter besonders bevorzugt sind diejenigen, in denen

a) X und Y Wasserstoff, $OR^7$ gleich wie der 9a-Wasserstoff orientiertes Acetoxy und $R^2$ n-Butyl oder
b) X und Y Wasserstoff, $OR^7$ gleich wie der 9a-Wasserstoff orientiertes Trifluoracetoxy und $R^2$ tert. Butyl oder
c) X o-Chlor, $R^2$ wie der 9a-Wasserstoff orientiertes Phenyl und $OR^7$ Acetoxy bedeuten.

Die Verbindungen der Formeln III-1 und III-2 sind vorwiegend neuroleptisch wirksam. Bevorzugt sind die Benzimidozolinon (bzw. -thion)-Derivate der Formel III-1, in denen X Wasserstoff, o-Fluor oder o-Chlor, Y Wasserstoff, $R^3$ Sauerstoff oder Schwefel, $R^4$ Wasserstoff, $R^5$ Fluor, Chlor oder Trifluormethyl und $R^6$ Wasserstoff, Chlor oder Fluor darstellen. Ferner sind die folgenden Verbindungen der Formel III-1 besonders bevorzugt:

a) $X=o\text{-}Cl$; Y, $R^4$, $R^5$, $R^6=H$; $R^3=O$
b) $X=o\text{-}Cl$; Y, $R^4=H$; $R^3=O$; $R^5$, $R^6=CH_3$
c) $X=o\text{-}Cl$; Y, $R^4=H$; $R^3=O$; $R^5$, $R^6=Cl$
d) $X=o\text{-}Cl$; Y, $R^5$, $R^6=H$; $R^3=O$; $R^4=CH_3$
e) $X=o\text{-}Cl$; Y, $R^4$, $R^6=H$; $R^5=CF_3$; $R^3=O$
f) $X=o\text{-}Cl$; Y, $R^4$, $R^6=H$; $R^5=Cl$; $R^3=O$
g) $X=o\text{-}Cl$; Y, $R^4$, $R^6=H$; $R^5=Cl$; $R^3=S$
h) $X=o\text{-}Cl$; Y, $R^4=H$; $R^3=S$; $R^5$, $R^6=Cl$
i) $X=o\text{-}Cl$; Y, $R^4=H$; $R^3=O$; $R^5$ und $R^6$ zusammen $-(CH_2)_4-$
j) $X=o\text{-}F$; Y, $R^4=H$; $R^3=O$; $R^5$, $R^6=CH_3$
k) $X=o\text{-}F$; Y, $R^4=H$; $R^3=O$; $R^5$, $R^6=Cl$
l) $X=o\text{-}F$; Y, $R^4=H$; $R^3=S$; $R^5$, $R^6=Cl$
m) $X=o\text{-}F$; Y, $R^4=H$; $R^3=O$; $R^5$ und $R^6$ zusammen $-(CH_2)_4-$
n) $X=o\text{-}F$; Y, $R^4$, $R^6=H$; $R^5=CF_3$; $R^3=O$
o) $X=o\text{-}F$; Y, $R^4$, $R^6=H$; $R^5=CF_3$; $R^3=S$
p) $X=o\text{-}F$; Y, $R^4$, $R^6=H$; $R^5=Cl$; $R^3=O$
q) $X=o\text{-}F$; Y, $R^4$, $R^6=H$; $R^5=Cl$; $R^3=S$.

Die erfindungsgemäßen Phenyl-chinolizidine der Formel I mit den oben angegebenen Bedeutungen für X, Y, $R^1$ und $R^2$ können dadurch hergestellt werden, daß man

1. zur Herstellung von Verbindungen der allgemeinen Formel

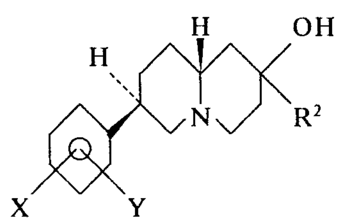

(II)

worin X, Y und $R^2$ dasselbe wie oben bedeuten,
entweder
a) ein Keton der allgemeinen Formel V

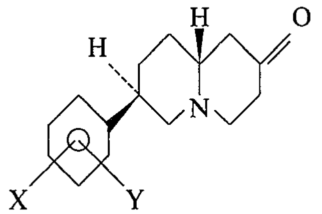

(V)

worin X und Y dasselbe wie oben bedeuten,

mit einer metallorganischen, den Rest $R^2$ liefernden Verbindung umsetzt, und gegebenenfalls das erhaltene Gemisch der Diastereomeren auftrennt, oder
b) an die $\Delta^1$- bzw. $\Delta^2$-Doppelbindung einer Verbindung der allgemeinen Formel

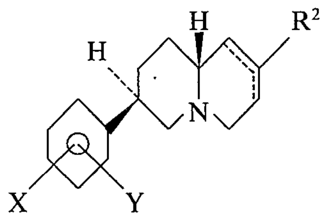

(VI)

worin X, Y und $R^2$ dasselbe wie oben bedeuten und die gestrichelte Bindung eine Doppelbindung in 1,2- oder 2,3-Stellung darstellt,

durch Umsetzung mit einem Hydratisierungsmittel Wasser anlagert,

oder daß man
2. zur Herstellung von Verbindungen der allgemeinen Formel

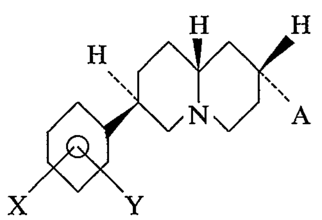

(III)

worin X, Y und A dasselbe wie oben bedeuten,

eine Verbindung der allgemeinen Formel

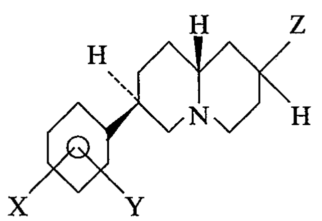

(VII)

worin X und Y dasselbe wie oben bedeuten und Z eine abspaltbare Gruppe darstellt,

mit einer Verbindung der allgemeinen Formel

$$H—A \qquad (VIII)$$

worin A dasselbe wie oben bedeutet,

umsetzt, oder daß man

3. zur Herstellung einer Verbindung der allgemeinen Formel

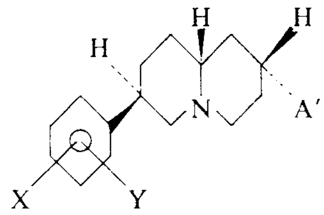

(III-3)

worin X und Y dasselbe wie oben bedeuten und A' wie A ist, wobei jedoch $R^3$ Sauerstoff darstellt,

eine Verbindung der allgemeinen Formel

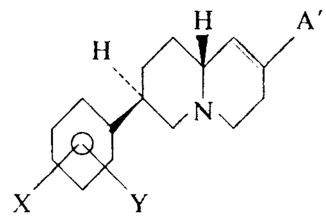

(IX)

worin X, Y und A' dasselbe wie oben bedeuten,

katalytisch hydriert, oder daß man

4. zur Herstellung einer Verbindung der allgemeinen Formel

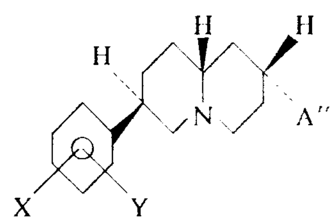

(III-4)

worin X, Y dasselbe wie oben bedeuten und A" wie A ist, wobei jedoch $R^3$ Sauerstoff und $R^4$ Wasserstoff darstellt,

ein Phenylen-diamin der allgemeinen Formel

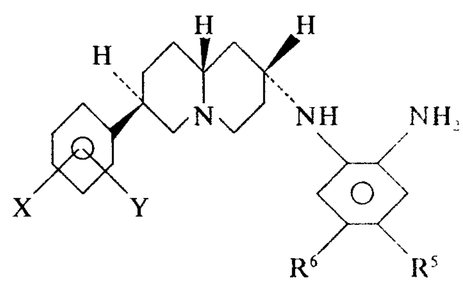

(X)

worin X, Y, $R^5$ und $R^6$ dasselbe wie oben bedeuten,

mit einem Cyclisierungsmittel der allgemeinen Formel

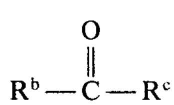

(XI)

worin $R^b$ und $R^c$ Halogen, Amino oder 1-Imidazolyl bedeuten,

oder mit Essigsäure oder einem Orthoessigsäureester kondensiert, oder daß man

5. zur Herstellung von Verbindungen der allgemeinen Formel

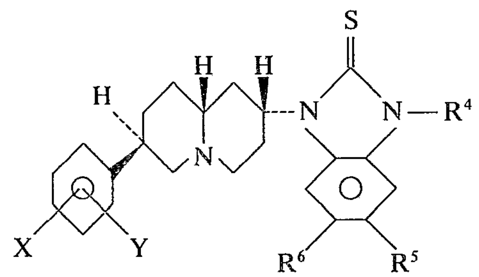

(III-5)

worin X, Y sowie $R^4-R^6$ dasselbe wie oben bedeuten,

entweder

a) eine entsprechende Oxo-verbindung der allgemeinen Formel

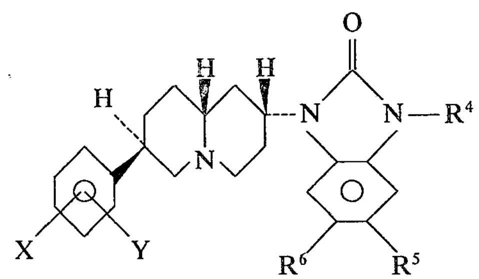

(III-6)

worin X, Y sowie $R^4-R^6$ dasselbe wie oben bedeuten,

mit $P_2S_5$ umsetzt, oder

b) ein Phenylen-diamin der obigen allgemeinen Formel X mit Thiophosgen, Thiocarbonyldimidazol oder $CS_2$ umsetzt und anschließend gegebenenfalls N-alkyliert,

oder daß man

6. zur Herstellung von Verbindungen der allgemeinen Formel

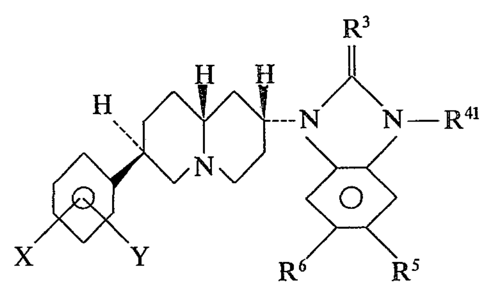

(III-7)

worin X, Y, $R^3$, $R^5$ und $R^6$ dasselbe wie oben bedeuten und $R^{41}$ $C_{1-6}$-Alkyl darstellt,

eine Verbindung der allgemeinen Formel

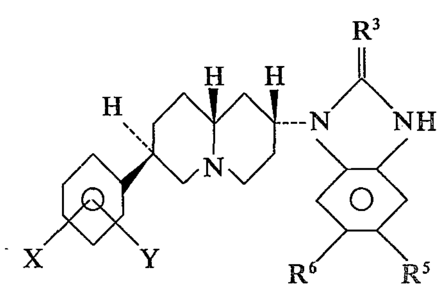

(III-8)

worin X, Y, $R^3$, $R^5$ und $R^6$ dasselbe wie oben bedeuten,

N-alkyliert, oder daß man

7. zur Herstellung von Äthern und Estern der allgemeinen Formel

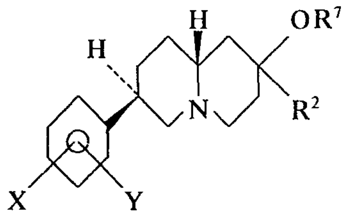

(IV)

worin X, Y und $R^2$ dasselbe wie oben bedeuten und $R^7$ $C_{1-6}$-Alkyl oder Acyl darstellt,

einen Alkohol der obigen allgemeinen Formel II mit einem den Rest $R^7$ abgebenden Verätherungs- bzw. Veresterungsmittel umsetzt und daß man erhaltene Racemate gegebenenfalls spaltet und erhaltene Basen gegebenenfalls in Säureadditionssalze überführt.

Sämtliche der in den vorstehenden Verfahrensvarianten 1 – 7 genannten Reaktionen können nach an sich bekannten Methoden durchgeführt werden.

Zur Überführung eines Ketons V in ein tertiäres Carbinol II gemäß der Verfahrensvariante 1a) können die üblichen, den Rest $R^2$ liefernden metallorganischen Verbindungen, wie die entsprechenden Grignard-verbindungen $R^2$Mg-Halogen, $R^2$-Lithium-verbindungen oder $R^2$-Lithium-Kupfer-verbindungen verwendet werden.

Mit der Carbinolbildung aus dem Keton V tritt ein drittes Asymmetriezentrum (in 2-Stellung) auf. Der neu eintretende Substituent $R^2$ kann dabei mit Bezug auf das 9a-Wasserstoffatom die cis- und/oder trans-Position einnehmen. So werden z. B. bei der im nachfolgenden Beispiel 3 beschriebenen Umsetzung eines racemischen Ketons V zwei diastereomere Produkte II (je als Racemat) erhalten, während bei der im nachstehenden Beispiel 1 beschriebenen Umsetzung eines racemischen Ketons V von den beiden theoretisch möglichen diastereomeren Racematen II praktisch nur das eine Racemat, und zwar dasjenige mit $R^2$ in trans-Position zum 9a-Wasserstoff, wie dies durch die Formel II-1 ausgedrückt wird, erhalten wird.

Erhaltene Diastereomeren-Gemische wie auch erhaltene Racemate lassen sich nach an sich bekannten Methoden auftrennen bzw. spalten.

Zur Anlagerung von Wasser an die $\Delta^1$- bzw. $\Delta^2$-Doppelbindung von Verbindungen der Formel VI gemäß der Verfahrensvariante 1b) kann als Hydratisierungsmittel beispielsweise wässerige Schwefelsäure benützt werden.

Zur Herstellung der Benzimidazol-Derivate III gemäß der Verfahrensvariante 2 kann man eine Verbindung VII, die als abspaltbare Gruppe Z z. B. Cl, Br, Mesyloxy oder Tosyloxy enthält, mit einer Verbindung VIII, vorzugsweise in Form eines Alkalimetallsalzes, umsetzen.

Zur Herstellung der Benzimidazolderivate III-3 nach der Verfahrensvariante 3 kann man die $\Delta^1$-Doppelbindung einer Verbindung IX katalytisch hydrieren, wobei als Katalysatoren übliche Hydrierungskatalysatoren, wie $PtO_2$ oder Pd/C (z. B. 10%ig) verwendet werden können.

Zur Herstellung des Benzimidazolderivate III-4 nach der Verfahrensvariante 4 kann man ein Phenylendiamin X mit einem Cyclisierungsmittel XI, wie z. B. Phosgen, Carbonyldiimidazol oder Harnstoff, kondensieren, wobei ein Benzimidazolinon-Derivat des Typus III-1 entsteht, oder auch mit Essigsäure oder einem Orthoessigsäureester, z. B. dem Trimethyl- oder Triäthylester, wobei ein Methylbenzimidazol-Derivat des Typus III-2 erhalten wird.

Zur Herstellung der Thione III-3 kann man entweder gemäß Variante 5a nach an sich bekannten Methoden den Sauerstoff des Imidazolrings einer Oxo-verbindung III-6 mit $P_2S_5$ gegen Schwefel austauschen, oder gemäß Variante 5b auf an sich bekannte Art ein Phenylendiamin X mit Thiophosgen, Thiocarbonyldiimidazol oder $CS_2$ umsetzen. Die anschließende, fakultative N-Alkylierung an $R^4$ erfolgt wie nachstehend für die Verfahrensvariante 6 angegeben.

Die Verfahrensvarianten 4 und 5b eignen sich besonders gut zur Herstellung von Verbindungen III-4 bzw. III-5, worin $R^5$ von $R^6$ verschieden ist.

Zur Herstellung von im Imidazolring N-alkylierten Verbindungen III-7 nach der Verfahrensvariante 6 kann man ein übliches N-Alkylierungsmittel, wie ein nieder-Alkylhalogenid, z. B. Methyljodid, verwenden.

Zur Herstellung der Äther und Ester IV nach der Verfahrensvariante 7 kann man auf an sich bekannte Art ein Carbinol II, z. B. durch Umsetzung mit einem Säureanhydrid $(R^7CO)_2O$ in Gegenwart von Pyridin oder mit einem Isopropenylester $CH_2{=}C(CH_3)O-CO-R_7$ in Gegenwart von p-Toluolsulfonsäure, verestern bzw. durch Umsetzung mit einem nieder-Alkylhalogenid (basische Verätherung) oder einem $R^7OH$/Schwefelsäure-Gemisch (saure Verätherung) veräthern.

Die im folgenden als Zwischenprodukte bzw. Ausgangsstoffe genannten Verbindungen können, soweit sie nicht bekannt sind, nach an sich bekannten Methoden hergestellt werden. So können z. B.

die in der Verfahrensvariante 1a) als Ausgangsmaterial verwendeten Ketone V auf an sich bekannte Art durch Decarboxylierung von β-Keto-estern der allgemeinen Formel XIV mit der in dieser Formel angegebenen relativen Konfiguration bezüglich der Positionen 1, 7 und 9a

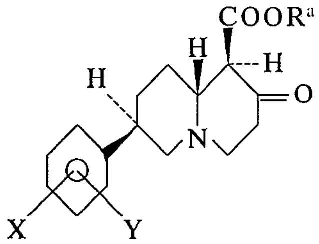

(XIV)

worin X und Y dasselbe wie oben bedeuten und $R^a$ niederer-Alkyl darstellt,

erhalten werden (vgl. z. B. Beispiel 1b).

Die β-Keto-ester XIV können ihrerseits aus den entsprechenden ungesättigten β-Keto-estern der allgemeinen Formel XIII

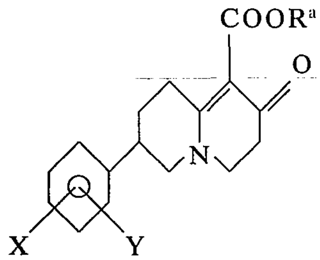

(XIII)

worin X, Y und $R^a$ dasselbe wie oben bedeuten,

auf an sich bekannte Art durch Hydrierung erhalten werden (vgl. z. B. das Beispiel 1c).

Die Ester XIII können aus entsprechenden im Phenyl gegebenenfalls substituierten 5-Phenyl-2-piperidonen nach an sich bekannten Methoden (vgl. z. B. das Beispiel 1d) erhalten werden.

Die in der Verfahrensvariante 6 als Ausgangsmaterial genannten Benzimidazoline III-8 können aus den entsprechenden ungesättigten Verbindungen der allgemeinen Formel XV (mit der in dieser Formel angegebenen relativen Konfiguration in den Stellungen 7 und 9a)

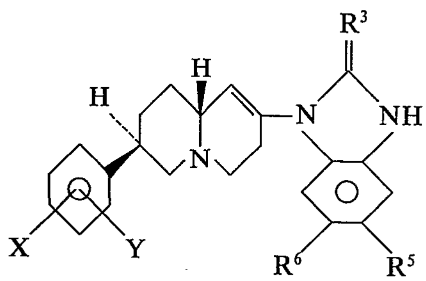

(XV)

worin X, Y, $R^3$, $R^5$ und $R^6$ dasselbe wie oben bedeuten,

durch Hydrierung erhalten werden.

Durch N-Alkylierung von XV wie in Verfahrensvariante 6 erhält man N-alkylierte Ausgangsverbindungen IX.

Die Verbindungen XV können ihrerseits aus den bereits genannten β-Keto-estern XIV nach dem in Beispiel 7b beschriebenen Verfahren bzw. in Analogie dazu erhalten werden.

Die Verbindungen VI und VII können nach dem in den Beispielen 13 und 14 beschriebenen Verfahren bzw. in Analogie dazu erhalten werden.

Die erfindungsgemäßen Phenyl-chinolizidine der Formel I zeichnen sich durch wertvolle pharmakologische Eigenschaften aus. Sie sind neuroleptisch, zentraldämpfend, tranquillisierend, antiemetisch und/oder analgetisch wirksam und können demgemäß als Antipsychotica bzw. Antiemetica bzw. Analgetica bei der Bekämpfung von Psychosen und Neurosen bzw. von Übelkeit bzw. von Schmerz Verwendung finden.

Die neuroleptischen Eigenschaften der Verbindungen wurden zahlenmäßig mit den nachstehend beschriebenen Tests ermittelt:

»Pole Climbing« Test (Ratte)

Zur Versuchsdurchführung wurde pro Dosis eine Gruppe von 10 Ratten eingesetzt, die daraufhin trainiert worden waren, durch Hinaufspringen an eine vertikale isolierte Stange im Versuchskäfig, dem unmittelbar nach einem akustischen Signal (konditionierter Reiz), via Bodengitter ausgelösten Elektroschock (unkonditionierter Reiz) zu entkommen. Die Hemmung der konditionierten Reaktion bei 50% der Tiere während einer Beobachtungszeit von 6 Stunden wird durch den Parameter ED 50 BCR, die Hemmung der unkonditionierten Reaktion durch den Parameter ED 10 BUR bestimmt.

Spiroperidol-binding Test (in vitro)

In den in vitro Versuchen wurde Kalbsstriatum als Rezeptor verwendet (Lit.: Creese et al., Life Sci. 17, 993 (1975) und analog der von Fields et al., Brain Research 136, 578 (1977) beschriebenen Methoden mit [$^3$H] Spiroperidol inkubiert. Mit einem Computerprogramm wurde aus 4 verschiedenen Konzentrationen in dreifacher Ausführung die IC 50 bestimmt, welche die Konzentration der Testsubstanz angibt, bei welcher eine 50%ige Ablösung der spezifischen Bindung von [$^3$H] Spiroperidol am Rezeptor eintritt.

In der nachstehenden Tabelle A sind einige Versuchsresultate zusammengestellt.

Tabelle A

| Verbindung | Pole-Climbing | | Spiroperidol-Binding |
|---|---|---|---|
| | BCR | BUR | |
| | $ED_{50}$ i.p. (mg/kg) | $ED_{10}$ i.p. (mg/kg) | $IC_{50}$ (nanomolar) |
| rac.-(9a$\beta$H)-2$\alpha$-tert.Butyl-7$\beta$-(2,4-dichlorphenyl)octa-hydro-2H-chinolizin-2-ol · HCl | 6,4 | 5,4 | 79 |
| (−)-(2S,7R,9aR)-2-tert.Butyl-7-(2,4-dichlorphenyl)octahydro-2H-chinolizin-2-ol · HCl | 4,7 | 3,8 | 41 |
| rac.-1-[(9a$\beta$H)-7$\beta$-(o-Chlorphenyl)-octahydro-2H-chino-lizin-2$\alpha$-yl]-5,6-dimethyl-2-benzimidazolinon · HCl | 1,75 | 4,55 | 5 |
| rac.-1-[(9a$\beta$H)-7$\beta$-(o-Chlorphenyl)-octahydro-2H-chino-lizin-2$\alpha$-yl]-5-trifluormethyl-2-benzimidazolinon · HCl | 0,21 | 0,41 | 10 |
| rac.-1-[(9a$\beta$H)-7$\beta$-(o-Fluorphenyl)-octahydro-2H-chino-lizin-2$\alpha$-yl]-5-chlor-2-benzimidazolinon · HCl | 0,064 | 0,175 | 3,1 |

Die analgetischen Eigenschaften der Verbindungen wurden zahlenmäßig mit den nachstehenden beschriebenen Tests ermittelt:

Writhing Test (Maus)

Zur Durchführung der Versuche wurden 8 männliche Mäuse (20−22 g) pro Dosis eingesetzt. 60 Minuten nach erfolgter oraler Verabreichung der Testsubstanz wurde den Tieren 10 ml/kg der Testlösung intraperitoneal injiziert. Anschließend an eine Latenzzeit von 5 Minuten wurde die Zahl der Tiere registriert, bei denen während 5 Minuten nicht mehr als 1 charakteristisches Writhing-Symptom (konvulsive Streckbewegung des Körpers) auftrat. Die ED 50 gibt diejenige Dosis an, bei welcher 50% der Tiere nicht mehr als 1 Writhing zeigen.

Hot-plate Test (Maus)

8 Mäuse (20−22 g) wurden pro Dosis eingesetzt. Die Tiere wurden 60 Minuten nach oraler Gabe der Testsubstanz auf eine erwärmte Metallplatte von 60±0,5° C gesetzt. Unbehandelte Mäuse begannen

innerhalb von max. 10 Sekunden ihre Pfoten zu lecken. Die ED 50 ist als jene Dosis definiert, bei der 50% der Tiere mit dem Pfotenlecken erst nach einer Latenzzeit von mehr als 10 Sekunden beginnen.

In der nachstehenden Tabelle B sind einige Versuchsresultate zusammengestellt:

Tabelle B

| Verbindung | Writhing 60′ $ED_{50}$ p.o. mg/kg | Hot-plate 60′ $ED_{50}$ p.o. mg/kg |
|---|---|---|
| (+)-2-Butyloctahydro-7-phenyl-2H-chinolizin-2-yl-acetat · HCl | 0,19 | 2,6 |
| rac.-(9a$\beta$H)-2$\alpha$-n-Butyl-7$\beta$-phenyl-octahydro-2H-chinolizin-2-yl-acetat · HCl | 0,75 | 25 |
| rac.-(9a$\beta$H)-2$\alpha$-tert.Butyl-7$\beta$-phenyl-octahydro-2H-chinolizin-2-yl-trifluoracetat · HCl | 8,7 | 86 |
| rac.-(9a$\beta$H)-2$\alpha$-Phenyl-7$\beta$-(m-methoxyphenyl)-octahydro-2H-chinolizin-2-yl-propionat · HCl | 2,8 | 12 |
| rac.-(9a$\beta$H)-2$\beta$-Phenyl-7$\beta$-(o-chlorphenyl)-octahydro-2H-chinolizin-2-yl-acetat · HCl | 116 | 284 |

Die erfindungsgemäßen Verbindungen der Formel I in Form des Racemats oder der Enantiomeren, sowie in Form der freien Basen wie von Säureadditionssalzen können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rekatal, z. B. in Form von Suppositorien, lokal oder perkutan, z. B. in Form von Salben, Crèmes, Gelées, Lösungen, oder parenteral, z. B. in Form von Injektionslösungen, erfolgen.

Zur Herstellung von Tabletten, Lacktabletten, Dragées und Hartgelatinekapseln können die erfindungsgemäßen Verbindungen mit pharmazeutisch inerten, anorganischen oder organischen Excipientien verabreicht werden. Als solche Excipientien kann man z. B. für Tabletten, Dragées und Hartgelatinekapseln Lactose, Maisstärke oder Derivate davon, Talk, Stearinsäure oder deren Salze etc. verwenden. Für Weichgelatinekapseln eignen sich als Excipientien z. B. vegetabile Öle, Wachse, Fette, halbfeste und flüssige Polyole etc.; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z. B. Wasser, Polyole, Saccharose, Invertzucker, Glukose und dergleichen. Für Injektionslösungen eignen sich als Excipientien z. B. Wasser, Alkohole, Polyole, Glyzerin, vegetabile Öle etc. Für Suppositorien, lokale oder perkutane Anwendungsformen eignen sich als Excipientien z. B. natürliche oder gehärtete Öle, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

In den genannten pharmazeutischen Präparaten kann die Dosierung etwa im Bereich von 0,5—100 mg liegen. Bei oraler Applikation kommen Wirkstoffmengen von etwa 0,05 mg/kg bis etwa 10 mg/kg pro Tag und bei parenteraler Applikation solche von etwa 0,01 mg/kg bis 1 mg/kg pro Tag in Betracht.

## Beispiel 1

a) Zu einer auf −75° C gekühlten Lösung von tert. Butyllithium in Pentan (100 ml; 1,2 molar) werden unter starkem Rühren 22,2 g rac-(9a$\beta$H)-7$\beta$-(o-Chlorphenyl)octahydro-2H-chinolizin-2-on, gelöst in 300 ml absolutem Tetrahydrofuran, tropfenweise unter Argon zugesetzt. Anschließend beläßt man weitere 30 Minuten bei −75° C und arbeitet dann wie folgt auf: Durch Zugabe von 100 ml gesättigter Ammoniumchloridlösung wird hydrolysiert und dann zwischen Essigester und Wasser verteilt. Nach nochmaliger Extraktion mit Essigester trocknet man die gesamte organische Phase über

Magnesiumsulfat und dampft ein. Aus dem Rückstand kann das Endprodukt (rac-(9a$\beta$H)-2$\alpha$-tert.Butyl-7$\beta$-(o-chlorphenyl)octahydro-2H-chinolizin-2-ol) aus 1400 ml Essigester durch Einleiten von gasförmigem Chlorwasserstoff als HCl-Salz kristallisiert werden: Ausbeute 20,4 g (71%). Smp. 276–278° C. Als Variante zu diesem Reinigungsverfahren kann der Rückstand an Kieselgel mit Äther–Hexan = 1 : 1 chromatographiert werden.

Wenn anstelle von rac-(9a$\beta$H)-7$\beta$-(o-Chlorphenyl)octahydro-2H-chinolizin-2-on äquimolare Mengen entsprechend substituierter Ketone zur Reaktion eingesetzt werden, können folgende tert. Butylcarbinole erhalten werden:

Tabelle 1

rac-(9a$\beta$H)-2$\alpha$-tert.Butyl-7$\beta$-($R_a$)octahydro-2H-chinolizin-2-ol:

| | | Smp. °C |
|---|---|---|
| $R_a$ = phenyl | HCl-Salz | 230 (Zers.) |
| p-chlorphenyl | HCl-Salz | 286–288 |
| 2,4-dichlorphenyl | HCl-Salz | 270–272 |
| 2,6-dichlorphenyl | HCl-Salz | 262–263 |
| p-trifluormethylphenyl | HCl-Salz | 285–286 |

Wenn anstelle von tert. Butyllithium äquimolare Mengen analoger Alkyl- oder Aryllithium-Reagenzien mit den entsprechenden Ketonen umgesetzt werden, können folgende Carbinole erhalten werden:

Tabelle 2

rac-(9a$\beta$H)-($R_b$)-7$\beta$-($R_a$)octahydro-2H-chinolizin-2-ol:

| | | | Smp. °C |
|---|---|---|---|
| $R_b$ = 2$\alpha$-sec.-Butyl | $R_a$ = 2,4-dichlorphenyl | HCl-Salz | 244–246 |
| 2$\alpha$-n-Butyl | 2,4-dichlorphenyl | HCl-Salz | 227–230 |
| 2$\alpha$-iso-Propyl | 2,4-dichlorphenyl | HCl-Salz | 201–205 |
| 2($\alpha$+$\beta$)-Methyl | 2,4-dichlorphenyl | HCl-Salz | 149–151 |
| 2$\alpha$-n-Butyl | phenyl | Base | 100–102 |
| 2$\alpha$-phenyl | o-chlorphenyl | HCl-Salz | 266–267 |
| 2$\beta$-phenyl | o-chlorphenyl | HCl-Salz | 175° amorph |
| 2$\alpha$-phenyl | m-methoxyphenyl | Base | 153–155 |
| 2$\beta$-phenyl | m-methoxyphenyl | Base | 122–124 |
| 2$\alpha$-phenyl | phenyl | Base | 156–158 |
| 2$\beta$-phenyl | phenyl | Base | 99–102 |
| 2$\alpha$-m-Methoxyphenyl | phenyl | $^1$H-NMR | ($CDCl_3$) : 3,77 (s) |
| 2$\beta$-m-Methoxyphenyl | phenyl | $^1$H-NMR | ($CDCl_3$) : 3,82 (s) |
| 2$\alpha$-p-chlorphenyl | phenyl | HCl-Salz | 249–250 |
| 2$\alpha$-m-trifluormethylphenyl | p-chlorphenyl | Base | 119–123 |
| 2$\beta$-m-trifluormethylphenyl | p-chlorphenyl | Base | 122–124 |

b) Das als Ausgangsmaterial verwendete rac-(9a$\beta$H)-7$\beta$-(o-Chlorphenyl)octahydro-2H-chinolizin-2-on kann wie folgt erhalten werden:

76,2 g Methyl-rac-(1$\alpha$H,9a$B$H)-7$\beta$-(o-chlorphenyl)octahydro-2-oxo-2H-chinolizin-1-carboxylat werden in 1,2 l 6N Salzsäure gelöst und 3 Stunden am Rückfluß gekocht. Die abgekühlte Reaktionslösung wird anschließend auf Eis gegossen und mit konz. Natronlauge alkalisch gestellt. Extraktion mit 3 × 500 ml Methylenchlorid und Eindampfen der über Magnesiumsulfat getrockneten organischen Phase ergeben 68 g Rohprodukt. Aus Äther-Hexan können 50,4 g rac-(9a$\beta$H)-7$\beta$-(o-Chlorphenyl)octahydro-2H-chinolizin-2-on kristallisiert werden. Smp. 80—82° C (81%).

Wenn anstelle von Methyl-rac-(1$\alpha$H,9a$\beta$H-7$\beta$-(o-chlorphenyl)octahydro-2-oxo-2H-chinolizin-1-carboxylat äquimolare Mengen entsprechend substituierter $\beta$-Ketoester decarboxyliert werden, erhält man folgende Produkte:

Tabelle 3

rac-(9a$\beta$H)-7$\beta$-($R_a$)octahydro-2H-chinolizin-2-on:

| | | | Smp. °C |
|---|---|---|---|
| $R_a$ = | phenyl | | 71—72 |
| | p-chlorphenyl | | 80—82 |
| | p-trifluormethylphenyl | | 103—104 |
| | o-methylphenyl | IR(Film) | 1726 $cm^{-1}$ |
| | m-methoxyphenyl | IR(Film) | 1726 $cm^{-1}$ |
| | 2,4-dichlorphenyl | | 116—117 |
| | 2,6-dichlorphenyl | | 116—118 |
| | o-fluorphenyl | | 74—76 |

c) Das im Absatz 1b) als Ausgangsmaterial verwendete Octahydro-carboxylat kann seinerseits aus dem entsprechendem Hexahydro-carboxylat wie folgt erhalten werden:

180 g Methyl-rac-7-(o-chlorphenyl)-3,4,6,7,8,9-hexahydro-2-oxo-2H-chinolizin-1-carboxylat suspendiert man in 3,6 l Monoglym(Dimethoxyäthan), kühlt auf —30° C und addiert unter Rühren 1,33 l DIBAH (Diisobutylaluminiumhydrid, 20%ige Lösung in Toluol). Anschließend wird 1 Stunde bei —20 bis —30°C gerührt und dann bei dieser Temperatur mit 1,72 l 2N Natronlauge hydrolysiert. Zur Aufarbeitung verteilt man zwischen 25 l Wasser und 20 l Chloroform; die organische Phase wird nochmals mit 2 × 5 l Wasser gewaschen, über Magnesiumsulfat getrocknet und eingedampft: 185,2 g Rohprodukt. Aus Äther-Hexan können 109 g Produkt kristallisiert werden. Durch Chromatographie der Mutterlauge an 1 kg Kieselgel mit Essigester werden nochmals 37 g Produkt erhalten. Gesamtausbeute an Methyl-rac-(1$\alpha$H,9a$\beta$H)-7$\beta$-(o-chlorphenyl)-octahydro-2-oxo-2H-chinolizin-1-carboxylat: 136 g (81%), Smp. 122—124° C.

Wenn anstelle von Methyl-rac-7-(o-chlorphenyl)-3,4,6,7,8,9-hexahydro-2-oxo-2H-chinolizin-1-carboxylat äquimolare Mengen entsprechend substituierter Edukte mit DIBAH reduziert werden, gelangt man zu folgenden Verbindungen:

Tabelle 4

Methyl-rac-(1αH,9aβH)-7β-($R_a$)octahydro-2-oxo-2H-chinolizin-1-carboxylat:

| | | | Smp. °C |
|---|---|---|---|
| $R_a$ = | phenyl | | 114–116 |
| | p-chlorphenyl | | 102–105 |
| | p-trifluormethylphenyl | | 108–111 |
| | o-methylphenyl | | 94–98 |
| | m-methoxyphenyl | IR(Film): | 1752, 1723, 1660 $cm^{-1}$ |
| | 2,4-dichlorphenyl | | 116–118 |
| | 2,6-dichlorphenyl | | 130–131 |
| | o-fluorphenyl | | 109–110 |

d) Das im Absatz 1c) als Ausgangsmaterial verwendete Hexahydro-carboxylat kann seinerseits wie folgt erhalten werden:

150 g 5-(o-Chlorphenyl)-2-piperidon werden in 2 l Methylenchlorid gelöst und zu 410 g Triäthyloxoniumtetrafluorborat (Meerweinsalz) in 1 l Methylenchlorid bei Zimmertemperatur unter Rühren innerhalb von 60 Minuten zugetropft. Anschließend kocht man 4 Stunden am Rückfluß und beläßt weitere 15 Stunden bei Zimmertemperatur. In die auf 0°C gekühlte Lösung werden sodann 372 g Kaliumcarbonat in 370 ml Wasser zugetropft, wobei intensiv gerührt wird. Man läßt weitere 2 Stunden bei Zimmertemperatur rühren, dekantiert anschließend die Methylenchlorid-Phase und extrahiert den Rückstand mit 2×500 ml Methylenchlorid. Die über Kaliumcarbonat getrocknete Methylenchlorid-Phase wird zu einem öligen, teils kristallinen Rückstand eingeengt, sodann in 1 l Hexan aufgekocht und heiß filtriert. Aus dem Filtrat werden nach dem Abdampfen des Hexans 151,4 g des öligen Lactimäthers erhalten: 3-(o-Chlorphenyl)-6-äthoxy-2,3,4,5-tetrahydropyridin (IR(Film): 1684 $cm^{-1}$).

Man löst den Lactimäther (151,4 g) in 1,4 l Methanol, gibt 1,3 g wasserfreie p-Toluolsulfonsäure zu und addiert innerhalb von 60 Minuten 85 g 3-Oxo-5-pentensäure-methylester (Nazarov-Reagens). Nach 20 Stunden bei Raumtemperatur werden die leichtflüchtigen Anteile im Vakuum entfernt, der Rückstand anschließend in Methylenchlorid aufgenommen und mit 500 ml ges. Natriumcarbonat-Lösung gewaschen. Die über Magnesiumsulfat getrocknete Methylenchlorid-Phase wird wiederum eingeengt und der Rückstand aus Essigester-Äther = 4 : 1 (800 ml) kristallisiert. Man erhält 123,7 g (54%) Methyl-rac-7-(o-chlorphenyl)-3,4,6,7,8,9-hexahydro-2-oxo-2H-chinolizin-1-carboxylat. Smp. 145–147°C.

Wenn anstelle von 5-(o-Chlorphenyl)-2-piperidon äquimolare Mengen entsprechend substituierter Piperidone verwendet werden, erhält man die folgenden Verbindungen:

Tabelle 5

Methyl-rac-7-($R_a$)-3,4,6,7,8,9-hexahydro-2-oxo-2H-chinolizin-1-carboxylat:

| | | Smp. °C |
|---|---|---|
| $R_a$ = | phenyl | 148–149 |
| | p-chlorphenyl | 185–187 |
| | p-trifluormethylphenyl | 144–145 |
| | o-methylphenyl | 183–184 |
| | m-methoxyphenyl | 133–134 |
| | 2,4-dichlorphenyl | 153–155 |
| | 2,6-dichlorphenyl | 159–162 |
| | o-fluorphenyl | 165–167 |

## Beispiel 2

Eine Lösung von 12,2 g rac-(9a$\beta$H)-2$\alpha$-tert.Butyl-7$\beta$-(2,4-dichlorphenyl)octahydro-2H-chinolizin-2-ol in 80 ml Methanol wird mit einer Lösung von 13,3 g (+)-2,2'-(1,1'-Binaphthyl)-phosphorsäure in 250 ml Methanol und 250 ml Methylenchlorid versetzt. Die ausgefallene Gallerte bringt man mit 1,2 l heißem Methanol in Lösung und dampft anschließend bis auf ein Volumen von 500 ml ein, worauf bei 0° C das (+)-Phosphat auskristallisiert: 8,7 g (72,5%), Smp. 288–290° C. Aus dem (+)-Phosphat kann die (+)-Base [(+) (2R,7S,9aS)-2-tert.Butyl-7-(2,4-dichlorphenyl)octahydro-2H-chinolizin-2-ol] (4,2 g) mit Ammoniak freigesetzt werden, die in 50 ml Äthanol gelöst und mit 2,5 ml 5N Chlorwasserstoff in Äthanol versetzt bei 0° C 4,0 g (+)-HCl-Salz liefert: Smp. 275–276° C $[\alpha]_D$ = +23,1° (c = 1% in Methanol).

Die Mutterlauge aus der Kristallisation des (+)-Phosphates wird mit konz. Ammoniak basisch gestellt und mit Essigester-Äther = 1 : 1 extrahiert. Die organische Phase wird mit Wasser neutral gewaschen, über Magnesiumsulfat getrocknet und eingedampft: 7,7 g. Diese Base bringt man mit 50 ml Methanol in Lösung und addiert 7,33 g (−)-2,2'-(1,1'-Binaphthyl)-phosphorsäure in 200 ml Methanol und 200 ml Methylenchlorid. Dann wird auf 250 ml eingeengt, 250 ml Aceton addiert und auf 0° C kalt gestellt. Es kristallisieren 8,2 g (−)-Phosphat aus: Smp. 279–281° C. Die daraus mit Ammoniak freigesetzte Base [(−)(2S,7R,9aR)-2-tert.Butyl-7-(2,4-dichlorphenyl)octahydro-2H-chinolizin-2-ol] (4,2 g) wird in 50 ml Äthanol gelöst und mit 2,5 ml 5N Chlorwasserstoff in Äthanol versetzt. Bei 0° C kristallisieren 4,1 g (−)-HCl-Salz aus: Smp. 275–276° C, $[\alpha]_D$ = −23,1° (c = 1% in Methanol).

Analog können die folgenden Enantiomeren durch Spaltung des entsprechenden Racemats isoliert werden:

(+)-2-Butyloctahydro-7-phenyl-2H-chinolizin-2-ol; Base Smp. 99–101° C
(−)-2-Butyloctahydro-7-phenyl-2H-chinolizin-2-ol; Base Smp. 99–101° C.

## Beispiel 3

Aus 0,54 g Magnesiumspänen in 5 ml abs. Äther und 5 g m-Brombenzotrifluorid in 10 ml Äther wird zunächst das Grignard-Reagens durch 2stündiges Kochen unter Rückfluß hergestellt. Zu der auf 0° C abgekühlten Suspension gibt man anschließend 2,64 g rac-(9a$\beta$H)-7$\beta$-(p-Chlorphenyl)octahydro-2H-chinolizin-2-on (erhalten gem. Beispiel 1b) in 25 ml abs. Äther tropfenweise zu und rührt eine weitere Stunde bei Raumtemperatur unter Argon. Zur Hydrolyse wird mit gesättigter Ammoniumchlorid-Lösung versetzt und das Reaktionsprodukt mit Methylenchlorid extrahiert. Die mit gesättigter Natriumchlorid-Lösung gewaschene organische Phase wird über Magnesiumsulfat getrocknet und eingedampft: Ausbeute 4,60 g. Dieser Rückstand ergibt nach Chromatographie an Kieselgel mit Essigester-Methanol 2 diastereomere Produkte:

1,4 g rac-(9a$\beta$H)-2$\alpha$-(m-Trifluormethylphenyl)-7$\beta$-(p-chlorophenyl)octahydro-2H-chinolizin-2-ol, Smp.: 119–123° C (34%)

1,2 g rac-(9a$\beta$H)-2$\beta$-(m-Trifluormethylphenyl)-7$\beta$-(p-chlorophenyl)octahydro-2H-chinolizin-2-ol, Smp.: 122–124° C (29%)

Wenn äquimolare Mengen analoger Alkyl- oder Aryllithium-Reagenzien verwendet werden, können die in Beispiel 1a, Tabelle 2 aufgeführten Verbindungen erhalten werden.

Beispiel 4

20,0 g rac-(9a$\beta$H)-2$\alpha$-tert.Butyl-7$\beta$-(o-chlorphenyl)octahydro-2H-chinolizin-2-ol (erhalten gemäß Beispiel 1a) löst man in 130 ml Isopropenylacetat und addiert 11,7 g wasserfreie p-Toluolsulfonsäure. Darauf wird 1 Stunde unter Rühren am Rückfluß gekocht (Argon). Nach Abkühlen der Reaktionslösung werden noch 20 ml Äther zugegeben. Die Lösung wird 2 Stunden stehengelassen und dann das ausgefallene p-Toluolsolfonat des Produkts abfiltriert und getrocknet: 22,5 g. Aus dem p-Toluolsolfonat kann die Base (rac-(9a$\beta$H)-2$\alpha$-tert.Butyl-7$\beta$-(o-chlorphenyl)octahydro-2H-chinolizin-2-yl-acetat) mit Natriumbicarbonat-Lösung freigesetzt werden: 15,8 g. (70%). HCl-Salz Smp. 241–242° C.

Wenn äquimolare Mengen der im Beispiel 1a ausgeführten Alkylcarbinole zur Reaktion eingesetzt werden, können die in Tabelle 6 angegebenen Acetate erhalten werden:

Tabelle 6

rac-(9a$\beta$H)-2$\alpha$-($R_b$)-7$\beta$-($R_a$)octahydro-2H-chinolizin-2-yl-acetat:

| | | | Smp. °C |
|---|---|---|---|
| $R_b$ = tert.Butyl | $R_a$ = phenyl | HCl-Salz | 247–248 |
| tert.Butyl | p-chlorphenyl | HCl-Salz | 244–246 |
| tert.Butyl | 2,4-dichlorphenyl | HCl-Salz | 246–247 |
| n-Butyl | phenyl | HCl-Salz | 248–250 |

Beispiel 5

5,0 g rac-(9a$\beta$H)-2$\alpha$-tert.Butyl-7$\beta$-(o-chlorphenyl)octahydro-2H-chinolizin-2-ol werden in 20 ml Pyridin gelöst und innerhalb von 15 Minuten tropfenweise mit 20 ml Trifluoracetanhydrid bei Raumtemperatur versetzt. Nach 22 Stunden bei Raumtemperatur dampft man die Lösung im Vakuum ein, löst den Rückstand in 50 ml Toluol und engt wiederum ein. Anschließend wird der ölige Rückstand an 100 g Kieselgel mit Essigester chromatographiert. Das Eluat liefert 5,4 g (83,5%) der Produkt-Base (rac-(9a$\beta$H)-2$\alpha$-tert.Butyl-7$\beta$-(o-chlorphenyl)octahydro-2H-chinolizin-2-yl-trifluoracetat). HCl-Salz Smp. 172–173° C.

Wenn äquimolare Mengen der in den Beispielen 1a und 3 aufgeführten Alkyl- oder Arylcarbinole zur Reaktion verwendet werden, können die in Tabelle 7 aufgeführten Trifluoracetate erhalten werden:

Tabelle 7

rac-(9a$\beta$H)-($R_b$)-7$\beta$-($R_a$)octahydro-2H-chinolizin-2-yl-trifluoracetat:

| | | | Smp. °C |
|---|---|---|---|
| $R_b$ = 2$\alpha$-tert.Butyl | $R_a$ = phenyl | HCl-Salz | 170–172 |
| 2$\alpha$-phenyl | phenyl | HCl-Salz | 164–166 |

Wenn anstelle von Trifluoracetanhydrid äquimolare Mengen der in den Beispielen 1a und 3 aufgeführten Alkyl- oder Arylcarbinole mit analogen Carbonsäureanhydriden umgesetzt werden, gelangt man zu folgenden Carbinolacylaten:

Tabelle 8

rac-(9a$\beta$H)-($R_b$)-7$\beta$-($R_a$)octahydro-2H-chinolizin-2-yl-($R_c$):

| | | | | Smp. °C |
|---|---|---|---|---|
| $R_b$ = 2$\alpha$-phenyl | $R_a$ = o-chlorphenyl | c = acetat | HCl-Salz | 220–222 |
| 2$\beta$-phenyl | o-chlorphenyl | acetat | HCl-Salz | 218–220 |
| 2$\alpha$-phenyl | phenyl | propionat | HCl-Salz | 242–243 |
| 2$\alpha$-phenyl | m-methoxyphenyl | propionat | HCl-Salz | 212–214 |
| 2$\beta$-phenyl | m-methoxyphenyl | propionat | HCl-Salz | 233–235 |
| 2$\alpha$-m-methoxyphenyl | phenyl | propionat | HCl-Salz | 233–234 |
| 2$\beta$-m-methoxyphenyl | phenyl | propionat | Oxalat | 120–122 |

Beispiel 6

a) 3,0 g rac-(9a$\beta$H)-2$\beta$-phenyl-7$\beta$-(o-chlorphenyl)octahydro-2H-chinolizin-2-ol (erhalten gem. Beispiel 1a) löst man in 75 ml Methanol und versetzt innerhalb von 15 Minuten mit 15 ml konz. Schwefelsäure (96%) und kocht dann 2 Stunden am Rückfluß. Die abgekühlte Reaktionslösung wird auf konz. Natronlauge/Eis gegossen und mit Essigester (3 x 200 ml) extrahiert. Die über Magnesiumsulfat getrocknete organische Phase ergibt nach dem Einengen 3,2 g Rohprodukt, das an 300 g Kieselgel mit Hexan-Äther chromatographiert wird: 1,7 g (54%) rac-(9a$\beta$H)-2$\beta$-Methoxy-2-phenyl-7$\beta$-(o-chlorphenyl)octahydro-2H-chinolizin. HCl-Salz, Smp. 258–260° C.

Wenn anstelle von rac-(9a$\beta$H)-2$\beta$-phenyl-7$\beta$-(o-chlorphenyl)octahydro-2H-chinolizin-2-ol entsprechend substituierte Arylcarbinole mit $C_1-C_4$-Alkoholen zur Reaktion gebracht werden, erhält man folgende O-($C_1-C_4$)Alkyl-Verbindungen:

Tabelle 9

rac-(9a$\beta$H)-2$\beta$-($R_b$)-2-phenyl-7$\beta$-($R_a$)octahydro-2H-chinolizin:

| | | | Smp. °C |
|---|---|---|---|
| $R_b$ = methoxy | $R_a$ = phenyl | HCl-Salz | 277–279 |
| aethoxy | phenyl | HCl-Salz | 254–255 |

b) 0,5 g rac-(9a$\beta$H)-2$\alpha$-phenyl-7$\beta$-(o-chlorphenyl)octahydro-2H-chinolizin-2-ol (erhalten gem. Beispiel 1a) werden in 5 ml HMPA (Hexamethyl-phosphorsäuretriamid) gelöst und bei 0° C mit 0,1 g Natriumhydrid (50–60% in Mineralöl) versetzt. Man rührt 1/2 Stunde bei 0° C und fügt dann 0,2 ml Methyljodid zu. Anschließend wird 15 Stunden bei Zimmertemperatur gerührt. Zur Aufarbeitung gießt man auf 50 ml Wasser und extrahiert 2 x mit 50 ml Essigester. Die Essigesterphase wird zusätzlich 3 x mit je 50 ml Wasser gewaschen, anschließend über Magnesiumsulfat getrocknet und eingedampft. Chromatographie an 50 g Kieselgel mit Hexan-Äther ergibt 0,2 g (38%) rac-(9a$\beta$H)-2$\beta$-Methoxy-2-phenyl-7$\beta$-(o-chlorphenyl)octahydro-2H-chinolizin. HCl-Salz, Smp. 258–260° C.

Beispiel 7

a) Eine Lösung von 7,45 g rac-1-(7$\beta$-)(o-Chlorphenyl)-3,6,7,8,9,9a$\beta$-hexahydro-4H-chinolizin-2-yl)-5,6-dimethyl-2-benzimidazolinon in 30 ml konz. Schwefelsäure und 300 ml abs. Äthanol wird mit 0,40 g Platinoxid während 6 Stunden bei Raumtemperatur hydriert. Anschließend filtriert man vom Katalysator ab, dampft das Filtrat ein und verteilt den Rückstand zwischen Chloroform und 2N Natronlauge. Die über Kaliumcarbonat getrocknete Chloroformphase ergibt nach dem Eindampfen 7,5 g Rohprodukt. Zur Reinigung wird das HCl-Salz aus Methanol-Äther kristallisiert: 6,7 g (90%) rac-1-((9a$\beta$H)-7$\beta$-(o-Chlorphenyl)octahydro-2H-chinolizin-2$\alpha$-yl)-5,6-dimethyl-2-benzimidazolinon-hydrochlorid, Smp.: 310° C. Als Varinate zu diesem Reinigungsverfahren kann die Base an Kieselgel mit Methylenchlorid-Methanol chromatographiert werden. Smp. Base: 268 – 272° C.

Wenn anstelle von rac-1-(7β-(o-Chlorphenyl)-3,6,7,8,9,9aβ-hexahydro-4H-chinolizin-2α-yl)-5,6-dimethyl-2-benzimidazolinon äquimolare Mengen entsprechend substituierter Edukte mit den in Tabelle 10 angegebenen Katalysatoren hydriert werden, gelangt man zu folgenden Produkten:

Tabelle 10

rac-1-((9aβH)-7β-($R_a$)-octahydro-2H-chinolizin-2α-yl)-(B)-2-benzimidazolinon:

| | | | Katalysator: | | Smp. °C |
|---|---|---|---|---|---|
| $R_a$ = | o-Chlorphenyl | B = 5,6-dichlor | $PtO_2$ | HCl-Salz | 300–305 |
| | o-Chlorphenyl | 5,6-H | $PtO_2$ | HCl-Salz | 315–318 |
| | o-Chlorphenyl | 5,6-dimethoxy | $PtO_2$ | HCl-Salz | 229–235 |
| | o-Chlorphenyl | 5-$(CH_2)_4$-6 | $PtO_2$ | HCl-Salz | 245–250 |
| | p-Chlorphenyl | 5,6-H | $PtO_2$ | Base | 244–248 |
| | p-Chlorphenyl | 5,6-dimethyl | $PtO_2$ | Base | 289–295 |
| | o-Methylphenyl | 5,6-H | 10% Pd/C | HCl-Salz | 285–290 |
| | o-Methylphenyl | 5,6-dimethyl | 10% Pd/C | HCl-Salz | 291–293 |
| | o-Methylphenyl | 5,6-dichlor | $PtO_2$ | Base | 294–297 |
| | Phenyl | 5,6-H | 10% Pd/C | HCl-Salz | 223–228 |
| | Phenyl | 5,6-dimethyl | 10% Pd/C | HCl-Salz | 295–302 |
| | Phenyl | 5,6-dichlor | $PtO_2$ | Base | 295–300 |
| | Phenyl | 5,6-dimethoxy | 10% Pd/C | Base | 248–250 |
| | o-Fluorphenyl | 5,6-dimethyl | 10% Pd/C | HCl-Salz | >300 |
| | o-Fluorphenyl | 5,6-dichlor | $PtO_2$ | HCl-Salz | >250 |
| | o-Fluorphenyl | 5,6-difluor | $PtO_2$ | HCl-Salz | >300 |
| | o-Fluorphenyl | 5-$(CH_2)_4$-6 | $PtO_2$ | HCl-Salz | 248–250 |

b) Das als Ausgangsmaterial verwendete rac-1-(7β-(o-Chlorphenyl)-3,6,7,8,9,9aβ-hexahydro-4H-chinolizin-2-yl)-5,6-dimethyl-2-benzimidazolinon kann wie folgt erhalten werden:

6,35 g 4,5-Dimethyl-1,2-phenylendiamin löst man in 100 ml Xylol, erwärmt auf Rückflußtemperatur und addiert innerhalb von 2 Stunden 15 g Methyl-rac-(1αH,9aβH)-7β-(o-chlorphenyl)octahydro-2-oxo-2H-chinolizin-1-carboxylat (erhalten gem. Beispiel 1c) in 100 ml Xylol. Danach kocht man 7 Stunden unter Rückfluß und kühlt dann ab. Aus der Reaktionslösung kristallisieren 13,65 g rac-1(7β-(o-Chlorphenyl)-3,6,7,8,9,9aβ-hexahydro-4H-chinolizin-2-yl)-5,6-dimethyl-2-benzimidazolinon aus (72%). Smp. 239 – 241° C.

Wenn anstelle von Methyl-rac-(1αH,9aβH)-7β-(o-chlorphenyl)octahydro-2-oxo-2H-chinolizin-1-carboxylat äquimolare Mengen entsprechend substituierter β-Ketoester mit 1,2-Phenylendiaminen umgesetzt werden, gelangt man zu folgenden Produkten:

Tabelle 11

rac-1-(7β-($R_a$)-3,6,7,8,9-aβ-hexahydro-4H-chinolizin-2-yl)-(B)-2-benzimidazolinon:

| | | | | Smp. °C |
|---|---|---|---|---|
| $R_a$ = o-Chlorphenyl | B = | 5,6-dichlor | HCl-Salz | 281–284 |
| o-Chlorphenyl | | 5,6-H | HCl-Salz | 267–269 |
| o-Chlorphenyl | | 5,6-dimethoxy | HCl-Salz | 225 (Zers.) |
| o-Chlorphenyl | | 5-$(CH_2)_4$-6 | Base | 246–249 |
| p-Chlorphenyl | | 5,6-H | HCl-Salz | 274–277 |
| p-Chlorphenyl | | 5,6-dimethyl | HCl-Salz | 228–230 |
| o-Methylphenyl | | 5,6-H | HCl-Salz | 277–279 |
| o-Methylphenyl | | 5,6-dimethyl | HCl-Salz | 226–230 |
| o-Methylphenyl | | 5,6-dichlor | HCl-Salz | 286–289 |
| phenyl | | 5,6-dimethyl | Base | 259–262 |
| phenyl | | 5,6-dichlor | Base | 293–296 |
| phenyl | | 5,6-dimethoxy | Base | 241–243 |
| 2'-Methoxy-5'-chlor-phenyl | | 5,6-dimethyl | Base | 248–251 |
| o-Fluorphenyl | | 5,6-dimethyl | Base | 260–263 |
| o-Fluorphenyl | | 5,6-dichlor | Base | 298–302 |
| o-Fluorphenyl | | 5,6-difluor | Base | 257–259 |
| o-Fluorphenyl | | 5-$(CH_2)_4$-6 | Base | 238–241 |

## Beispiel 8

3,0 g rac-1-((9aβH)-7β-(o-Chlorphenyl)octahydro-2H-chinolizin-2α-yl)-2-benzimidazolinon (erhalten gem. Beispiel 7a) werden in 30 ml trockenem Monoglym suspendiert und mit 0,41 g Natriumhydrid (50–60% in Mineralöl) versetzt und 1 Stunde bei Raumtemperatur gerührt. Anschließend kühlt man auf 0°C und addiert 1,23 g Methyljodid. Nach 30 Minuten bei Raumtemperatur wird auf 50 ml Wasser gegossen und mit Essigester extrahiert. Die über Magnesiumsulfat getrocknete organische Phase wird eingeengt und das Rohprodukt an Kieselgel mit Methylenchlorid-Methanol chromatographiert. Es resultieren 2,10 g (58%) rac-1-((9aβH)-7β-(o-Chlorphenyl)octahydro-2H-chinolizin-2α-yl)-3-methyl-2-benzimidazolinon. HCl-Salz, Smp. 315–320° C.

## Beispiel 9

0,5 g rac-1-((9aβH)-7β-(o-Chlorphenyl)octahydro-2H-chinolizin-2α-yl)-5,6-dimethyl-2-benzimidazolinon (erhalten gem. Beispiel 7a) werden mit 1,0 g Phosphorpentasulfid in 5 ml Pyridin 3 Stunden auf 150°C erwärmt. Anschließend dampft man das Reaktionsgemisch ein und verteilt den Rückstand zwischen 2N Natronlauge und Chloroform-Isopropanol. Die organische Phase wird mit gesättigter Natriumchlorid-Lösung gewaschen, über Magnesiumsulfat getrocknet und eingedampft. Chromatographie des Rohprodukts an Kieselgel mit Methylenchlorid-Methanol ergibt 0,22 g rac-1-((9aβH)-7β-(o-Chlorphenyl)octahydro-2H-chinolizin-2α-yl)-5,6-dimethyl-2-benzimidazolinthion. Smp. 293–297° C.

Beispiel 10

a) Eine Lösung 7,20 g rac-(9a$\beta$H)-7$\beta$-(o-Fluorphenyl)octahydro-2$\alpha$-($\alpha'$,$\alpha'$,$\alpha'$-trifluor-2-amino-p-toluidino)-2H-chinolizin in 180 ml Methylenchlorid wird mit 4,59 g N,N'-Carbonyl-diimidazol (93%ig) versetzt und über Nacht bei Raumtemperatur gerührt. Das ausgefallene Reaktionsprodukt wird nach 20 Stunden abfiltriert, mit 3 x 100 ml Methylenchlorid gewaschen und anschließend getrocknet. Man erhält 6,45 g (83,5%) kristallines rac-1-[(3a$\beta$H)-7$\beta$-(o-Fluorphenyl)-octahydro-2H-chinolizin-2$\alpha$-yl]-5-(trifluormethyl)-2-benzimidazolinon. Smp. 304 – 306° C.

Wenn anstelle von rac-(9a$\beta$H)-7$\beta$-(o-Fluorphenyl)octahydro-2$\alpha$-($\alpha'$,$\alpha'$,$\alpha'$-trifluor-2-amino-p-toluidino)-2H-chinolizin entsprechend substituierte Phenylendiamine mit N,N'-Carbonyl-diimidazol umgesetzt werden, gelangt man zu folgenden Produkten:

Tabelle 12

rac-1-[(9a$\beta$H)-7$\beta$-($R_a$)octahydro-2H-chinolizin-2$\alpha$-yl]-(B)-2-benzimidazolinon:

| | | | | Smp. °C |
|---|---|---|---|---|
| $R_a$ = | o-Fluorphenyl | B = | 5-chlor | 283–285 (Base); 298–304 (HCl-Salz) |
| | o-Chlorphenyl | | 5-chlor | 223–230 (Base) |
| | o-Chlorphenyl | | 5-trifluormethyl | 257–259 (Base); 240–245 (HCl-Salz) |
| | o-Chlorphenyl | | 5,6-dichlor | 300–305 (HCl-Salz) |
| | o-Fluorphenyl | | 5,6-dichlor | >250 (HCl-Salz) |

b) Das als Ausgangsmaterial in Beispiel 10a verwendete rac-(9a$\beta$H)-7$\beta$-(o-Fluorphenyl)octahydro-2$\alpha$-($\alpha'$,$\alpha'$,$\alpha'$-trifluor-2-amino-p-toluidino)-2H-chinolizin kann wie folgt erhalten werden:

25,7 g rac-(9a$\beta$H)-7$\beta$-(o-Fluorphenyl)octahydro-2$\alpha$-($\alpha'$,$\alpha'$,$\alpha'$-trifluor-2-nitro-p-toluidino)-2H-chinolizin werden in 0,9 l Tetrahydrofuran und 0,9 l Methanol mit 13 g Raney-Nickel während 20 Stunden bei Raumtemperatur und Atmosphärendruck hydriert. Anschließend saugt man vom Katalysator ab und dampft das Filtrat ein. Es resultieren 23,6 g (99%) kristallines rac-(9a$\beta$H)-7$\beta$-(o-Fluorphenyl)octahydro-2$\alpha$-($\alpha'$,$\alpha'$,$\alpha'$-trifluor-2-amino-p-toluidino)-2H-chinolizin, das gegebenenfalls aus Alkohol-Essigester umkristallisiert werden kann. Smp. 133 – 138° C.

Werden anstelle von rac-(9a$\beta$H)-7$\beta$-(o-Fluorphenyl)octahydro-2$\alpha$-($\alpha'$,$\alpha'$,$\alpha'$-trifluor-2-nitro-p-toluidino)-2H-chinolizin entsprechend substituierte Nitroverbindungen reduziert, so gelangt man zu folgenden Produkten:

Tabelle 13

rac-(9a$\beta$H)-7$\beta$-($R_a$) octahydro-2$\alpha$-(B)-2H-chinolizin:

| | | | | Smp. °C |
|---|---|---|---|---|
| $R_a$ = | o-Fluorphenyl | B = | 2-amino-4-chlor-anilino | 137–141 |
| | o-Fluorphenyl | | 2-amino-4,5-dichlor-anilino | 164–166 |
| | o-Chlorphenyl | | 2-amino-4-chlor-anilino | 170–172 |
| | o-Chlorphenyl | | 2-amino-4,5-dichlor-anilino | 148–150 |
| | o-Chlorphenyl | | $\alpha'$,$\alpha'$,$\alpha'$-trifluor-2-amino-p-toluidino | 151–153 |

c) Das als Ausgangsmaterial in Beispiel 10b eingesetzte rac-(9a$\beta$H)-7$\beta$-(o-Fluorphenyl)octahydro-2$\alpha$)($\alpha'$,$\alpha'$,$\alpha'$-trifluor-2-nitro-p-toluidino)-2H-chinolizin kann wie folgt erhalten werden:

20,0 g rac-(9a$\beta$H)-7$\beta$-(o-Fluorphenyl)-2$\alpha$-(amino-octahydro-2H-chinolizin werden in 300 ml Cyclohexanol (99%) gelöst, mit 8,62 g Natriumcarbonat (wasserfrei) versetzt und auf 160° C erwärmt. Bei dieser Temperatur tropft man innerhalb von 3 Stunden eine Lösung von 20,0 g 4-Chlor-3-nitro-benzotrifluorid

in 200 ml Cyclohexanol (>99%) zu. Anschließend wird die Reaktionslösung für weitere 4 Stunden bei 160°C gehalten. Nach Abkühlen auf Raumtemperatur wird vom anorganischen Rückstand abfiltriert und das Filtrat im Vakuum eingedampft. Den Abdampfrückstand löst man in 500 ml heißem Essigester, setzt 5 g Aktivkohle zu und kocht 5 Minuten am Rückfluß. Danach wird heiß durch Hyflo Super Cel filtriert, das Filtrat auf 200 ml eingeengt und 100 ml n-Hexan zugesetzt. Es kristallisieren 22,3 g rac-(9a$\beta$H)-7$\beta$-(o-Fluorphenyl)octahydro-2$\alpha$-($\alpha'$,$\alpha'$,$\alpha'$-trifluor-2-nitro-p-toluidino)-2H-chinolizin. Smp. 163—165°C. Durch Chromatographie der Mutterlauge an 250 g Kieselgel mit n-Hexan/Essigester = 4 : 1 werden nochmals 5,45 g Produkt erhalten. Gesamtausbeute 78,8%.

Falls anstelle von rac-(9a$\beta$H)-7$\beta$-(o-Fluorphenyl)-2$\alpha$-(amino)octahydro-2H-chinolizin entsprechend substituierte Amino-chinolizidine mit entsprechend substituierten o-Chlor-nitrobenzolen umgesetzt werden, so erhält man folgende Produkte:

Tabelle 14

rac-(9a$\beta$H)-7$\beta$-($R_a$)octahydro-2$\alpha$-(B)-2H-chinolizin:

| | | Smp. °C |
|---|---|---|
| $R_a$ = o-Fluorphenyl | B = 2-nitro-4-chlor-anilino | 185—187 |
| o-Fluorphenyl | 2-nitro-4,5-dichlor-anilino | 202—203 |
| o-Chlorphenyl | 2-nitro-4-chlor-anilino | 183—185 |
| o-Chlorphenyl | 2-nitro-4,5-dichlor-anilino | 197—199 |
| o-Chlorphenyl | $\alpha'$,$\alpha'$,$\alpha'$-trifluor-2-nitro-p-toluidino | 154—155 |

d) Das als Ausgangsmaterial in Beispiel 10c) eingesetzte rac-(9a$\beta$H)-7$\beta$-(o-Fluorphenyl)-2$\alpha$-(amino)octahydro-2H-chinolizin kann wie folgt erhalten werden:

84,3 g rac-(9a$\beta$H)-7$\beta$-(o-Fluorphenyl-octahydro-2H-chinolizin-2-on (vgl. Beispiel 1b) Tabelle 3) werden in 1700 ml Methanol mit 47,4 g Hydroxylamin-Hydrochlorid und 94,3 g Kaliumcarbonat (wasserfrei) während 1½ Stunden auf Rückfluß erhitzt. Anschließend destilliert man das Methanol ab und verteilt den Rückstand zwischen 750 ml Wasser, 600 ml Chloroform und 150 ml Isopropanol. Die wäßrige Phase wird zweimal mit je 250 ml eines Gemisches aus 200 ml Chloroform und 50 ml Isopropanol nachextrahiert, die gesammelten organischen Phasen über Magnesiumsulfat getrocknet und anschließend eingedampft. Es resultieren 85,3 g Oxim, welches in 1,5 l Tetrahydrofuran, 0,5 l Alkohol sowie 1 l (5% g/g Ammoniak in Alkohol) gelöst und mit 45 g Raney-Nickel während 30 Stunden hydriert wird (Raumtemperatur, Atmosphärendruck). Danach wird vom Katalysator abfiltriert und das Filtrat eingedampft. Aus dem Rückstand können durch Lösen in 400 ml Alkohol und Addieren von 130 ml 5N Chlorwasserstoff in Alkohol 53,5 g des Dihydrochlorid-Salzes von rac-(9a$\beta$H)-7$\beta$-(o-Fluorphenyl)-2$\alpha$-(amino)octahydro-2H-chinolizin auskristallisiert werden. Smp. 299—302°C. Das diastereomere rac-(9a$\beta$H)-7$\beta$-(o-Fluorphenyl)-2$\beta$-(amino)octahydro-2H-chinolizin ist als Dihydrochlorid in der Mutterlauge gelöst.

Wenn anstelle von rac-(9a$\beta$H)-7$\beta$-(o-Fluorphenyl)octahydro-2H-chinolizin-2-on entsprechend substituierte Chinolizidinone aminiert werden, so erhält man folgende Produkte:

Tabelle 15

rac-(9a$\beta$H)-7$\beta$-($R_a$)-2$\alpha$-(amino)octahydro-2H-chinolizin:

$R_a$ = o-Chlorphenyl    Smp. °C (· 2 HCl): 293—296°C

## Beispiel 11

Eine Lösung von 7,20 g rac-(9a$\beta$H)-7$\beta$-(o-Fluorphenyl)octahydro-2$\alpha$-($\alpha'$,$\alpha'$,$\alpha'$-trifluor-2-amino-p-toluidino)-2H-chinolizin (Beispiel 10b) in 180 ml Methylenchlorid wird mit 4,75 g N,N'-Thiocarbonyl-diimidazol versetzt und über Nacht bei Zimmertemperatur gerührt. Nach 20 Stunden wird das ausgefallene Reaktionsprodukt abfiltriert, mit 2 × 100 ml Methylenchlorid gewaschen und anschließend getrocknet. Man erhält 5,80 g (73,0%) kristallines rac-1-[(9a$\beta$H)-7$\beta$-(o-Fluorphenyl)octahydro-2H-chinolizin-2$\alpha$-yl]-5-(trifluormethyl)-2-benzimidazolinthion. Smp. 308—309°C.

Wenn anstelle von rac-(9aβH)-7β-(o-Fluorphenyl)octahydro-2α-(α',α',α'-trifluor-2-amino-p-toluidino)-2H-chinolizin entsprechend substituierte Phenylendiamine mit N,N'-Thiocarbonyl-diimidazol umgesetzt werden, gelangt man zu folgenden Produkten:

Tabelle 16

rac-1-[(9aβH)-7β-($R_a$)octahydro-2H-chinolizin-2α-yl]-(B)-2-benzimidazolinthion:

| | | Smp. °C |
|---|---|---|
| $R_a$ = o-Fluorphenyl | B = 5-chlor | >300 |
| o-Fluorphenyl | 5,6-dichlor | >310 |
| o-Chlorphenyl | 5-chlor | 264–267 |
| o-Chlorphenyl | 5,6-dichlor | >295 |

Beispiel 12

Eine Lösung von 5,80 g rac-(9aβH)-2α-(2-Amino-4,5-dichlor-anilino)-7β-(o-chlorphenyl)octahydro-2H-chinolizin (Beispiel 10b)) in 60 ml m-Xylol werden mit 20 ml Orthoessigsäuretriäthylester während 6 Stunden am Rückfluß gekocht. Nach Abkühlen der Reaktionslösung gießt man auf kalte 2N Natronlauge und extrahiert 2mal mit je 300 ml Methylenchlorid. Die über Natriumcarbonat (wasserfrei) getrocknete organische Phase wird anschließend eingedampft. Den Rückstand löst man in 50 ml 5N Salzsäure in Alkohol und erwärmt während 1 Stunde auf Rückfluß. Das ausgefallene Dihydrochlorid des Reaktionsprodukts wird abfiltriert und getrocknet. Es resultieren 5,60 g des Dihydrochlorid-Salzes von rac-(9aβH)-7β-(o-Chlorphenyl)-2α-(5,6-dichlor-2-methyl-1-benzimidazolyl)octahydro-2H-chinolizin. Smp. 231 – 236° C. Ausbeute 77%.

Falls anstelle von rac-(9aβH)-2α-(2-Amino-4,5-dichloranilino)-7β-(o-chlorphenyl)octahydro-2H-chinolizin entsprechend substituierte Phenylendiamine mit Orthoessigsäuretriäthylester umgesetzt werden, können folgende Produkte erhalten werden:

Tabelle 17

rac-(9aβH)-7β-($R_a$)-2α-((B)-2-methyl-1-benzimidazolyl)-octahydro-2H-chinolizin · 2 HCl:

| | | Smp. °C |
|---|---|---|
| $R_a$ = o-Chlorphenyl | B = 5-chlor | 240–245 |

Beispiel 13

2,70 g eines (1 : 1)-Gemisches von rac-(9aβH)-2-Phenyl-7β-(o-chlorphenyl)-3,4,6,7,8,9-hexahydro-2H-chinolizin und rac-(9aβH)-2-Phenyl-7β-(o-chlorphenyl)-1,4,6,7,8,9-hexahydro-2H-chinolizin werden mit 70 ml Wasser und 14 ml Schwefelsäure (98%) 15 Stunden unter Rückfluß gekocht. Nach Abkühlen der Reaktionslösung wird auf gesättigte Kaliumcarbonat/Wasser-Lösung gegossen, mit 200 ml Wasser verdünnt und dreimal mit je 200 ml Essigester extrahiert. Die vereinigten Essigester-Extrakte trocknet man über Magnesiumsulfat und dampft ein. Das Rohprodukt (2,35 g) chromatographiert man über 100 g Kieselgel mit Essigester als Eluens. In einer ersten Fraktion werden 1,55 g Eduktgemisch zurückgewonnen; aus einer weiteren Fraktion können 0,15 g (Ausbeute 3,5%) rac-(9aβH)-2α-Phenyl-7β-(o-chlorphenyl)octahydro-2H-chinolizin-2-ol erhalten werden. Smp. (HCl-Salz): 265 – 275° C.

Das als Ausgangsmaterial eingesetzte Doppelbindungsisomerengemisch kann wie folgt erhalten werden:

5,00 g rac-(9aβH)-2β-Phenyl-7β-(o-chlorophenyl)octahydro-2H-quinolizin-2-ol (Beispiel 1a, Tabelle 2) löst man in 25 ml Pyridin und addiert 2,1 ml Thionylchlorid. Nach zweistündigem Rühren bei Zimmertemperatur wird im Vakuum eingedampft und anschließend zwischen Chloroform und Wasser verteilt. Die über Magnesiumsulfat getrocknete organische Phase dampft man ein und filtriert das Rohprodukt (3,90 g) mit Essigester über Aluminiumoxid (200 g, Aktivität III). Auf diese Weise können 2,75 g eines (1 : 1)-Gemisches von rac-(9aβH)-2-Phenyl-7β-(o-chlorphenyl)-3,4,6,7,8,9-hexahydro-2H-chinolizin und rac-(9aβH)-2-Phenyl-7β-(o-chlorphenyl)-1,4,6,7,8,9-hexahydro-2H-chinolizin eluiert werden. NMR($CDCl_3$): 5,93 + 6,08 ppm (je 2 breite Singuletts im Verhältnis 1 : 1, zusammen 1 Proton).

Beispiel 14

0,068 g Natriumhydrid (50% in Mineralöl) in 10 ml 1,2-Dimethoxyäthan suspendiert, werden mit 0,160 g Benzimidazolinon und anschließend mit 0,500 g eines (1 : 1)-Gemisches von rac-(9aβH)-7β-(o-Chlorphenyl)-octahydro-2H-chinolizin-2α-yl-p-toluolsulfonat und rac-(9aβH)-7β-(o-Chlorphenyl)octahydro-2H-chinolizin-2β-yl-p-toluolsulfonat in 10 ml 1,2-Dimethoxyäthan versetzt und anschließend 45 Stunden auf Rückfluß erhitzt. Nach Abkühlen verteilt man zwischen Chloroform und Wasser, trocknet die organische Phase über Magnesiumsulfat und dampft ein. Das erhaltene Rohprodukt (0,450 g) chromatographiert man an 50 g Kieselgel mit Chloroform (1% Methanol) als Eluens und erhält 0,040 g (Ausbeute 9%) rac-1-((9aβH)-7β-(o-Chlorphenyl)-octahydro-2H-chinolizin-2α-yl)-2-benzimidazolinone. Smp. (HCl-Salz): 315—317°C.

Das als Ausgangsmaterial verwendete (1 : 1)-Gemisch diastereomerer p-Toluolsulfonate kann wie folgt hergestellt werden:

5,00 g rac-(9aβH)-7β-(o-Chlorphenyl)-octahydro-2H-chinolizin-2-on (Beispiel 1b) in 50 ml Methanol werden mit 0,7 g Natriumborhydrid versetzt und 45 Minuten bei Zimmertemperatur gerührt. Anschließend verteilt man zwischen Wasser und Essigester, trocknet die organische Phase über Magnesiumsulfat und engt ein. Das erhaltene Rohprodukt wird über 200 g Kieselgel mit Diäthyläther chromatographiert und das Eluat eingedampft: 5,0 g als Gemisch diastereomerer Alkohle. Das Gemisch der Alkohole löst man in 50 ml Pyridin und setzt 7,3 g p-Toluolsulfonsäurechlorid zu. Nach zweistündigem Rühren bei Zimmertemperatur wird eingedampft, der Rückstand zwischen 2-n Natronlauge und Essigester verteilt und nach Trocknen der organischen Phase eingeengt. Durch Chromatographie an 100 g Kieselgel mit Methylenchlorid (1% Methanol) können 2,20 g eines (1 : 1)-Gemisches von rac-(9aβH)-7β-(o-Chlorphenyl)-octahydro-2H-chinolizin-2β-yl-p-toluolsulfonat und rac-(9aβH)-7β-(o-Chlorphenyl)-octahydro-2H-chinolizin-2α-yl-p-toluolsulfonat (NMR($CDCl_3$): 2 Singuletts 2,33+2,43 ppm) sowie 5,80 g rac-(9aβH)-7β-(o-Chlorphenyl)-octahydro-2H-chinolizin-2α-yl-p-toluolsulfonat (NMR($CDCl_3$): 1 Singulett 2,43 ppm) erhalten werden.

Beispiel 15

| Tabletten | pro Tablette |
|---|---|
| (−)-(2S,7R,9aR)-2-tert.-Butyl-7-(2,4-dichlorphenyl)octahydro-2H-chinolizin-2-ol · HCl | 100 mg |
| Lactose | 202 mg |
| Maisstärke | 80 mg |
| Hydrolysierte Maisstärke | 20 mg |
| Calciumstearat | 8 mg |
| Totalgewicht | 410 mg |

Der Wirkstoff, die Lactose, die Maisstärke und die hydrolysierte Maisstärke werden gemischt und mit Wasser zu einer zähen Paste granuliert. Diese Paste wird durch ein Sieb passiert und anschließend bei 45°C über Nacht getrocknet. Das getrocknete Granulat wird durch ein Sieb passiert und anschließend mit dem Calciumstearat vermischt. Die erhaltene Mischung wird zu Tabletten von einem Gewicht von 410 mg und mit einem Durchmesser von etwa 10 mm gepreßt.

Beispiel 16

| Tabletten | pro Tablette |
|---|---|
| (+)-(2-Butyloctahydro-7-phenyl-2H-chinolizin-2-yl-acetat · HCl | 10,0 mg |
| Lacatose | 129,0 mg |
| Maisstärke | 50,0 mg |
| Gelatinierte Maisstärke | 8,0 mg |
| Calciumstearat | 3,0 mg |
| Totalgewicht | 200,0 mg |

Der Wirkstoff, die Lactose, die Maisstärke und die gelatinierte Maisstärke werden innig miteinander vermischt. Die Mischung wird durch eine Zerkleinerungsmaschine passiert und anschließend mit Wasser zu einer dicken Paste befeuchtet. Die feuchte Masse wird durch ein Sieb passiert. Das feuchte Granulat wird bei 45° C getrocknet. Das getrocknete Granulat wird mit dem Calciumstearat gründlich vermischt. Das Granulat wird nun zu Tabletten von einem Gewicht von 200 mg und einem Durchmesser von etwa 8 mm gepreßt.

Beispiel 17

| Tabletten | pro Tablette |
|---|---|
| rac-1-((9a$\beta$H)-7$\beta$-(o-Fluorphenyl)-octahydro-2H-chinolizin-2$\alpha$-yl)-5-chlor-2-benzimidazolin · HCl | 10,0 mg |
| Lactose | 129,0 mg |
| Maisstärke | 50,0 mg |
| Gelatinierte Maisstärke | 8,0 mg |
| Calciumstearat | 3,0 mg |
| Totalgewicht | 200,0 mg |

Der Wirkstoff, die Lactose, die Maisstärke und die gelatinierte Maisstärke werden innig miteinander vermischt. Die Mischung wird durch eine Zerkleinerungsmaschine passiert und anschließend mit Wasser zu einer dicken Paste befeuchtet. Die feuchte Masse wird durch ein Sieb passiert. Das feuchte Granulat wird bei 45° C getrocknet. Das getrocknete Granulat wird mit dem Calciumstearat gründlich vermischt. Das Granulat wird nun zu Tabletten von einem Gehalt von 200 mg und einem Durchmesser von etwa 8 mm gepreßt.

Beispiel 18

| Tabletten | pro Tablette |
|---|---|
| (–)-(2S,7R,9aR)-2-tert.-Butyl-7-(2,4-dichlorphenyl)octahydro-2H-chinolizin-2-ol · HCl | 20,0 mg |
| Milchzucker | 115,0 mg |
| Maisstärke | 61,0 mg |
| Talk | 3,4 mg |
| Magnesiumstearat | 0,6 mg |
| Totalgewicht | 200,0 mg |

Die Ingredienzien werden innig miteinander vermischt und zu Tabletten von je 200 mg gepreßt. Anschließend werden sie mit Äthylcellulose und Carbowax überzogen.

Beispiel 19

| Kapseln | pro Kapsel |
|---|---|
| rac-1-[(9a$\beta$H)-7$\beta$-(o-Fluorphenyl)octahydro-2H-chinolizin-2$\alpha$-yl]-5-chlor-2-benzimidazolon · HCl | 10,0 mg |
| Lactose | 175,0 mg |
| Maisstärke | 30,0 mg |
| Talk | 5,0 mg |
| Totalgewicht | 220,0 mg |

Der Wirkstoff, die Lactose und die Maisstärke werden innig miteinander vermischt und durch eine Zerkleinerungsmaschine passiert. Die Mischung wird nun mit dem Talk gründlich vermischt und in harte Gelatinekapseln gefüllt.

## Beispiel 20

| Kapseln | pro Kapsel |
|---|---|
| (+)-2-Butyloctahydro-7-phenyl-2H-chinolizin-2-yl-acetat · HCl | 10,0 mg |
| Lactose | 175,0 mg |
| Maisstärke | 30,0 mg |
| Talk | 5,0 mg |
| Totalgewicht | 220,0 mg |

Der Wirkstoff, die Lactose und die Maisstärke werden innig miteinander vermischt und durch eine Zerkleinerungsmaschine passiert. Die Mischung wird nun mit dem Talk gründlich vermischt und in harte Gelatinekapseln gefüllt.

## Beispiel 21

Parenterale Zubereitungsform (0,2 mg)

Jede 1 ml Ampulle enthält:

| | |
|---|---|
| rac-1-[(9a$\beta$H)-7$\beta$-(o-Fluorphenyl)-octahydro-2H-chinolizin-2$\alpha$-yl]-5-chlor-2-benzimidazolon · HCl | 0,204 mg (2% Überschuß) |
| Glucose für Injektionszwecke | 40,0 mg |
| Wasser für Injektionszwecke q.s. ad | 1,0 ml |

2,04 g Wirkstoff werden mit 400 g Glucose versetzt, in Wasser für Injektionszwecke gelöst und mit Wasser für Injektionszwecke auf ein Volumen von 10 000 ml ergänzt. Die Lösung wird entweder steril filtriert, in farblose Ampullen abgefüllt, mit Stickstoff begast und versiegelt oder in farblose Ampullen abgefüllt, mit Stickstoff begast, versiegelt und anschließend 30 Minuten mit strömendem Dampf sterilisiert oder bei 120° C autoklaviert.

## Beispiel 22

Parenterale Zubereitungsform (0,5 mg)

Jede 1 ml Ampulle enthält:

| | |
|---|---|
| (+)-2-Butyloctahydro-7-phenyl-2H-chinolizin-2-yl-acetat · HCl | 0,510 mg (2% Überschuß) |
| Glucose für Injektionszwecke | 40,0 mg |
| Wasser für Injektionszwecke q.s. ad | 1,0 ml |

5,10 g Wikrstoff werden mit 400 g Glucose versetzt, in Wasser für Injektionszwecke gelöst und mit Wasser für Injektionszwecke auf ein Volumen von 10 000 ml ergänzt. Die Lösung wird entweder steril filtriert, in farblose Ampullen abgefüllt, mit Stickstoff begast und versiegelt oder in farblose Ampullen abgefüllt, mit Stickstoff begast, versiegelt und anschließend 30 Minuten mit strömendem Dampf sterilisiert oder bei 120° C autoklaviert.

**Patentansprüche für die Vertragsstaaten BE, CH, DE, FR, GB, IT, LU, NL, SE**

1. Phenyl-chinolizidine der allgemeinen Formel

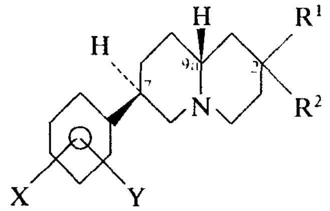

(I)

worin X, Y, $R^1$ und $R^2$ folgendes bedeuten:

X: Wasserstoff, Fluor, Chlor, $C_{1-6}$-Alkoxy, $C_{1-6}$-Alkyl oder Trifluormethyl,
Y: Wasserstoff, Fluor, Chlor, $C_{1-6}$-Alkoxy oder $C_{1-6}$-Alkyl,
$R^1$: Hydroxy, $C_{1-6}$-Alkoxy, Acyloxy oder, falls $R^2$ die nachstehend definierte Gruppe A darstellt, Wasserstoff,
$R^2$: $C_{1-6}$-Alkyl, gegebenenfalls durch Halogen, $C_{1-6}$-Alkoxy oder Trifluormethyl substituiertes Phenyl, oder (falls $R^1$ = Wasserstoff) eine Gruppe A mit der Bedeutung

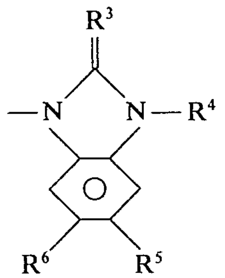

oder

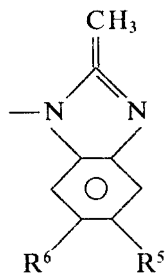

A

mit folgender Bedeutung für $R^3 - R^6$:

$R^3$: Sauerstoff oder Schwefel,
$R^4$: Wasserstoff oder $C_{1-6}$-Alkyl,
$R^5$ und $R^6$ unabhängig voneinander: Wasserstoff, Methyl, Fluor, Chlor, Methoxy, Trifluormethyl oder zusammen $-(CH_2)_4-$

in Form des Racemats oder der Enantiomeren, sowie Säureadditionssalze solcher Verbindungen.

2. Verbindungen gemäß Anspruch 1, worin $R^2$ $C_{1-6}$-Alkyl, gegebenenfalls durch Halogen, $C_{1-6}$-Alkoxy oder Trifluormethyl substituiertes Phenyl, oder (falls $R^1$ = Wasserstoff) eine Gruppe A mit der Bedeutung

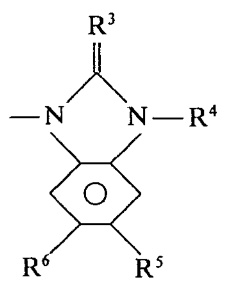

darstellt, wobei $R^3$ und $R^4$ dasselbe wie in Anspruch 1 bedeuten und $R^5$ und $R^6$ unabhängig voneinander Wasserstoff, Methyl, Chlor oder Methoxy darstellen.

3. Verbindungen gemäß Anspruch 1 der allgemeinen Formel

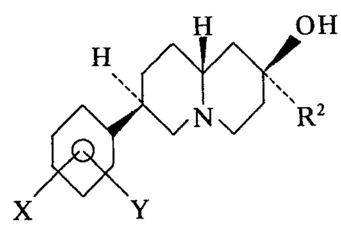

(II-1)

worin X, Y und $R^2$ dasselbe wie in Anspruch 1 bedeuten,

in Form des Racemats oder der Enantiomeren, sowie Säureadditionssalze solcher Verbindungen.

4. Verbindungen gemäß Anspruch 3, worin $R^2$ verzweigtes $C_{3-6}$-Alkyl, X Fluor oder Chlor und Y Wasserstoff, Fluor oder Chlor darstellen, wobei X und Y in ortho- und/oder para-Stellung stehen.

5. Eine Verbindung gemäß Anspruch 4, worin $R^2$ tert.-Butyl, X o-Chlor und Y p-Chlor bedeutet.

6. Eine Verbindung gemäß Anspruch 3, worin $R^2$ tert.-Butyl, X p-Trifluormethyl und Y Wasserstoff bedeutet.

7. Verbindungen gemäß Anspruch 1 der allgemeinen Formel

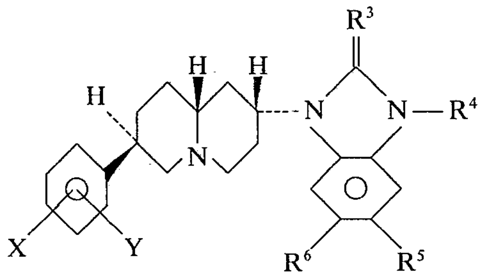

(III-1)

worin X, Y sowie $R^3-R^6$ dasselbe wie in Anspruch 1 bedeuten,

in Form des Racemats oder der Enantiomeren, sowie Säureadditionssalze solcher Verbindungen.

8. Verbindungen gemäß Anspruch 7, worin X Wasserstoff, o-Fluor oder o-Chlor, Y Wasserstoff, $R^3$ Sauerstoff oder Schwefel, $R^4$ Wasserstoff, $R^5$ Fluor, Chlor oder Trifluormethyl und $R^6$ Wasserstoff, Chlor oder Fluor darstellten.

9. Eine Verbindung gemäß Anspruch 8, worin X o-Chlor, Y Wasserstoff, $R^3$ Sauerstoff, $R^4$ sowie $R^6$ Wasserstoff und $R^5$ Trifluormethyl bedeuten.

10. Eine Verbindung gemäß Anspruch 8, worin X o-Fluor, Y Wasserstoff, $R^3$ Sauerstoff, $R^4$ sowie $R^6$ Wasserstoff und $R^5$ Chlor bedeuten.

11. Eine Verbindung gemäß Anspruch 7, worin X, Y und $R^3-R^6$ eine der folgenden Bedeutungen aufweisen:

a) X=o-Cl; Y, $R^4$, $R^5$, $R^6$=H; $R^3$=O
b) X=o-Cl; Y, $R^4$=H; $R^3$=O; $R^5$, $R^6$=$CH_3$
c) X=o-Cl; Y, $R^4$=H; $R^3$=O; $R^5$, $R^6$=Cl
d) X=o-Cl; Y, $R^5$, $R^6$=H; $R^3$=O; $R^4$=$CH_3$
f) X=o-Cl; Y, $R^4$, $R^6$=H; $R^5$=Cl; $R^3$=O
g) X=o-Cl; Y, $R^4$, $R^6$=H; $R^5$=Cl; $R^3$=S
h) X=o-Cl; Y, $R^4$=H; $R^3$=S; $R^5$, $R^6$=Cl
i) X=o-Cl; Y, $R^4$=H; $R^3$=O; $R^5$ und $R^6$ zusammen $-(CH_2)_4-$
j) X=o-F; Y, $R^4$=H; $R^3$=O; $R^5$, $R^6$=$CH_3$
k) X=o-F; Y, $R^4$=H; $R^3$=O; $R^5$, $R^6$=Cl
l) X=o-F; Y, $R^4$=H; $R^3$=S; $R^5$, $R^6$=Cl
m) X=o-F; Y, $R^4$=H; $R^3$=O; $R^5$ und $R^6$ zusammen $-(CH_2)_4-$
n) X=o-F; Y, $R^4$, $R^6$=H; $R^5$=$CF_3$; $R^3$=O
o) X=o-F; Y, $R^4$, $R^6$=H; $R^5$=$CF_3$; $R^3$=S
q) X=o-F; Y, $R^4$, $R^6$=H; $R^5$=Cl; $R^3$=S.

12. Verbindungen gemäß Anspruch 7, worin $R^5$ und $R^6$ unabhängig voneinander Wasserstoff, Methyl, Chlor oder Methoxy bedeuten.

13. Verbindungen gemäß Anspruch 1 der allgemeinen Formel

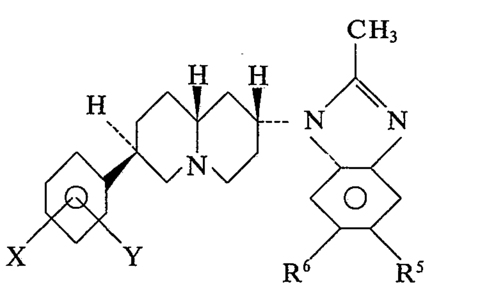

(III-2)

worin X, Y, $R^5$ und $R^6$ dasselbe wie in Anspruch 1 bedeuten,

in Form des Racemats oder der Enantiomeren, sowie Säureadditionssalze solcher Verbindungen.

14. Verbindungen gemäß Anspruch 1 der allgemeinen Formel

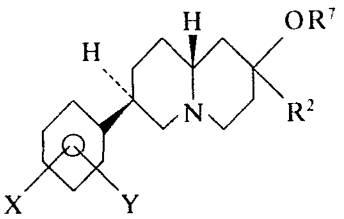

(IV)

worin X, Y und $R^2$ dasselbe wie in Anspruch 1 bedeuten und $R^7$ nieder-Alkyl oder Acyl darstellt,

in Form des Racemats oder der Enantiomeren, sowie Säureadditionssalze solcher Verbindungen.

15. Eine Verbindung gemäß Anspruch 14, worin X und Y Wasserstoff, $OR^7$ $\beta$-orientiertes Acetoxy und $R^2$ n-Butyl bedeuten.

16. Eine Verbindung gemäß Anspruch 14, worin X und Y Wasserstoff, $OR^7$ $\beta$-orientiertes Trifluoracetoxy, und $R^2$ tert.Butyl bedeuten.

17. Eine Verbindung gemäß Anspruch 14, worin X o-Chlor, $OR^7$ $\alpha$-orientiertes Acetoxy und $R^2$ Phenyl bedeuten.

18. Phenyl-chinolizidine gemäß einem der Ansprüche 1—17 als pharmazeutische Wirkstoffe, insbesondere als antipsychotische, antiemetische und/oder analgetische Wirkstoffe.

19. Verfahren zur Herstellung von Phenyl-chinolizidinen der allgemeinen Formel

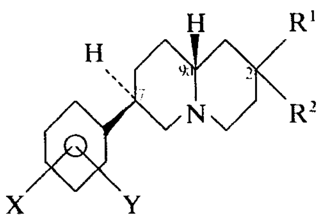

(I)

worin X, Y, $R^1$ und $R^2$ folgendes bedeuten:

X: Wasserstoff, Fluor, Chlor, $C_{1-6}$-Alkoxy, $C_{1-6}$-Alkyl oder Trifluormethyl,

Y: Wasserstoff, Fluor, Chlor, $C_{1-6}$-Alkoxy oder $C_{1-6}$-Alkyl,

$R^1$: Hydroxy, $C_{1-6}$-Alkoxy, Acyloxy oder, falls $R^2$ die nachstehend definierte Gruppe A darstellt, Wasserstoff,

$R^2$: $C_{1-6}$-Alkyl, gegebenenfalls durch Halogen, $C_{1-6}$-Alkoxy oder Trifluormethyl substituiertes Phenyl, oder (falls $R^1$ = Wasserstoff) eine Gruppe A mit der Bedeutung

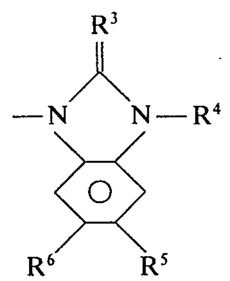

oder

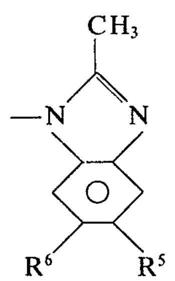

A

mit folgender Bedeutung für $R^3$—$R^6$:

$R^3$: Sauerstoff oder Schwefel,

$R^4$: Wasserstoff oder $C_{1-6}$-Alkyl,

$R^5$ und $R^6$ unabhängig voneinander: Wasserstoff, Methyl, Fluor, Chlor, Methoxy, Trifluormethyl oder zusammen $-(CH_2)_4-$,

in Form des Racemats oder der Enantiomeren, sowie von Säureadditionssalzen solcher Verbindungen, welche Verfahren dadurch gekennzeichnet sind, daß man

1) zur Herstellung von Verbindungen der allgemeinen Formel

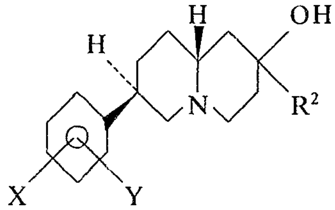

(II)

worin X, Y und $R^2$ dasselbe wie oben bedeuten,

entweder

a) ein Keton der allgemeinen Formel

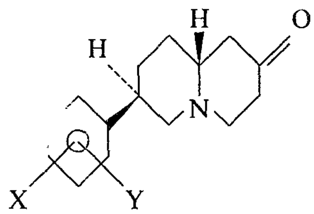

(V)

worin X und Y dasselbe wie oben bedeuten,

mit einer metallorganischen, den Rest $R^2$ liefernden Verbindung umsetzt, und gegebenenfalls das erhaltene Gemisch der Diast ;omeren auftrennt, oder

b) an die $\Delta^1$- bzw. $\Delta^2$-Doppelbindung einer Verbindung der allgemeinen Formel

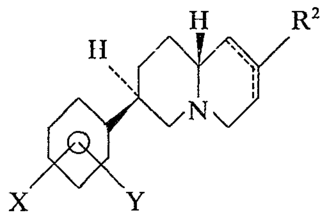

(VI)

worin X, Y und $R^2$ dasselbe wie oben bedeuten und die gestrichelte Bindung eine Doppelbindung in 1,2- oder 2,3-Stellung darstellt,

durch Umsetzung mit einem Hydratisierungsmittel Wasser anlagert,

oder daß man

2) zur Herstellung von Verbindungen der allgemeinen Formel

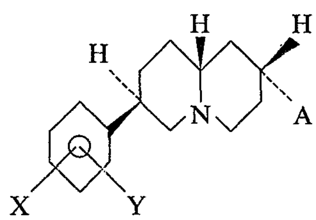

(III)

worin X, Y und A und $R^3$—$R^6$ dasselbe wie oben bedeuten,

eine Verbindung der allgemeinen Formel

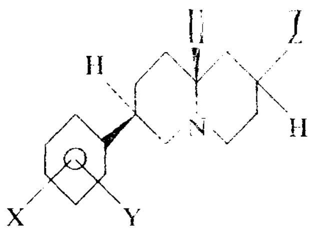

(VII)

worin X und Y dasselbe wie oben bedeuten und Z eine abspaltbare Gruppe darstellt,

mit einer Verbindung der allgemeinen Formel

$$H-A \qquad (VIII)$$

worin A dasselbe wie oben bedeutet,

umsetzt, oder daß man

3) zur Herstellung einer Verbindung der allgemeinen Formel

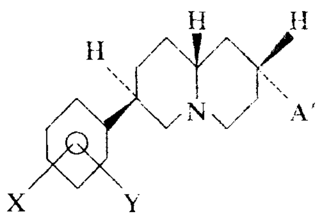

(III-3)

worin X und Y dasselbe wie oben bedeuten und A' wie A ist, wobei jedoch $R^3$ Sauerstoff darstellt,

eine Verbindung der allgemeinen Formel

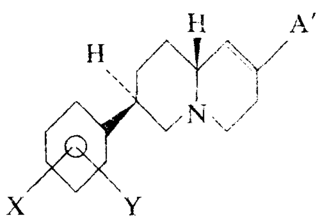

(IX)

worin X, Y und A' dasselbe wie oben bedeuten,

katalytisch hydriert, oder daß man

4) zur Herstellung einer Verbindung der allgemeinen Formel

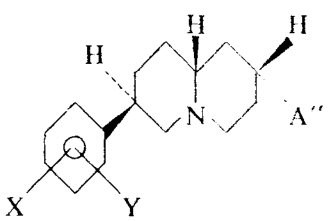

(III-4)

worin X, Y dasselbe wie oben bedeuten und A" wie A ist, wobei jedoch $R^3$ Sauerstoff und $R^4$ Wasserstoff darstellt,

ein Phenylen-diamin der allgemeinen Formel

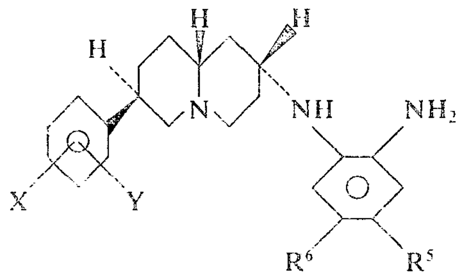

(X)

worin X, Y, $R^5$ und $R^6$ dasselbe wie oben bedeuten,

mit einem Cyclisierungsmittel der allgemeinen Formel

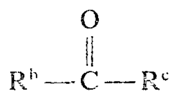

(XI)

worin $R^b$ und $R^c$ Halogen, Amino oder 1-Imidazolyl bedeuten,

oder mit Essigsäure oder einem Orthoessigsäureester kondensiert, oder daß man

5) zur Herstellung von Verbindungen der allgemeinen Formel

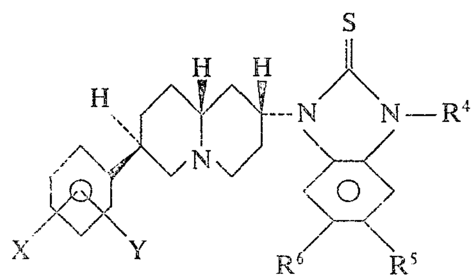

(III-5)

worin X, Y sowie $R^4-R^6$ dasselbe wie oben bedeuten,

entweder

a) eine entsprechende Oxo-Verbindung der allgemeinen Formel

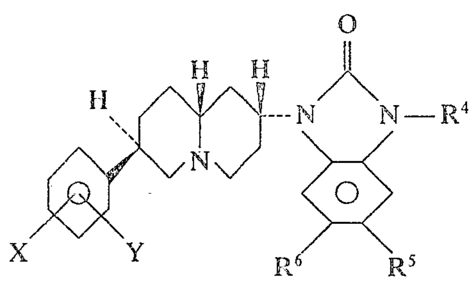

(III-6)

worin X, Y sowie $R^4-R^6$ dasselbe wie oben bedeuten,

mit $P_2S_5$ umsetzt, oder

b) ein Phenylen-diamin der obigen allgemeinen Formel X mit Thiophosgen, Thiocarbonyldiimidazol oder $CS_2$ umsetzt und anschließend gegebenenfalls N-alkyliert,

oder daß man

6) zur Herstellung von Verbindungen der allgemeinen Formel

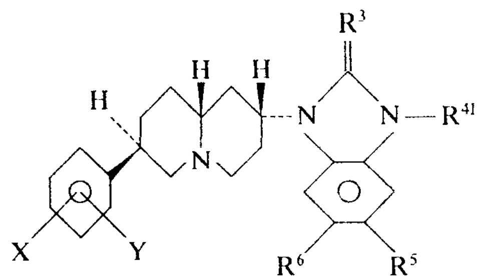

(III-7)

worin X, Y, $R^3$, $R^5$ und $R^6$ dasselbe wie oben bedeuten und $R^{41}$ $C_{1-6}$-Alkyl darstellt,

eine Verbindung der allgemeinen Formel

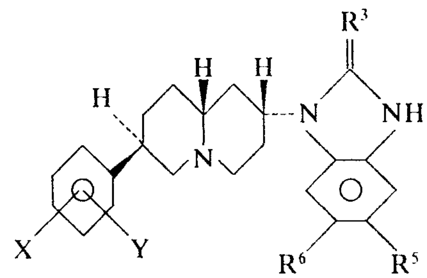

(III-8)

worin X, Y, $R^3$, $R^5$ und $R^6$ dasselbe wie oben bedeuten,

N-alkyliert, oder daß man

7) zur Herstellung von Äthern und Estern der allgemeinen Formel

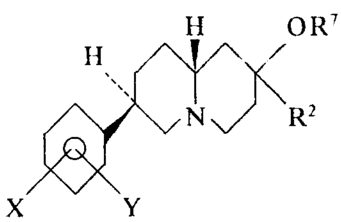

(IV)

worin X, Y und $R^2$ dasselbe wie oben bedeuten und $R^7$ $C_{1-6}$-Alkyl oder Acyl darstellt,

einen Alkohol der obigen allgemeinen Formel II mit einem den Rest $R^7$ abgebenden Verätherungs- bzw. Veresterungsmittel umsetzt und daß man erhaltene Racemate gegebenenfalls spaltet und erhaltene Basen gegebenenfalls in Säureadditionssalze überführt.

20. Arzneimittel, insbesondere zur Verwendung als Antibiotika, Antiemetica und/oder Analgetica, enthaltend ein Phenylchinolizidin der vorstehend angegebenen Formel I gemäß Anspruch 1 in Form des Racemats oder eines Enantiomeren oder eines Säureadditionssalzes einer solchen Verbindung.

21. Verbindungen der vorstehend angegebenen Formel I gemäß Anspruch 1 in Form des Racemats oder der Enantiomeren, sowie Säureadditionssalze solcher Verbindungen zur Verwendung bei der Bekämpfung von Krankheiten, insbesondere von Psychosen, Neurosen, Übelkeit oder von Schmerz.

**Patentansprüche für den Vertragsstaat AT**

1. Verfahren zur Herstellung von Phenyl-chinolizidinen der allgemeinen Formel

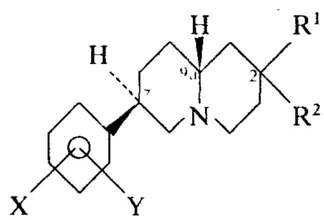

(I)

worin X, Y, $R^1$ und $R^2$ folgendes bedeuten:

X: Wasserstoff, Fluor, Chlor, $C_{1-6}$-Alkoxy, $C_{1-6}$-Alkyl oder Trifluormethyl,
Y: Wasserstoff, Fluor, Chlor, $C_{1-6}$-Alkoxy oder $C_{1-6}$-Alkyl,
$R^1$: Hydroxy, $C_{1-6}$-Alkoxy, Acyloxy oder, falls $R^2$ die nachstehend definierte Gruppe A darstellt, Wasserstoff,
$R^2$: $C_{1-6}$-Alkyl, gegebenenfalls durch Halogen, $C_{1-6}$-Alkoxy oder Trifluormethyl substituiertes Phenyl, oder (falls $R^1$ = Wasserstoff) eine Gruppe A mit der Bedeutung

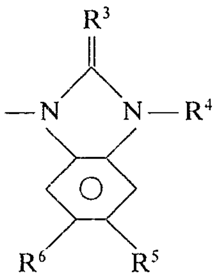
oder
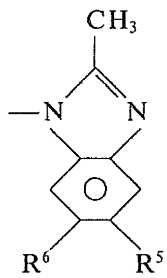
A

mit folgender Bedeutung für $R^3 - R^6$:

$R^3$: Sauerstoff oder Schwefel,
$R^4$: Wasserstoff oder $C_{1-6}$-Alkyl,
$R^5$ und $R^6$ unabhängig voneinander: Wasserstoff, Methyl, Fluor, Chlor, Methoxy, Trifluormethyl oder zusammen $-(CH_2)_4-$,

in Form des Racemats oder der Enantiomeren, sowie von Säureadditionssalzen solcher Verbindungen, welche Verfahren dadurch gekennzeichnet sind, daß man

1) zur Herstellung von Verbindungen der allgemeinen Formel

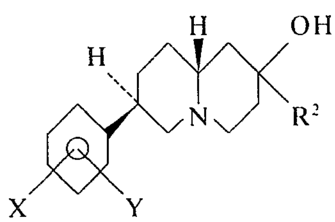
(II)

worin X, Y und $R^2$ dasselbe wie oben bedeuten,

entweder
a) ein Keton der allgemeinen Formel

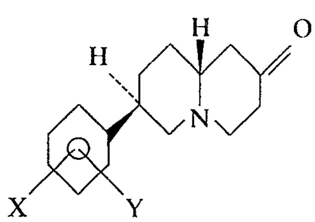
(V)

worin X und Y dasselbe wie oben bedeuten,

mit einer metallorganischen, den Rest $R^2$ liefernden Verbindung umsetzt, und gegebenenfalls das erhaltene Gemisch der Diastereomeren auftrennt, oder
b) an die $\Delta^1$- bzw. $\Delta^2$-Doppelbindung einer Verbindung der allgemeinen Formel

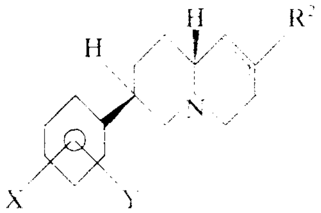

(VI)

worin X, Y und $R^4$ dasselbe wie oben bedeuten und die gestrichelte Bindung eine Doppelbindung in 1,2- oder 2,3-Stellung darstellt,

durch Umsetzung mit einem Hydratisierungsmittel Wasser anlagert,

oder daß man
2) zur Herstellung von Verbindungen der allgemeinen Formel

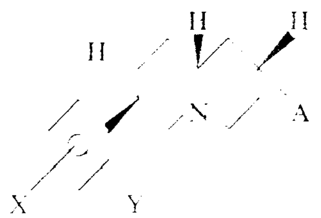

(III)

worin X, Y und A und $R^3-R^6$ dasselbe wie oben bedeuten,

eine Verbindung der allgemeinen Formel

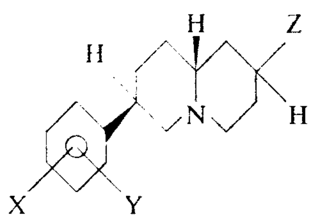

(VII)

worin X und Y dasselbe wie oben bedeuten und Z eine abspaltbare Gruppe darstellt,

mit einer Verbindung der allgemeinen Formel

H — A (VIII)

worin A dasselbe wie oben bedeutet,

umsetzt, oder daß man
3) zur Herstellung einer Verbindung der allgemeinen Formel

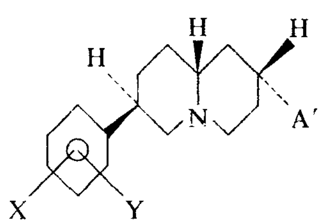

(III-3)

worin X und Y dasselbe wie oben bedeuten und A′ wie A ist, wobei jedoch $R^3$ Sauerstoff darstellt,

eine Verbindung der allgemeinen Formel

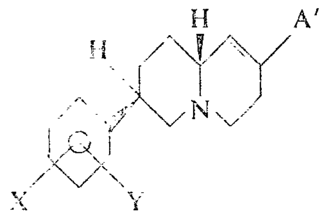

(IX)

worin X, Y und A' dasselbe wie oben bedeuten,

katalytisch hydriert, oder daß man

4) zur Herstellung einer Verbindung der allgemeinen Formel

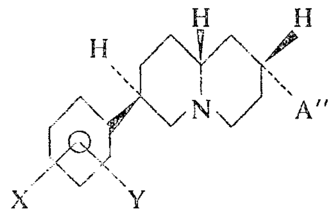

(III-4)

worin X, Y dasselbe wie oben bedeuten und A'' wie A ist, wobei jedoch $R^3$ Sauerstoff und $R^4$ Wasserstoff darstellt,

ein Phenylen-diamin der allgemeinen Formel

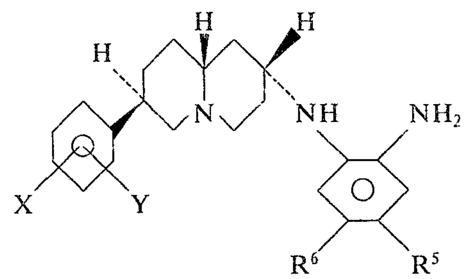

(X)

worin X, Y, $R^5$ und $R^6$ dasselbe wie oben bedeuten,

mit einem Cyclisierungsmittel der allgemeinen Formel

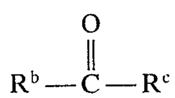

(XI)

worin $R^b$ und $R^c$ Halogen, Amino oder 1-Imidazolyl bedeuten,

oder mit Essigsäure oder einem Orthoessigsäureester kondensiert, oder daß man

5) zur Herstellung von Verbindungen der allgemeinen Formel

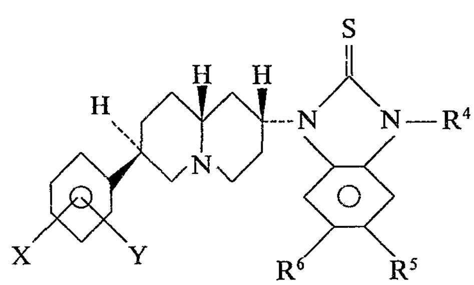

(III-5)

worin X, Y sowie $R^4 - R^6$ dasselbe wie oben bedeuten,

entweder

a) eine entsprechende Oxo-verbindung der allgemeinen Formel

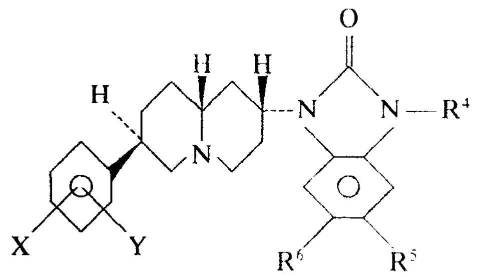

(III-6)

worin X, Y sowie $R^4 - R^6$ dasselbe wie oben bedeuten,

mit $P_2S_5$ umsetzt, oder

b) ein Phenylen-diamin der obigen allgemeinen Formel X mit Thiophosgen, Thiocarbonyldiimidazol oder $CS_2$ umsetzt und anschließend gegebenenfalls N-alkyliert,

oder daß man

6) zur Herstellung von Verbindungen der allgemeinen Formel

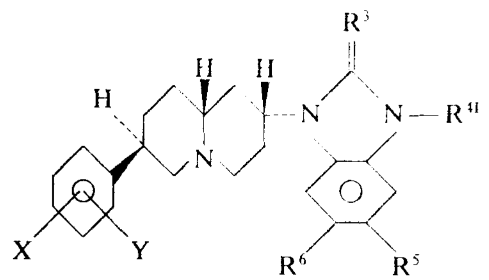

(III-7)

worin X, Y, $R^3$, $R^5$ und $R^6$ dasselbe wie oben bedeuten und $R^{41}$ $C_{1-6}$-Alkyl darstellt,

eine Verbindung der allgemeinen Formel

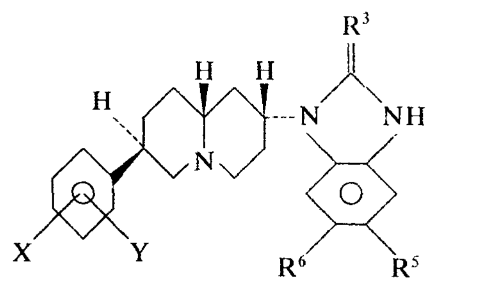

(III-8)

worin X, Y, $R^3$, $R^5$ und $R^6$ dasselbe wie oben bedeuten,

N-alkyliert, oder daß man

7) zur Herstellung von Äthern und Estern der allgemeinen Formel

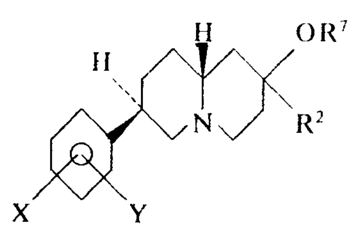

(IV)

worin X, Y und $R^2$ dasselbe wie oben bedeuten und $R^7$ $C_{1-6}$-Alkyl oder Acyl darstellt,

einen Alkohol der obigen allgemeinen Formel II mit einem den Rest $R^7$ abgebenden Veräthe-rungs- bzw. Veresterungsmittel umsetzt und daß man erhaltene Racemate gegebenenfalls spaltet und erhaltene Basen gegebenenfalls in Säureadditionssalze überführt.

2. Verfahren nach Anspruch 1 zur Herstellung von Verbindungen, worin $R^2$ $C_{1-6}$-Alkyl, gegebenenfalls durch Halogen, $C_{1-6}$-Alkoxy oder Trifluormethyl substituiertes Phenyl, oder (falls $R^1$ = Wasserstoff) eine Gruppe A mit der Bedeutung

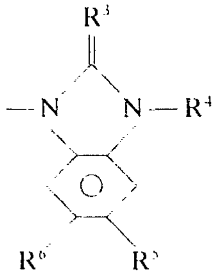

darstellt, wobei $R^3$ und $R^4$ dasselbe wie in Anspruch 1 bedeuten und $R^5$ und $R^6$ unabhängig voneinander Wasserstoff, Methyl, Chlor oder Methoxy darstellen, dadurch gekennzeichnet, daß man entsprechend substituierte Ausgangsverbindungen einsetzt.

3. Verfahren nach Anspruch 1 zur Herstellung von Verbindungen der allgemeinen Formel

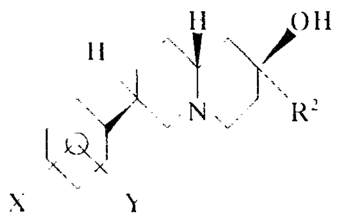

(II-1)

worin X, Y und $R^2$ dasselbe wie in Anspruch 1 bedeuten,

in Form des Racemats oder der Enantiomeren, sowie von Säureadditionssalzen solcher Verbindungen, dadurch gekennzeichnet, daß man entsprechend substituierte Ausgangsverbindungen einsetzt.

4. Verfahren nach Anspruch 3 zur Herstellung von Verbindungen, worin $R^2$ verzweigtes $C_{1-6}$-Alkyl, X Fluor oder Chlor und Y Wasserstoff, Fluor oder Chlor darstellen, wobei X und Y in ortho- und/oder para-Stellung stehen, dadurch gekennzeichnet, daß man entsprechend substituierte Ausgangsverbindungen einsetzt.

5. Verfahren nach Anspruch 4 zur Herstellung einer Verbindung, worin $R^2$ tert.Butyl, X o-Chlor und Y p-Chlor bedeutet, dadurch gekennzeichnet, daß man entsprechend substituierte Ausgangsverbindungen einsetzt.

6. Verfahren nach Anspruch 3 zur Herstellung einer Verbindung, worin $R^2$ tert.Butyl, X p-Trifluormethyl und Y Wasserstoff bedeutet, dadurch gekennzeichnet, daß man entsprechend substituierte Ausgangsverbindungen einsetzt.

7. Verfahren nach Anspruch 1 zur Herstellung von Verbindungen der allgemeinen Formel

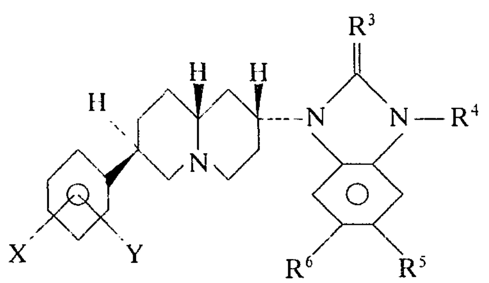

(III-1)

worin X, Y sowie $R^3 - R^6$ dasselbe wie in Anspruch 1 bedeuten,

in Form des Racemats oder der Enantiomeren, sowie von Säureadditionssalzen solcher Verbindungen, dadurch gekennzeichnet, daß man entsprechend substituierte Ausgangsverbindungen einsetzt.

8. Verfahren nach Anspruch 7 zur Herstellung von Verbindungen, worin X Wasserstoff, o-Fluor oder o-Chlor, Y Wasserstoff, $R^3$ Sauerstoff oder Schwefel, $R^4$ Wasserstoff, $R^5$ Fluor, Chlor oder Trifluormethyl und $R^6$ Wasserstoff, Chlor oder Fluor darstellen, dadurch gekennzeichnet, daß man entsprechend substituierte Ausgangsverbindungen einsetzt.

9. Verfahren nach Anspruch 8 zur Herstellung einer Verbindung, worin X o-Chlor, Y Wasserstoff, $R^3$

Sauerstoff, $R^4$ sowie $R^6$ Wasserstoff und $R^5$ Trifluormethyl bedeuten, dadurch gekennzeichnet, daß man entsprechend substituierte Ausgangsverbindungen einsetzt.

10. Verfahren nach Anspruch 8 zur Herstellung einer Verbindung, worin X o-Fluor, Y Wasserstoff, $R^3$ Sauerstoff, $R^4$ sowie $R^6$ Wasserstoff und $R^5$ Chlor bedeuten, dadurch gekennzeichnet, daß man entsprechend substituierte Ausgangsverbindungen einsetzt.

11. Verfahren nach Anspruch 7 zur Herstellung einer Verbindung, worin X, Y und $R^3-R^6$ eine der folgenden Bedeutungen aufweisen:

a) $X = o\text{-}Cl$; Y, $R^4$, $R^5$, $R^6 = H$; $R^3 = O$
b) $X = o\text{-}Cl$; Y, $R^4 = H$; $R^3 = O$; $R^5$, $R^6 = CH_3$
c) $X = o\text{-}Cl$; Y, $R^4 = H$; $R^3 = O$; $R^5$, $R^6 = Cl$
d) $X = o\text{-}Cl$; Y, $R^5$, $R^6 = H$; $R^3 = O$; $R^4 = CH_3$
f) $X = o\text{-}Cl$; Y, $R^4$, $R^6 = H$; $R^5 = Cl$; $R^3 = O$
g) $X = o\text{-}Cl$; Y, $R^4$, $R^6 = H$; $R^5 = Cl$; $R^3 = S$
h) $X = o\text{-}Cl$; Y, $R^4 = H$; $R^3 = S$; $R^5$, $R^6 = Cl$
i) $X = o\text{-}Cl$; Y, $R^4 = H$; $R^3 = O$; $R^5$ und $R^6$ zusammen $-(CH_2)_4-$
j) $X = o\text{-}F$; Y, $R^4 = H$; $R = O$; $R^5$, $R^6 = CH_3$
k) $X = o\text{-}F$; Y, $R^4 = H$; $R^3 = O$; $R^5$, $R^6 = Cl$
l) $X = o\text{-}F$; Y, $R^4 = H$; $R^3 = S$; $R^5$, $R^6 = Cl$
m) $X = o\text{-}F$; Y, $R^4 = H$; $R^3 = O$; $R^5$ und $R^6$ zusammen $-(CH_2)_4-$
n) $X = o\text{-}F$; Y, $R^4$, $R^6 = H$; $R^5 = CF_3$; $R^3 = O$
o) $X = o\text{-}F$; Y, $R^4$, $R^6 = H$; $R^5 = CF_3$; $R^3 = S$
q) $X = o\text{-}F$; Y, $R^4$, $R^6 = H$; $R^5 = Cl$; $R^3 = S$,

dadurch gekennzeichnet, daß man entsprechend substituierte Ausgangsverbindungen einsetzt.

12. Verfahren nach Anspruch 7 zur Herstellung von Verbindungen, worin $R^5$ und $R^6$ unabhängig voneinander Wasserstoff, Methyl, Chlor oder Methoxy bedeuten, dadurch gekennzeichnet, daß man entsprechend substituierte Ausgangsverbindungen einsetzt.

13. Verfahren nach Anspruch 1 zur Herstellung von Verbindungen der allgemeinen Formel

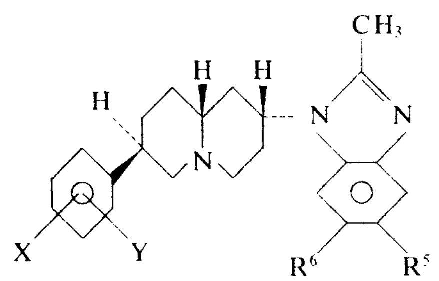

(III-2)

worin X, Y, $R^5$ und $R^6$ dasselbe wie in Anspruch 1 bedeuten,

in Form des Racemats oder der Enantiomeren, sowie von Säureadditionssalzen solcher Verbindungen, dadurch gekennzeichnet, daß man entsprechend substituierte Ausgangsverbindungen einsetzt.

14. Verfahren nach Anspruch 1 zur Herstellung von Verbindungen der allgemeinen Formel

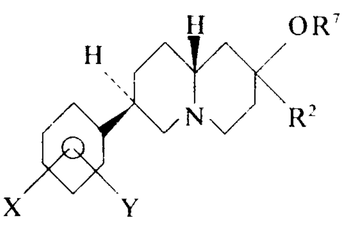

(IV)

worin X, Y und $R^2$ dasselbe wie in Anspruch 1 bedeuten und $R^7$ $C_{1-6}$-Alkyl oder Acyl darstellt,

in Form des Racemats oder der Enantiomeren, sowie von Säureadditionssalzen solcher Verbindungen, dadurch gekennzeichnet, daß man entsprechend substituierte Ausgangsverbindungen einsetzt.

15. Verfahren nach Anspruch 14 zur Herstellung einer Verbindung, worin X und Y Wasserstoff, $OR^7$ $\beta$-orientiertes Acetoxy und $R^2$ n-Butyl bedeuten, dadurch gekennzeichnet, daß man entsprechend substituierte Ausgangsverbindungen einsetzt.

16. Verfahren nach Anspruch 14 zur Herstellung einer Verbindung, worin X und Y Wasserstoff, $OR^7$ $\beta$-orientiertes Trifluoracetoxy und $R^2$ tert.Butyl bedeuten, dadurch gekennzeichnet, daß man entsprechend substituierte Ausgangsverbindungen einsetzt.

17. Verfahren nach Anspruch 14 zur Herstellung einer Verbindung, worin X o-Chlor, $OR^7$ α-orientiertes Acetoxy und $R^2$ Phenyl bedeuten, dadurch gekennzeichnet, daß man entsprechend substituierte Ausgangsverbindungen einsetzt.

**Claims for the contracting states: BE, CH, DE, FR, GB, IT, LU, NL, SE**

1. Phenyl-quinolizidines of the general formula

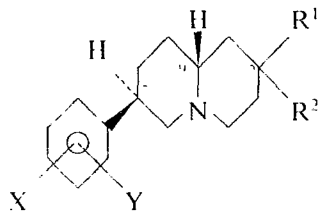

(I)

wherein X, Y, $R^1$ and $R^2$ signify the following:

C: hydrogen, fluorine, chlorine, $C_{1-6}$-alkoxy, $C_{1-6}$-alkyl or trifluoromethyl,
Y: hydrogen, fluorine, chlorine, $C_{1-6}$-alkoxy or $C_{1-6}$-alkyl,
$R^1$: hydroxy, $C_{1-6}$-alkoxy, acyloxy or, where $R^2$ represents the group A defined hereinafter, hydrogen,
$R^2$: $C_{1-6}$-alkyl, phenyl optionally substituted by halogen, $C_{1-6}$-alkoxy or trifluoromethyl, or (where $R^1$ = hydrogen) a group A with the significance

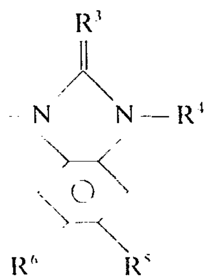

or

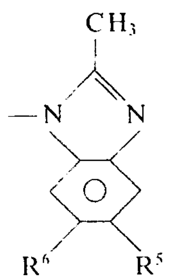

A

with the following significance for $R^3 - R^6$:

$R^3$: oxygen or sulphur,
$R^4$: hydrogen or $C_{1-6}$-alkyl,
$R^5$ and $R^6$ independently of one another: hydrogen, methyl, fluorine, chlorine, methoxy, trifluoromethyl or together $-(CH_2)_4-$,

in the form of the racemate or the enantiomers, as well as acid addition salts of such compounds.

2. Compounds according to claim 1, wherein $R^2$ represents $C_{1-6}$-alkyl, phenyl optionally substituted by halogen, $C_{1-6}$-alkoxy or trifluoromethyl, or (where $R^1$ = hydrogen) a group A with the significance

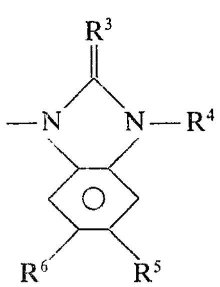

whereby $R^3$ and $R^4$ signify the same as in claim 1 and $R^5$ and $R^6$ independently of one another represent hydrogen, methyl, chlorine or methoxy.

3. Compounds according to claim 1 of the general formula

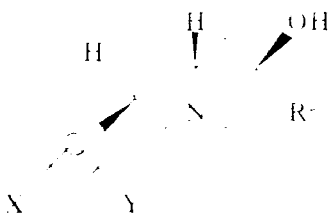

(II-1)

wherein X, Y and $R^2$ signify the same as in claim 1,

in the form of the racemate or the enantiomers, as well as acid addition salts of such compounds.

4. Compounds according to claim 3, wherein $R^2$ represents branched $C_{3-6}$-alkyl, X represents fluorine or chlorine and Y represents hydrogen, fluorine or chlorine, whereby X and Y are present in the ortho- and/or para-position.

5. A compound according to claim 4, wherein $R^2$ signifies tert.butyl, X signifies o-chloro and Y signifies p-chloro.

6. A compound according to claim 3, wherein $R^2$ signifies tert.butyl, X signifies p-trifluormethyl and Y signifies hydrogen.

7. Compounds according to claim 1 of the general formula

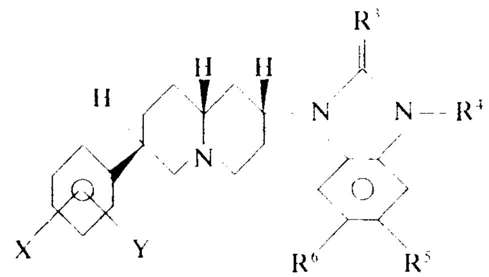

(III-1)

wherein X, Y as well as $R^3$ – $R^6$ signify the same as in claim 1,

in the form of the racemate or the enantiomers, as well as acid addition salts of such compounds.

8. Compounds according to claim 7, wherein X represents hydrogen, o-fluoro or o-chloro, Y represents hydrogen, $R^3$ represents oxygen or sulphur, $R^4$ represents hydrogen, $R^5$ represents fluorine, chlorine or trifluoromethyl and $R^6$ represents hydrogen, chlorine or fluorine.

9. A compound according to claim 8, wherein X signifies o-chloro, Y signifies hydrogen, $R^3$ signifies oxygen, $R^4$ as well as $R^6$ signify hydrogen and $R^5$ signifies trifluoromethyl.

10. A compound according to claim 8, wherein X signifies o-fluoro, Y signifies hydrogen, $R^3$ signifies oxygen, $R^4$ as well as $R^6$ signify hydrogen and $R^5$ signifies chlorine.

11. A compound according to claim 7, wherein X, Y and $R^3$ – $R^6$ have the following significances:

a) X = o-Cl; Y, $R^4$, $R^5$, $R^6$ = H; $R^3$ = O
b) X = o-Cl; Y, $R^4$ = H; $R^3$ = O; $R^5$, $R^6$ = $CH_3$
c) X = o-Cl; Y, $R^4$ = H; $R^3$ = O; $R^5$, $R^6$ = Cl
d) X = o-Cl; Y, $R^5$, $R^6$ = H; $R^3$ = O; $R^4$ = $CH_3$
f) X = o-Cl; Y, $R^4$, $R^6$ = H; $R^5$ = Cl; $R^3$ = O
g) X = o-Cl; Y, $R^4$, $R^6$ = H; $R^5$ = Cl; $R^3$ = S
h) X = o-Cl; Y, $R^4$ = H; $R^3$ = S; $R^5$, $R^6$ = Cl
i) X = o-Cl; Y, $R^4$ = H; $R^3$ = O; $R^5$ and $R^6$ together $-(CH_2)_4-$
j) X = o-F; Y, $R^4$ = H; $R^3$ = O; $R^5$, $R^6$ = $CH_3$
k) X = o-F; Y, $R^4$ = H; $R^3$ = O; $R^5$, $R^6$ = Cl
l) X = o-F; Y, $R^4$ = H; $R^3$ = S; $R^5$, $R^6$ = Cl
m) X = o-F; Y, $R^4$ = H; $R^3$ = O; $R^5$ and $R^6$ together $-(CH_2)_4-$
n) X = o-F; Y, $R^4$, $R^6$ = H; $R^5$ = $CF_3$; $R^3$ = O
o) X = o-F; Y, $R^4$, $R^6$ = H; $R^5$ = $CF_3$; $R^3$ = S
q) X = o-F; Y, $R^4$, $R^6$ = H; $R^5$ = Cl; $R^3$ = S.

12. Compounds according to claim 7, wherein $R^5$ and $R^6$ independently of one another signify hydrogen, methyl, chlorine or methoxy.

13. Compounds according to claim 1 of the general formula

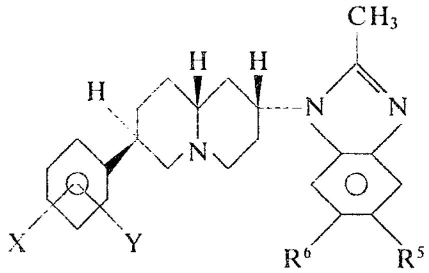

(III-2)

wherein X, Y, $R^2$ and $R^2$ signify the same as in claim 1,

in the form of the racemate or the enantiomers, as well as acid addition salts of such compounds.

14. Compounds according to claim 1 of the general formula

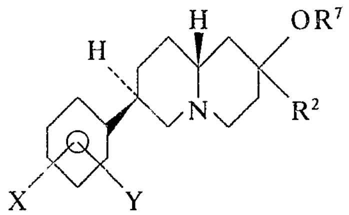

(IV)

wherein X, Y and $R^2$ signify the same as in claim 1 and $R^7$ represents lower-alkyl or acyl,

in the form of the racemate or the enantiomers, as well as acid addition salts of such compounds.

15. A compound according to claim 14, wherein X and Y signify hydrogen, $OR^7$ signifies $\beta$-orientated acetoxy and $R^2$ signifies n-butyl.

16. A compound according to claim 14, wherein X and Y signify hydrogen, $OR^7$ signifies $\beta$-orientated trifluoroacetoxy and $R^2$ signifies tert.butyl.

17. A compound according to claim 14, wherein X signifies o-chloro, $OR^7$ signifies $\alpha$-orientated acetoxy and $R^2$ signifies phenyl.

18. Phenyl-quinolizidines according to one of claims 1—17 as pharmaceutically active substances, especially as antipsychotic, antiemetic and/or analgesic active substances.

19. Processes for the manufacture or phenyl-quinolizidines of the general formula

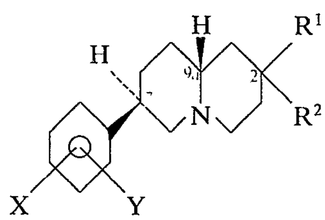

(I)

wherein X, Y, $R^1$ and $R^2$ signify the following:

X: hydrogen, fluorine, chlorine, $C_{1-6}$-alkoxy, $C_{1-6}$-alkyl or trifluoromethyl,
Y: hydrogen, fluorine, chlorine, $C_{1-6}$-alkoxy or $C_{1-6}$-alkyl,
$R^1$: hydroxy, $C_{1-6}$-alkoxy, acyloxy or, where $R^2$ represents the group A defined hereinafter, hydrogen,
$R^2$: $C_{1-6}$-alkyl, phenyl optionally substituted by halogen, $C_{1-6}$-alkoxy or trifluoromethyl, or (where $R^1$ = hydrogen) a group A with the significance

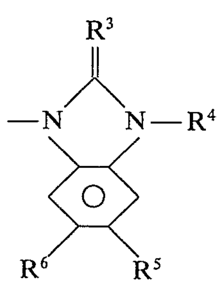

or

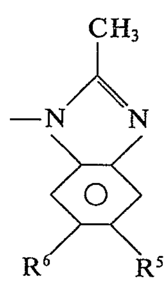

A

with the following significance for $R^3$ – $R^6$:

$R^3$: oxygen or sulphur,
$R^4$: hydrogen or $C_{1-6}$-alkyl,
$R^5$ and $R^6$ independently of one another: hydrogen, methyl, fluorine, chlorine, methoxy, trifluoromethyl or together $-(CH_2)_4-$,

in the form of the racemate or the enantiomers, as well as of acid addition salts of such compounds, which processes are characterized by

1) for the manufacture of compounds of the general formula

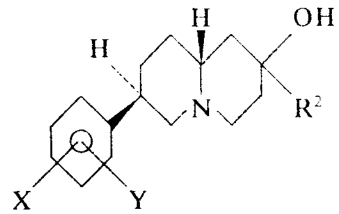

(II)

wherein X, Y and $R^2$ signify the same as above,
either
a) reacting a ketone of the general formula

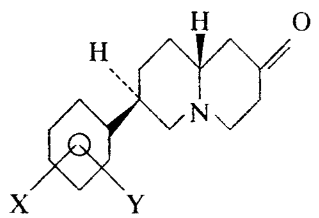

(V)

wherein X and Y signify the same as above,

with a metal-organic compound yielding the group $R^2$ and, if desired, separating the mixture of diastereomers obtained, or
b) adding water at the $\Delta^1$- or $\Delta^2$-double bond of a compound of the general formula

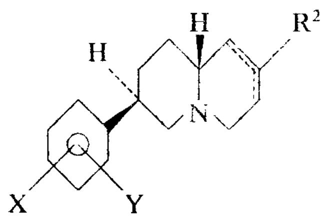

(VI)

wherein X, Y $R^2$ signify the same as above and the dotted bond represents a double-bond in the 1,2- or 2,3-position,

by reaction with a hydrating agent,

or by
2) for the manufacture of compounds of the general formula

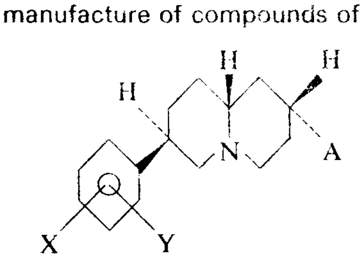

(III)

wherein X, Y, A and $R^3$–$R^6$ signify the same as above,

reacting a compound of the general formula

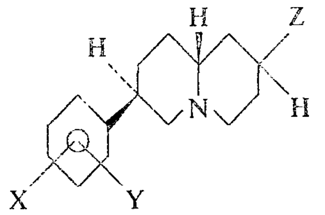

(VII)

wherein X and Y signify the same as above and Z represents a cleavable group,

with a compound of the general formula

H---A (VIII)

wherein A signifies the same as above,

or by

3) for the manufacture of a compound of the general formula

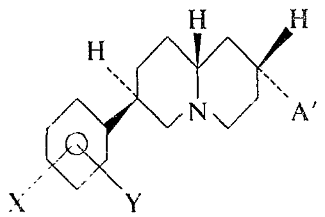

(III-3)

wherein X and Y signify the same as above and A′ is as A, whereby, however, $R^3$ represents oxygen,

catalytically hydrogenating a compound of the general formula

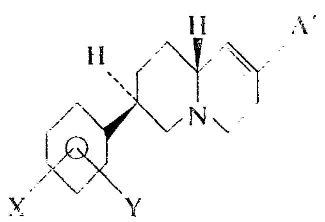

(IX)

wherein X, Y and A′ signify the same as above,

or by

4) for the manufacture of a compound of the general formula

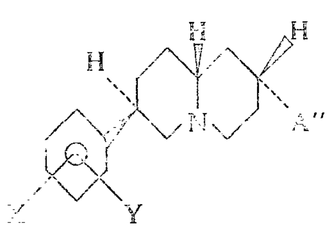

(III-4)

wherein X and Y signify the same as above and A″ is as A, whereby, however, $R^3$ represents oxygen and $R^4$ represents hydrogen,

condensing a phenylene-diamine of the general formula

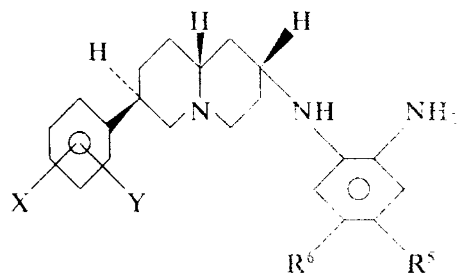

(X)

wherein X, Y, $R^5$ and $R^6$ signify the same as above,

with a cyclizing agent of the general formula

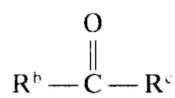

(XI)

wherein $R^b$ and $R^c$ signify halogen, amino or 1-imidazolyl,

or with acetic acid or an orthoacetic acid ester, or by

5) for the manufacture of compounds of the general formula

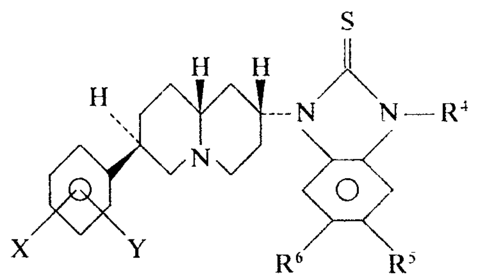

(III-5)

wherein X, Y as well as $R^4$–$R^6$ signify the same as above,

either

a) reacting a corresponding oxo compound of the general formula

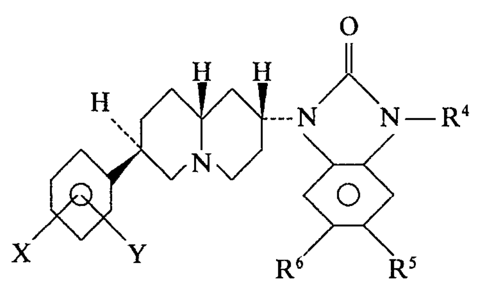

(III-6)

wherein X, Y as well as $R^4$–$R^6$ signify the same as above,

with $P_2S_5$, or

b) reacting a phenylene-diamine of general formula X above with thiophosgene, thiocarbonyl-diimidazole or $CS_2$ and subsequently N-alkylating the product if desired,

or by

6) for the manufacture of compounds of the general formula

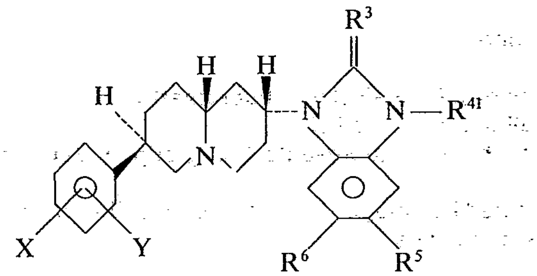

(III-7)

wherein X, Y, $R^3$, $R^5$ and $R^6$ signify the same as above and $R^{41}$ represents $C_{1-6}$-alkyl,

N-alkylating a compound of the general formula

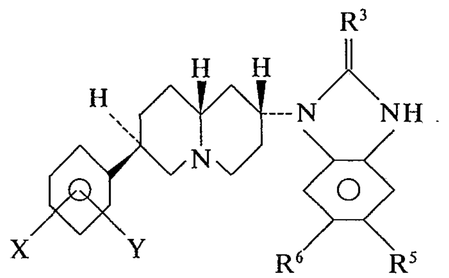

(III-8)

wherein X, Y, $R^3$, $R^5$ and $R^6$ signify the same as above,

or by

7) for the manufacture of ethers and esters of the general formula

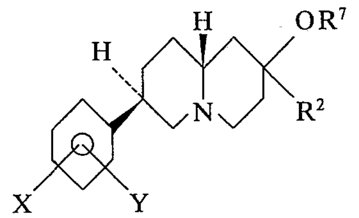

(IV)

wherein X, Y and $R^2$ signify the same as above and $R^7$ represents $C_{1-6}$-alkyl or acyl,

reacting an alcohol of general formula II above with an etherifying or esterifying agent yielding the group $R^7$ and, if desired, resolving a racemate obtained and, if desired, converting a base obtained into an acid addition salt.

**20. A medicament, especially for use as an antibiotic, antiemetic and/or analgesic, containing a phenylquinolizidine of the previously given formula I according to claim 1 in the form of the racemate or an enantiomer or an acid addition salt of such a compound.**

**21. Compounds of the previously given formula I according to claim 1 in the form of the racemate or the enantiomers as well as acid addition salts of such compounds for use in the control of illnesses, especially of psychoses, neuroses, nausea or of pain.**

**Claims for the contracting state: AT**

**1. Processes for the manufacture of phenyl-quinolizidines of the general formula**

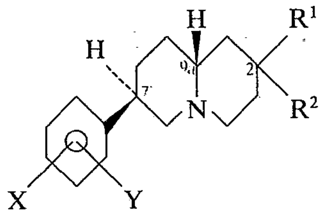

(I)

wherein X, Y, $R^1$ and $R^2$ signify the following:

X: hydrogen, fluorine, chlorine, $C_{1-6}$-alkoxy, $C_{1-6}$-alkyl or trifluoromethyl,
Y: hydrogen, fluorine, chlorine, $C_{1-6}$-alkoxy or $C_{1-6}$-alkyl,
$R^1$: hydroxy, $C_{1-6}$-alkoxy, acyloxy or, where $R^2$ represents the group A defined hereinafter, hydrogen,
$R^2$: $C_{1-6}$-alkyl, phenyl optionally substituted by halogen, $C_{1-6}$-alkoxy or trifluoromethyl, or (where $R^1$ = hydrogen) a group A with the significance

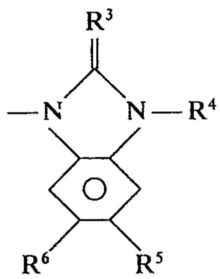
or
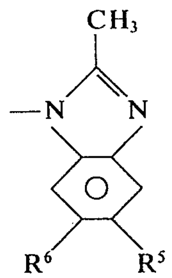
A

with the following significance for $R^3 - R^6$:

$R^3$: oxygen or sulphur,
$R^4$: hydrogen or $C_{1-6}$-alkyl,
$R_5$ and $R^6$ independently of one another: hydrogen, methyl, fluorine, chlorine, methoxy, trifluoromethyl or together $-(CH_2)_4-$,

in the form of the racemate or the enantiomers, as well as of acid addition salts of such compounds, which processes are characterized by

1) for the manufacture of compounds of the general formula

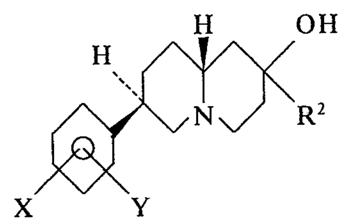
(II)

wherein X, Y and $R^2$ signify the same as above,
either
a) reacting a ketone of the general formula

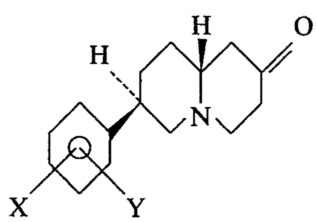
(V)

wherein X and Y signify the same as above,

with a metal-organic compound yielding the group $R^2$ and, if desired, separating the mixture of diastereomers obtained, or
b) adding water at the $\Delta^1$- or $\Delta^2$-double bond of a compound of the general formula

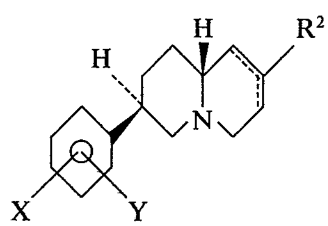
(VI)

wherein X, Y $R^2$ signify the same as above and the dotted bond represents a double-bond in the 1,2- or 2,3-position,

by reaction with a hydrating agent,

or by

2) for the manufacture of compounds of the general formula

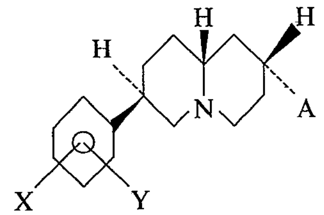

(III)

wherein X, Y, A and $R^3$–$R^6$ signify the same as above,

reacting a compound of the general formula

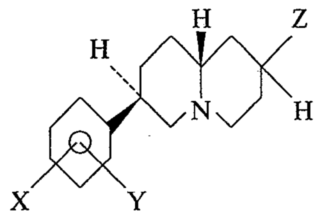

(VII)

wherein X and Y signify the same as above and Z represents a cleavable group,

with a compound of the general formula

H—A (VIII)

wherein A signifies the same as above,

or by

3) for the manufacture of a compound of the general formula

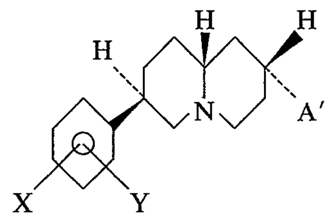

(III-3)

wherein X and Y signify the same as above and A′ is as A, whereby, however, $R^3$ represents oxygen,

catalytically hydrogenating a compound of the general formula

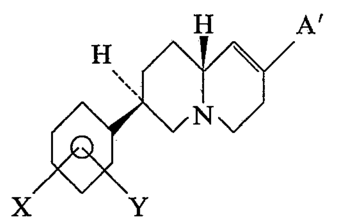

(IX)

wherein X, Y and A′ signify the same as above,

or by

4) for the manufacture of a compound of the general formula

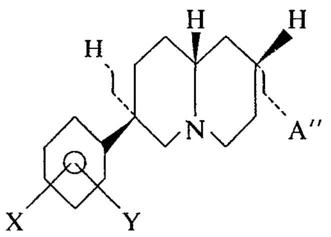

(III-4)

wherein X and Y signify the same as above and A″ is as A, whereby, however, $R^3$ represents oxygen and $R^4$ represents hydrogen,

condensing a phenylene-diamine of the general formula

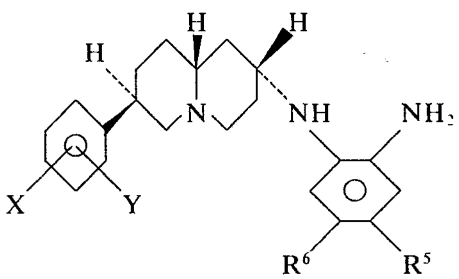

(X)

wherein X, Y, $R^5$ and $R^6$ signify the same as above,

with a cyclizing agent of the general formula

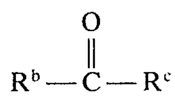

(XI)

wherein $R^b$ and $R^c$ signify halogen, amino or 1-imidazolyl,

or with acetic acid or an orthoacetic acid ester, or by

5) for the manufacture of compounds of the general formula

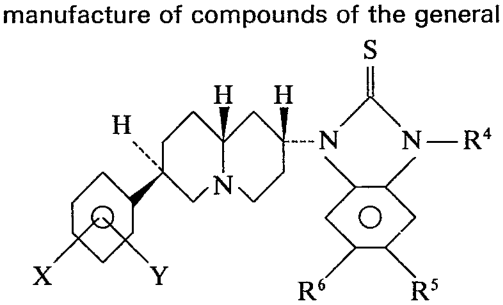

(III-5)

wherein X, Y as well as $R^4$–$R^6$ signify the same as above,

either

a) reacting a corresponding oxo compound of the general formula

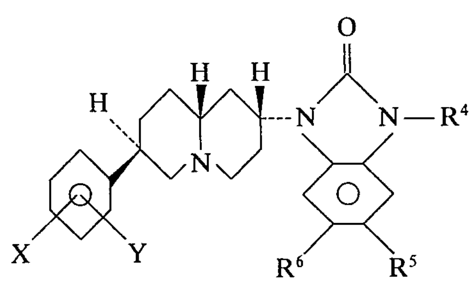

(III-6)

wherein X, Y as well as $R^4$–$R^6$ signify the same as above,

with $P_2S_2$, or

b) reacting a phenylene-diamine of general formula X above with thiophosgene, thiocarbonyl-diimidazole or $CS_2$ and subsequently N-alkylating the product if desired,

or by

6) for the manufacture of compounds of the general formula

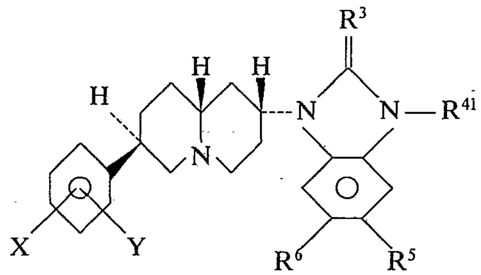

(III-7)

wherein X, Y, $R^3$, $R^5$ and $R^6$ signify the same as above and $R^{41}$ represents $C_{1-6}$-alkyl,

N-alkylating a compound of the general formula

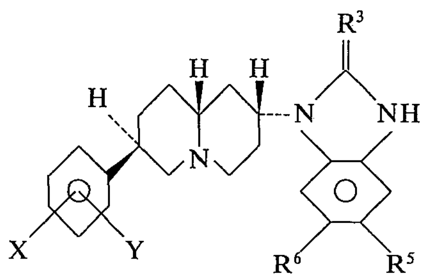

(III-8)

wherein X, Y, $R^3$, $R^5$ and $R^6$ signify the same as above,

or by

7) for the manufacture of ethers and esters of the general formula

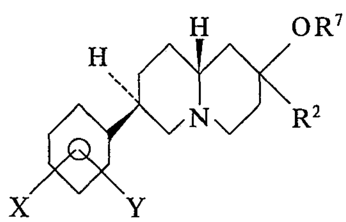

(IV)

wherein X, Y and $R^2$ signify the same as above and $R^7$ represents $C_{1-6}$-alkyl or acyl,

reacting an alcohol of general formula II above with an etherifying or esterifying agent yielding the group $R^7$ and, if desired, resolving a racemate obtained and, if desired, converting a base obtained into an acid addition salt.

2. Processes according to claim 1 for the manufacture of compounds, wherein $R^2$ represents $C_{1-6}$-alkyl, phenyl optionally substituted by halogen, $C_{1-6}$-alkoxy or trifluoromethyl, or (where $R^1$ = hydrogen) a group A with the significance

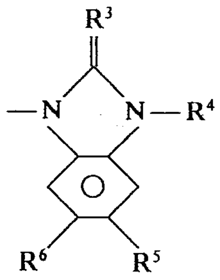

whereby $R^3$ and $R^4$ signify the same as in claim 1 and $R^5$ and $R^6$ independently of one another represent hydrogen, methyl, chlorine or methoxy, characterized in that correspondingly substituted starting compounds are used.

3. Processes according to claim 1 for the manufacture of compounds of the general formula

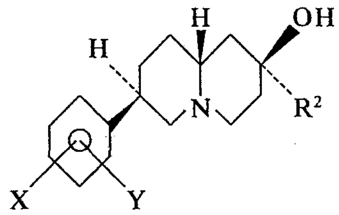

(II-1)

wherein X, Y and $R^2$ signify the same as in claim 1,

in the form of the racemate or the enantiomers, as well as of acid addition salts of such compounds, characterized in that correspondingly substituted starting compounds are used.

4. Processes according to claim 3 for the manufacture of compounds, wherein $R^2$ represents branched $C_{3-6}$-alkyl, X represents fluorine or chlorine and Y represents hydrogen, fluorine or chlorine, whereby X and Y are present in the ortho- and/or para-position, characterized in that correspondingly substituted starting compounds are used.

5. Processes according to claim 4 for the manufacture of a compound, wherein $R^2$ signifies tert.butyl, X signifies o-chloro and Y signifies p-chloro, characterized in that correspondingly substituted starting compounds are used.

6. Processes according to claim 3 for the manufacture of a compound, wherein $R^2$ signifies tert.butyl, X signifies p-trifluoromethyl and Y signifies hydrogen, characterized in that correspondingly substituted starting compounds are used.

7. Processes according to claim 1 for the manufacture of compounds of the general formula

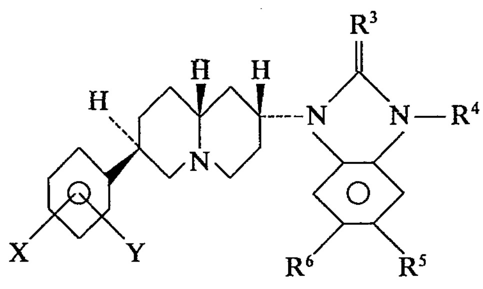

(III-1)

wherein X, Y as well as $R^3 - R^6$ signify the same as in claim 1,

in the form of the racemate or the enantiomers, as well as of acid addition salts of such compounds, characterized in that correspondingly substituted starting compounds are used.

8. Processes according to claim 7 for the manufacture of compounds, wherein X represents hydrogen, o-fluoro or o-chloro, Y represents hydrogen, $R^3$ represents oxygen or sulphur, $R^4$ represents hydrogen, $R^5$ represents fluorine, chlorine or trifluoromethyl and $R^6$ represents hydrogen, chlorine or fluorine, characterized in that correspondingly substituted starting compounds are used.

9. Processes according to claim 8 for the manufacture a compound, wherein X signifies o-Chloro, Y signifies hydrogen, $R^3$ signifies oxygen, $R^4$ as well as $R^6$ signify hydrogen and $R^5$ signifies trifluoromethyl, characterized in that correspondingly substituted starting compounds are used.

10. Processes according to claim 8 for the manufacture a compound, wherein X signifies o-fluoro, Y signifies hydrogen, $R^3$ signifies oxygen, $R^4$ as well as $R^6$ signify hydrogen and $R^5$ signifies chlorine, characterized in that correspondingly substituted starting compounds are used.

11. Processes according to claim 7 for the manufacture a compound, wherein X, Y and $R^3-R^6$ have the following significances:

a) $X=o\text{-}Cl$; Y, $R^4$, $R^5$, $R^6=H$; $R^3=O$
b) $X=o\text{-}Cl$; Y, $R^4=H$; $R^3=O$; $R^5$, $R^6=CH_3$
c) $X=o\text{-}Cl$; Y, $R^4=H$; $R^3=O$; $R^5$, $R^6=Cl$
d) $X=o\text{-}Cl$; Y, $R^5$, $R^6=H$; $R^3=O$; $R^4=CH_3$
f) $X=o\text{-}Cl$; Y, $R^4$, $R^6=H$; $R^5=Cl$; $R^3=O$
g) $X=o\text{-}Cl$; Y, $R^4$, $R^6=H$; $R^5=Cl$; $R^3=S$
h) $X=o\text{-}Cl$; Y, $R^4=H$; $R^3=S$; $R^5$, $R^6=Cl$
i) $X=o\text{-}Cl$; Y, $R^4=H$; $R^3=O$; $R^5$ and $R^6$ together $-(CH_2)_4-$
j) $X=o\text{-}F$; Y, $R^4=H$; $R^3=O$; $R^5$, $R^6=CH_3$
k) $X=o\text{-}F$; Y, $R^4=H$; $R^3=O$; $R^5$, $R^6=Cl$
l) $X=o\text{-}F$; Y, $R^4=H$; $R^3=S$; $R^5$, $R^6=Cl$
m) $X=o\text{-}F$; Y, $R^4=H$; $R^3=O$; $R^5$ and $R^6$ together $-(CH_2)_4-$
n) $X=o\text{-}F$; Y, $R^4$, $R^6=H$; $R^5=CF_3$; $R^3=O$
o) $X=o\text{-}F$; Y, $R^4$, $R^6=H$; $R^5=CF_3$; $R^3=S$
q) $X=o\text{-}F$; Y, $R^4$, $R^6=H$; $R^5=Cl$; $R^3=S$,

characterized in that correspondingly substituted starting compounds are used.

12. Processes according to claim 7 for the manufacture of compounds, wherein $R^5$ and $R^6$ independently of one another signify hydrogen, methyl, chlorine or methoxy, characterized in that correspondingly substituted starting compounds are used.

13. Processes according to claim 1 for the manufacture of compounds of the general formula

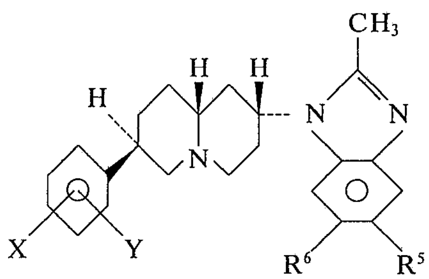

(III-2)

wherein X, Y, $R^5$ and $R^6$ signify the same as in claim 1,

in the form of the racemate or the enantiomers, as well as of acid addition salts of such compounds, characterized in that correspondingly substituted starting compounds are used.

14. Processes acccording to claim 1 for the manufacture of compounds of the general formula

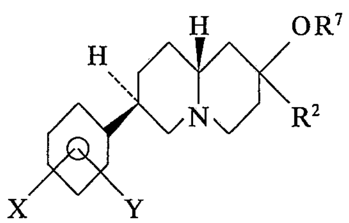

(IV)

wherein X, Y and $R^2$ signify the same as in claim 1 and $R^7$ represents lower-alkyl or acyl,

in the form of the racemate or the enantiomers, as well as of acid addition salts of such compounds, characterized in that correspondingly substituted starting compounds are used.

15. Processes according to claim 14 for the manufacture of a compound, wherein X and Y signify hydrogen, $OR^7$ signifies $\beta$-orientated acetoxy and $R^2$ signifies n-butyl, characterized in that correspondingly substituted starting compounds are used.

16. Processes according to claim 14 for the manufacture of a compound, wherein X and Y signify hydrogen, $OR^7$ signifies $\beta$-orientated trifluoroacetoxy and $R^2$ signifies tert.butyl, characterized in that correspondingly substituted starting compounds are used.

17. Processes according to claim 14 for the manufacture of a compound, wherein X signifies o-chloro, $OR^7$ signifies $\alpha$-orientated acetoxy and $R^2$ signifies phenyl, characterized in that correspondingly substituted starting compounds are used.

**Revendications pour les Etats contractants: BE, CH, DE, FR, GB, IT, LU, NL, SE**

1. Phényl-quinolizidines de formule générale

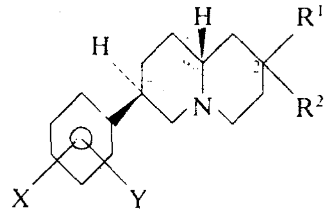

(I)

où X, Y, $R^1$ et $R^2$ ont les significations suivantes:

X: hydrogène, fluor, chlore, alcoxy en $C_1$ à $C_6$, alcoyle en $C_1$ à $C_6$ ou trifluorométhyle
Y: hydrogène, fluor, chlore, alcoxy en $C_1$ à $C_6$ ou alcoyle en $C_1$ à $C_6$,
$R^1$: hydroxy, alcoxy en $C_1$ à $C_6$, acyloxy ou, si $R^2$ représente le groupe A défini ci-dessous, un hydrogène,
$R^2$: alcoyle en $C_1$ à $C_6$, phényle éventuellement substitué par un halogène, un alcoxy en $C_1$ à $C_6$ ou un trifluorométhyle, ou (si $R^1$ = hydrogène) un groupe A représentant

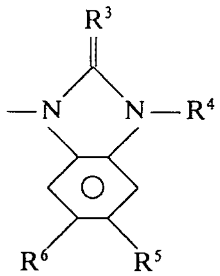 ou 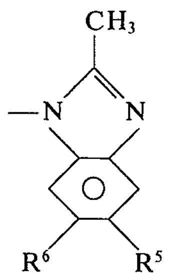 A

où $R^3 - R^6$ ont les significations suivantes:

$R^3$: oxygène ou soufre,
$R^4$: hydrogène ou alcoyle en $C_1$ à $C_4$,
$R^5$ et $R^6$ indépendamment l'un de l'autre: hydrogène, méthyle, fluor, chlore, méthoxy, trifluorométhyle ou, ensemble, $-(CH_2)_4-$,

sous la forme du racémate ou des énantiomères, ainsi que les sels d'addition acide de ces composés.

2. Composés selon la revendication 1, où $R^2$ représente un alcoyle en $C_1$ à $C_6$, un phényle éventuellement substitué par un halogène, un alcoxy en $C_1$ à $C_6$ ou un trifluorométhyle, ou (si $R^1$ = hydrogène) un groupe A représentant

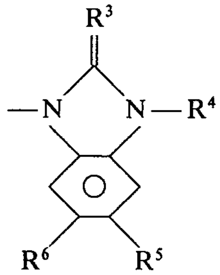

où $R^3$ et $R^4$ ont la même signification que dans la revendication 1, et où $R^5$ et $R^6$ représentent indépendamment l'un de l'autre un hydrogène, un méthyle, un chlore ou un méthoxy.

3. Composés selon la revendication I de formule générale

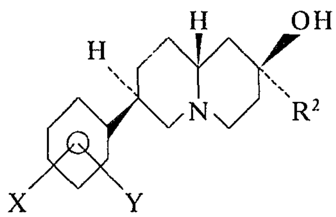

(II-1)

où X, Y et $R^2$ ont la même signification que dans la revendication 1,

sous la forme du racémate ou des énantiomères, ainsi que les sels d'addition acide de ces composés.

4. Composés selon la revendication 3, où $R^2$ représente un alcoyle en $C_3$ à $C_6$ ramifié, X un fluor ou un chlore et Y un hydrogène, un fluor ou un chlore, où X et Y sont en position ortho et/ou para.

5. Composé selon la revendication 4, où $R^2$ représente un tert-butyle, X un o-chloro et Y un p-chloro.

6. Composé selon la revendication 3, où $R^2$ représente un tert-butyle, X un p-trifluorométhyle et Y un hydrogène.

7. Composés selon la revendication 1 de formule générale

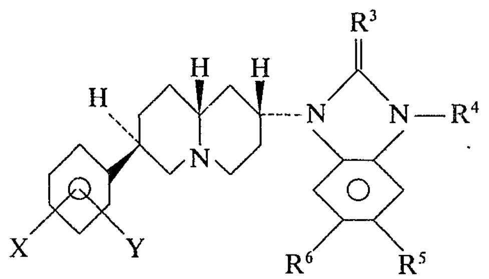

(III-1)

où X, Y, ainsi que $R^3 - R^6$ ont les mêmes significations que dans la revendication 1,

sous la forme du racémate ou des énantiomères, ainsi que les sels d'addition acide de tels composés.

8. Composés selon la revendication 7, où X représente un hydrogène, un o-fluoro ou un o-chloro, Y un hydrogène, $R^3$ un oxygène ou un soufre, $R^4$ un hydrogène, $R^5$ un fluor, un chlore ou un trifluorométhyle et $R^6$ un hydrogène, un chlore ou un fluor.

9. Composé selon la revendication 8, où X représente un o-chloro, Y un hydrogène, $R^3$ un oxygène, $R^4$ ainsi que $R^6$ un hydrogène et $R^5$ un trifluorométhyle.

10. Composé selon la revendication 8, où X représente un o-fluoro, Y un hydrogène, $R^3$ un oxygène, $R^4$ ainsi que $R^6$ un hydrogène et $R^5$ un chlore.

11. Composé selon la revendication 7, où X, Y et $R^3 - R^6$ ont l'une des significations suivantes:

a) X = o-Cl; Y, $R^4$, $R^5$, $R^6$ = H; $R^3$ = O
b) X = o-Cl; Y, $R^4$ = H; $R^3$ = O; $R^5$, $R^6$ = $CH_3$
c) X = o-Cl; Y, $R^4$ = H; $R^3$ = O; $R^5$, $R^6$ = Cl
d) X = o-Cl; Y, $R^5$, $R^6$ = H; $R^3$ = O; $R^4$ = $CH_3$
f) X = o-Cl; Y, $R^4$, $R^6$ = H; $R^5$ = Cl; $R^3$ = O
g) X = o-Cl; Y, $R^4$, $R^6$ = H; $R^5$ = Cl; $R^3$ = S
h) X = o-Cl; Y, $R^4$ = H; $R^3$ = S; $R^5$, $R^6$ = Cl
i) X = o-Cl; Y, $R^4$ = H; $R^3$ = O; $R^5$ et $R^6$ ensemble $-(CH_2)_4-$
j) X = o-F; Y, $R^4$ = H; $R^3$ = O; $R^5$, $R^6$ = $CH_3$
k) X = o-F; Y, $R^4$ = H; $R^3$ = O; $R^5$, $R^6$ = Cl
l) X = o-F; Y, $R^4$ = H; $R^3$ = S; $R^5$, $R^6$ = Cl
m) X = o-F; Y, $R^4$ = H; $R^3$ = O; $R^5$ et $R^6$ ensemble $-(CH_2)_4-$
n) X = o-F; Y, $R^4$, $R^6$ = H; $R^5$ = $CF_3$; $R^3$ = O
o) X = o-F; Y, $R^4$, $R^6$ = H; $R^5$ = $CF_3$; $R^3$ = S
q) X = o-F; Y, $R^4$, $R^6$ = H; $R^5$ = Cl; $R^3$ = S.

12. Composés selon la revendication 7, où $R^5$ et $R^6$ représentent indépendamment l'un de l'autre un hydrogène, un méthyle, un chlore ou un méthoxy.

13. Composés selon la revendication 1 de formule générale

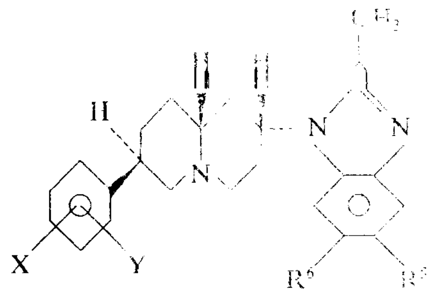

(III-2)

où X, Y, $R^5$ et $R^6$ ont les mêmes significations que dans la revendication 1.

sous la forme du racémate ou des énantiomères, ainsi que les sels d'addition acide de ces composés.

14. Composés selon la revendication 1 de formule générale

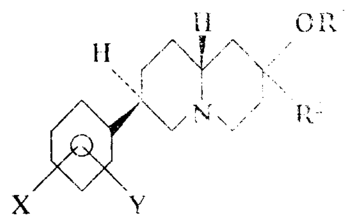

(IV)

où X, Y et $R^2$ ont les mêmes significations que dans la revendication 1 et où $R^7$ représente un alcoyle inférieur ou un acyle,

sous la forme du racémate ou des énantiomères, ainsi que les sels d'addition acide de ces composés.

15. Composé selon la revendication 14, où X et Y représentent un hydrogène, $OR^7$ un acétoxy orienté en $\beta$ et $R^2$ un n-butyle

16. Composé selon la revendication 14, où X et Y représentent un hydrogène, $OR^7$ un trifluoracétoxy orienté en $\beta$, et $R^2$ un tert-butyle.

17. Composé selon la revendication 14, où X représente un o-chloro, $OR^7$ un acétoxy orienté en $\alpha$ et $R^2$ un phényle.

18. Phényl-quinolizidines selon l'une des revendications 1—17 comme substances actives pharmaceutiques, en particulier comme substances antipsychotiques, antiémétiques et/ou analgésiques.

19. Procédés de préparation de phénylquinolizidines de formule générale

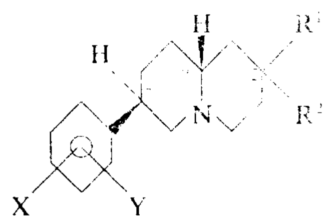

(I)

où X, Y, $R^1$ et $R^2$ ont les significations suivantes:

X: hydrogène, fluor, chlore, alcoxy en $C_1$ à $C_6$, alcoyle en $C_1$ à $C_6$ ou trifluorométhyle,
Y: hydrogène, fluor, chlore, alcoxy en $C_1$ à $C_6$ ou alcoyle en $C_1$ à $C_6$,
$R^1$: hydroxy, alcoxy en $C_1$ à $C_6$, acyloxy ou, si $R^2$ représente le groupe A défini ci-dessous, un hydrogène,
$R^2$: alcoyle en $C_1$ à $C_6$, phényle éventuellement substitué par un halogène, un alcoxy en $C_1$ à $C_6$ ou un trifluorométhyle, ou (si $R^1$ = hydrogène) un groupe A représentant

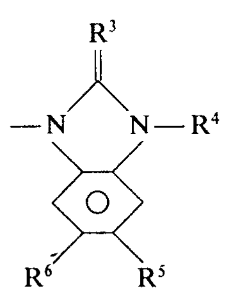

ou

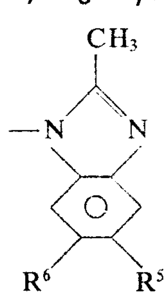

A

ou $R^3$ – $R^6$ ont les significations suivantes:

$R^3$: oxygène ou soufre,
$R^4$: hydrogène ou alcoyle en $C_1$ à $C_4$,
$R^5$ et $R^6$ indépendamment l'un de l'autre: hydrogène, méthyle, fluor, chlore, méthoxy, trifluorométhyle ou, ensemble, $-(CH_2)_4-$,

sous la forme du racémate ou des énantiomères, ainsi que des sels d'addition acides de ces composés, procédés caractérisés en ce que

1) pour préparer des composés de formule générale

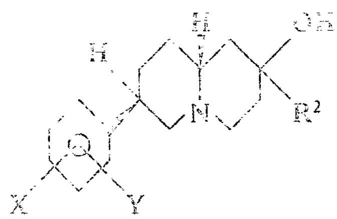

(II)

où X, Y et $R^2$ ont les mêmes significations que ci-dessus

a) on fait réagir une cétone de formule générale

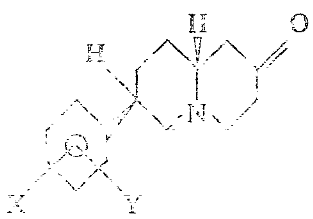

(V)

où X et Y ont les mêmes significations que ci-dessus,

avec un composé organo-métallique donneur de radical $R^2$ et le cas échéant on sépare le mélange de diastéréoisomères obtenu ou

b) sur la double liaison $\Delta^1$- ou $\Delta^2$ d'un composé de formule générale

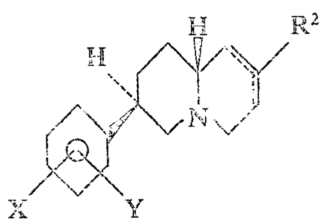

(VI)

où X, Y et $R^2$ ont les mêmes significations que ci-dessus et où la liaison pointillée représente une double liaison en position 1,2 ou en position 2,3,

on fixe de l'eau par réaction avec un agent hydratant,

ou

2) pour préparer des composés de formule générale

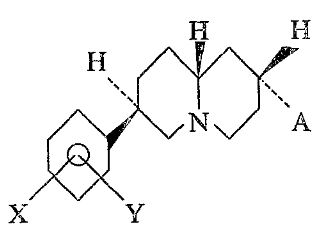

(III)

où X, Y, A et $R^3$–$R^6$ ont les mêmes significations que ci-dessus,

on fait réagir un composé de formule générale

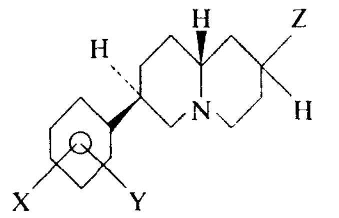

(VII)

où X et Y ont les mêmes significations que ci-dessus et où Z représente un groupe séparable,

avec un composé de formule générale

H — A (VIII)

où A a les mêmes significations que ci-dessus,

ou

3) pour préparer un composé de formule générale

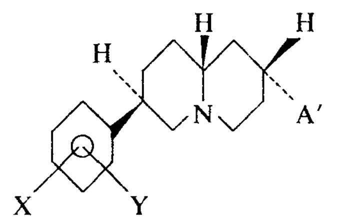

(III-3)

où X et Y ont les mêmes significations que ci-dessus et où A′ est semblable à A, où cependant $R^3$ représente un oxygène,

on hydrogène de façon catalytique un composé de formule générale

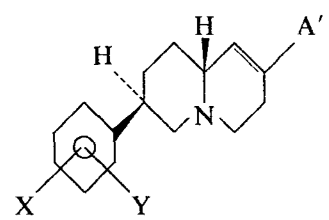

(IX)

où X, Y et A′ ont les mêmes significations que ci-dessus,

ou

4) pour préparer un composé de formule générale

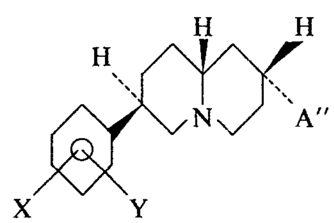

(III-4)

où X et Y ont les mêmes significations que ci-dessus et où A″ est semblable à A, où cependant $R^3$ représente un oxygène et $R^4$ un hydrogène,

on condense une phénylène-diamine de formule générale

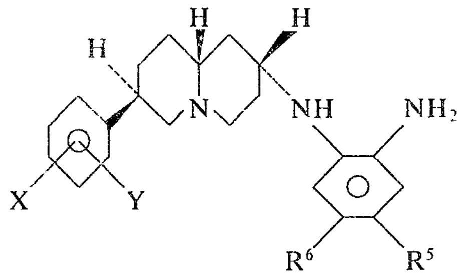

(X)

où X, Y, $R^5$ et $R^6$ ont les mêmes significations que ci-dessus,

avec un agent cyclisant de formule générale

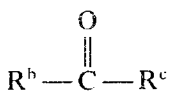

(XI)

où $R^b$ et $R^c$ représentent un halogène, un amino ou un l-imidazolyle,

ou avec de l'acide acétique ou un ester de l'acide orthoacétique,

ou

5) pour préparer des composés de formule générale

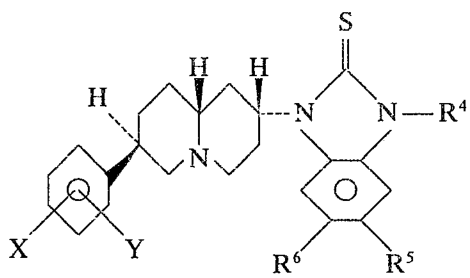

(III-5)

où X, Y, ainsi que $R^4$–$R^6$ ont les mêmes significations que ci-dessus,

a) on fait réagir un composé oxo correspondant de formule générale

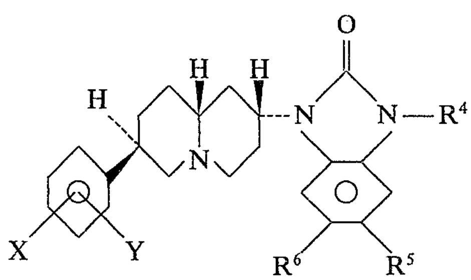

(III-6)

où X, Y ainsi que $R^4$–$R^6$ ont les mêmes significations que ci-dessus,

avec $P_2S_5$, ou

b) on fait réagir une phénylène-diamine de formule générale X ci-dessus avec du thiophosgène, du thiocarbonyldiimidazole ou $CS_2$ puis éventuellement on procède à une N-alcoylation,

ou

6) pour préparer des composés de formule générale

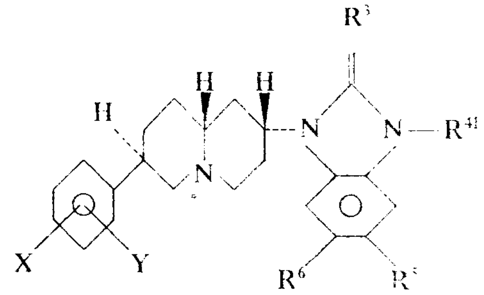

(III-7)

où X, Y, $R^3$, $R^5$ et $R^6$ ont les mêmes significations que ci-dessus et où $R^{4'}$ représente un alcoyle en $C_1$ à $C_6$,

on fait subir une N-alcoylation à un composé de formule générale

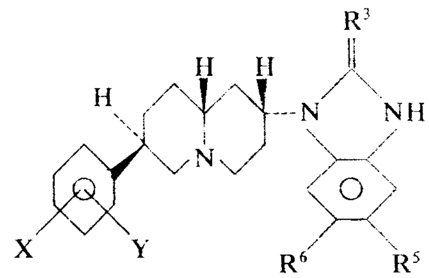

(III-8)

où X, Y, $R^3$, $R^5$ et $R^6$ ont les mêmes significations que ci-dessus

ou

7) pour préparer des éthers et des esters de formule générale

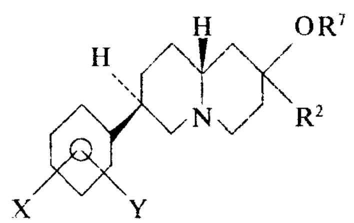

(IV)

où X, Y et $R^2$ ont les mêmes significations que ci-dessus et où $R^7$ représente un alcoyle en $C_1$ à $C_6$ ou un acyle,

on fait réagir un alcool de formule générale II ci-dessus avec un agent éthérifiant ou estérifiant donneur de radical $R^7$ et le cas échéant on dédouble les racémates obtenus, et éventuellement on transforme les bases obtenues en sels d'addition acide.

20. Médicaments, en particulier aux fins d'utilisation comme antibiotiques, antiémetiques et/ou analgésiques, contenant une phénylquinolizidine de formule générale I ci-dessus selon la revendication 1 sous la forme du racémate ou d'un énantiomère ou d'un sel d'addition acide d'un tel composé.

21. Composés de formule I donnée ci-dessus selon la revendication 1 sous la forme du racémate ou des énantiomères, ainsi que sels d'addition acide de tels composés aux fins d'application à la lutte contre les maladies, en particulier les psychoses, les névroses, les malaises ou la douleur.

**Revendications pour l'Etat contractant: AT**

1. Procédés de préparation de phénylquinolizidines de formule générale

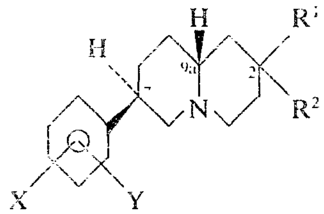

(I)

où X, Y, $R^1$ et $R^2$ ont les significations suivantes:

X: hydrogène, fluor, chlore, alcoxy en $C_1$ à $C_6$, alcoyle en $C_1$ à $C_6$ ou trifluorométhyle,
Y: hydrogène, fluor, chlore, alcoxy en $C_1$ à $C_6$ ou alcoyle en $C_1$ à $C_6$,
$R^1$: hydroxy, alcoxy en $C_1$ à $C_6$, acyloxy ou, si $R^2$ représente le groupe A défini ci-dessous, un hydrogène,
$R^2$: alcoyle en $C_1$ à $C_6$, phényle éventuellement substitué par un halogène, un alcoxy en $C_1$ à $C_6$ ou un trifluorométhyle, ou (si $R^1$ = hydrogène) un groupe A représentant

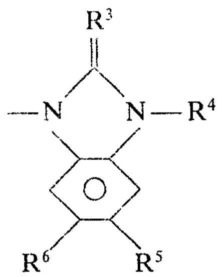

ou

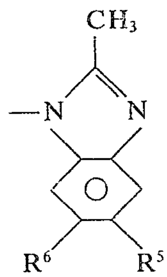

A

où $R^3 - R^6$ ont les significations suivantes:

$R^3$: oxygène ou soufre,
$R^4$: hydrogène ou alcoyle en $C_1$ à $C_4$,
$R^5$ et $R^6$ indépendamment l'un de l'autre: hydrogène, méthyle, fluor, chlore, méthoxy, trifluorométhyle ou, ensemble, $-(CH_2)_4-$,

sous la forme du racémate ou des énantiomères, ainsi que des sels d'addition acides de ces composés, procédés caractérisés en ce que

1) pour préparer des composés de formule générale

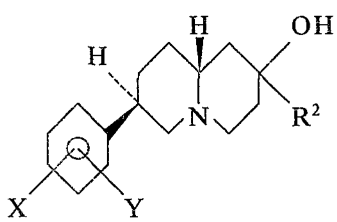

(II)

où X, Y et $R^2$ ont les mêmes significations que ci-dessus

a) on fait réagir une cétone de formule générale

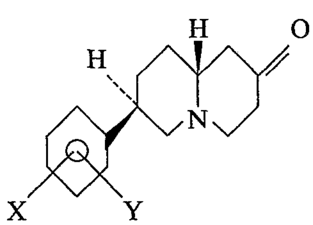

(V)

où X et Y ont les mêmes significations que ci-dessus,

avec un composé organo-métallique donneur de radical $R^2$ et le cas échéant on sépare le mélange de diastéréoisomères obtenu ou

b) sur la double liaison $\Delta^1$ ou $\Delta^2$ d'un composé de formule générale

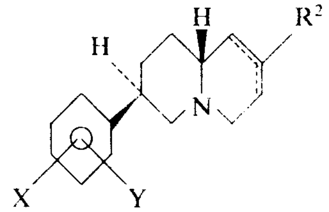

(VI)

où X, Y et $R^2$ ont les mêmes significations que ci-dessus et où la liaison pointillée représente une double liaison en position 1,2 ou en position 2,3,

on fixe de l'eau par réaction avec un agent hydratant,

ou

2) pour préparer des composées de formule générale

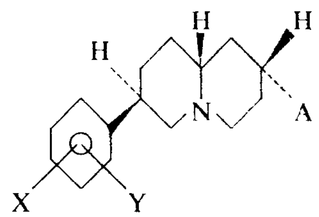

(III)

où X, Y, A et $R^3-R^6$ ont les mêmes significations que ci-dessus,

one fait réagir un composé de formule générale

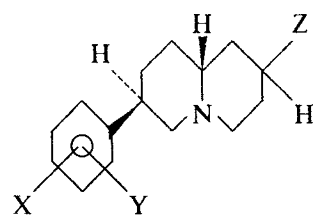

(VII)

où X et Y ont les mêmes significations que ci-dessus et où Z représente un groupe séparable,

avec un composé de formule générale

$$H—A \qquad (VIII)$$

où A a les mêmes significations que ci-dessus,

ou

3) pour préparer un composé de formule générale

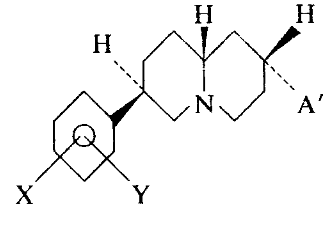

(III-3)

où X et Y ont les mêmes significations que ci-dessus et où A' est semblable à A, où cependant $R^3$ représente un oxygène,

on hydrogène de façon catalytique un composé de formule générale

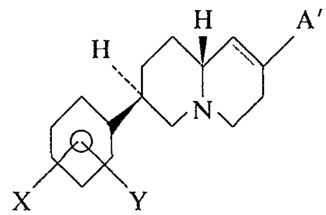

(IX)

où X, Y et A′ ont les mêmes significations que ci-dessus, ou

4. pour préparer un composé de formule générale

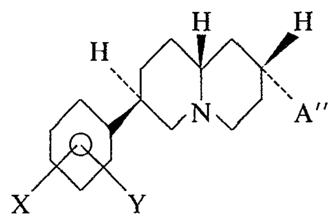

(III-4)

où X et Y ont les mêmes significations que ci-dessus et où A″ est semblable à A, où cependant $R^3$ représente un oxygène et $R^4$ un hydrogène,

on condense une phénylène-diamine de formule générale

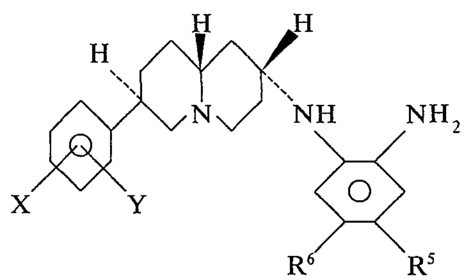

(X)

où X, Y, $R^5$ et $R^6$ ont les mêmes significations que ci-dessus,

avec un agent cyclisant de formule générale

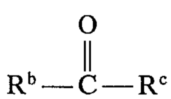

(XI)

où $R^b$ et $R^c$ représentent un halogène, un amino ou un l-imidazolyle,

ou avec de l'acide acétique ou un ester de l'acide orthoacétique, ou

5) pour préparer des composés de formule générale

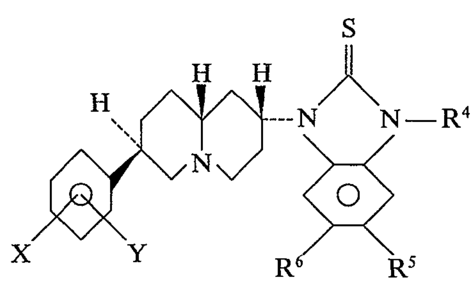

(III-5)

où X, Y, ainsi que $R^4$–$R^6$ ont les mêmes significations que ci-dessus,

a) on fait réagir un composé oxo correspondant de formule générale

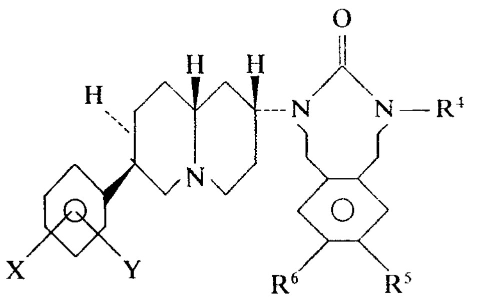

(III-6)

où X, Y ainsi que $R^4$–$R^6$ ont les mêmes significations que ci-dessus,

avec $P_2S_5$, ou

b) on fait réagir une phénylène-diamine de formule générale X ci-dessus avec du thiophosgène, du thiocarbonyldiimidazole ou $CS_2$ puis éventuellement on procède à une N-alcoylation,

ou

6) pour préparer des composés de formule générale

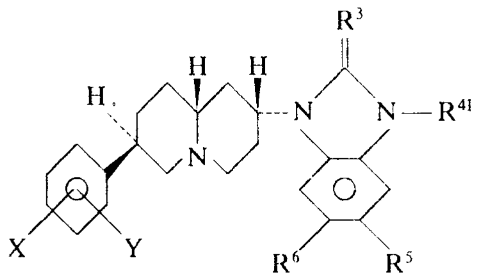

(III-7)

où X, Y, $R^3$, $R^5$ et $R^6$ ont les mêmes significations que ci-dessus et où $R^{41}$ représente un alcoyle en $C_1$ à $C_6$,

on fait subir une N-alcoylation à un composé de formule générale

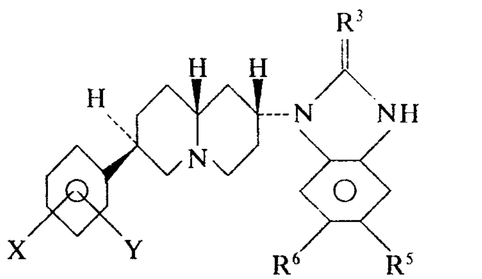

(III-8)

où X, Y, $R^3$, $R^5$ et $R^6$ ont les mêmes significations que ci-dessus,

ou

7) pour préparer des éthers et des esters de formule générale

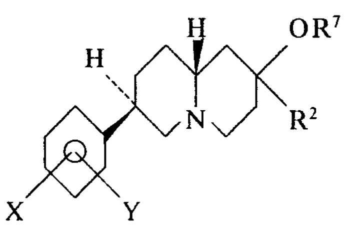

(IV)

où X, Y et $R^2$ ont les mêmes significations que ci-dessus et où $R^7$ représente un alcoyle en $C_1$ à $C_6$ ou un acyle,

on fait réagir un alcool de formule générale II ci-dessus avec un agent éthérifiant ou estérifiant donneur de radical $R^7$ et le cas échéant on dédouble les racémates obtenus, et éventuellement on transforme les bases obtenues en sels d'addition acide.

2. Procédés selon la revendication 1 de préparation de composés où $R^2$ représente un alcoyle en

$C_1$ à $C_6$, un phényle éventuellement substitué par un halogène, un alcoxy en $C_1$ à $C_6$ ou un trifluorométhyle, ou (si $R^1$ = hydrogène) un groupe A représentant

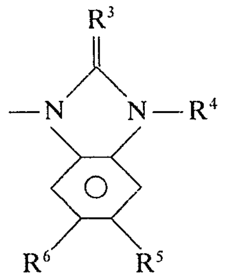

où $R^3$ et $R^4$ ont la même signification que dans la revendication 1, et où $R^5$ et $R^6$ représentent indépendamment l'un et l'autre un hydrogène, un méthyle, un chlore ou un méthoxy, caractérisés en ce que l'on utilise des composés de départ à substitution correspondante.

3. Procédés selon la revendication 1 de préparation de formule générale

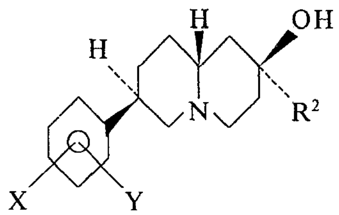

(II-1)

où X, Y et $R^2$ ont la même signification que dans la revendication 1,

sous la forme du racémate ou des énantiomères, ainsi que les sels d'addition acide de composés, caractérisés en ce que l'on utilise des composés de départ à substitution correspondante.

4. Procédés selon la revendication 3 de préparation de composés où $R^2$ représente un alcoyle en $C_3$ à $C_6$ ramifié, X un fluor ou un chlore et Y un hydrogène, un fluor ou un chlore, où X et Y sont en position ortho et/ou para, caractérisés en ce que l'on utilise des composés de départ à substitution correspondante.

5. Procédés selon la revendication 4 de préparation d'un composé où $R^2$ représente un tert-butyle, X un o-chloro et Y un p-chloro, caractérisés en ce que l'on utilise des composés de départ à substitution correspondante.

6. Procédés selon la revendication 3 de préparation d'un composé où $R^2$ représente un tert-butyle, X un p-trifluorométhyle et Y un hydrogène, caractérisés en ce que l'on utilise des composés de départ à substitution correspondante.

7. Procédés selon la revendication 1 de préparation de composés de formule générale

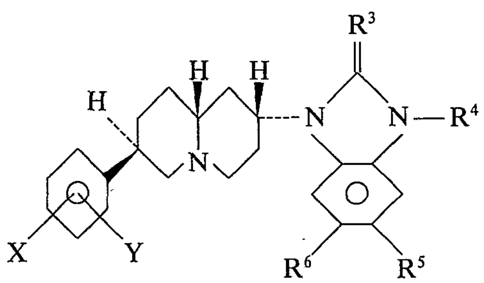

(III-1)

où X, Y, ainsi que $R^3 - R^6$ ont les mêmes signification que dans la revendication 1,

sous la forme du racémate ou des énantiomères, ainsi que les sels d'addition acide de tels composés, caractérisés en ce que l'on utilise des composés de départ à substitution correspondante.

8. Procédés selon la revendication 7 de préparation de composés où X représente un hydrogène, un o-fluoro ou un o-chloro, Y un hydrogène, $R^3$ un oxygène ou un soufre, $R^4$ un hydrogène, $R^5$ un fluor, un chlore ou un trifluorométhyle et $R^6$ un hydrogène, un chlore ou un fluor, caractérisés en ce que l'on utilise des composés de départ à substitution correspondante.

9. Procédés selon la revendication 8 de préparation d'un composé où X représente un p-chloro, Y un hydrogène, $R^3$ un oxygène, $R^4$ ainsi que $R^6$ un hydrogène et $R^5$ un trifluorométhyl, caractérisés en ce que l'on utilise des composés de départ à subtitution correspondante.

10. Procédés selon la revendication 7 de préparation d'un composé où X représente un o-fluoro, Y un

hydrogène, $R^3$ un oxygène, $R^4$ ainsi que $R^6$ un hydrogène et $R^5$ un chlore, caractérisés en ce que l'on utilise des composés de départ à substitution correspondante.

11. Procédés selon la revendication 7 de préparation d'un composé où X, Y et $R^3-R^6$ ont l'une des significations suivantes:

a) $X = o\text{-}Cl; Y, R^4, R^5, R^6 = H; R^3 = O$
b) $X = o\text{-}Cl; Y, R^4 = H; R^3 = O; R^5, R^6 = CH_3$
c) $X = o\text{-}Cl; Y, R^4 = H; R^3 = O; R^5, R^6 = Cl$
d) $X = o\text{-}Cl; Y, R^5, R^6 = H; R^3 = O; R^4 = CH_3$
f) $X = o\text{-}Cl; Y, R^4, R^6 = H; R^5 = Cl; R^3 = O$
g) $X = o\text{-}Cl; Y, R^4, R^6 = H; R^5 = Cl; R^3 = S$
h) $X = o\text{-}Cl; Y, R^4 = H; R^3 = S; R^5, R^6 = Cl$
i) $X = o\text{-}Cl; Y, R^4 = H; R^3 = O$; $R^5$ et $R^6$ ensemble $-(CH_2)_4-$
j) $X = o\text{-}F; Y, R^4 = H; R^3 = O; R^5, R^6 = CH_3$
k) $X = o\text{-}F; Y, R^4 = H; R^3 = O; R^5, R^6 = Cl$
l) $X = o\text{-}F; Y, R^4 = H; R^3 = S; R^5, R^6 = Cl$
m) $X = o\text{-}F; Y, R^4 = H; R^3 = O$; $R^5$ et $R^6$ ensemble $-(CH_2)_4-$
n) $X = o\text{-}F; Y, R^4, R^6 = H; R^5 = CF_3; R^3 = O$
o) $X = o\text{-}F; Y, R^4, R^6 = H; R^5 = CF_3; R^3 = S$
q) $X = o\text{-}F; Y, R^4, R^6 = H; R^5 = Cl; R^3 = S$,

caractérisés en ce que l'on utilise des composés de départ à substitution correspondante.

12. Procédés selon la revendication 7 de préparation de composés où $R^5$ et $R^6$ représentent indépendamment l'un de l'autre un hydrogène, un méthyle, un chlore ou un méthoxy, caractérisés en ce que l'on utilise des composés de départ à substitution correspondante.

13. Procédés selon la revendication 1 de préparation de composés de formule générale

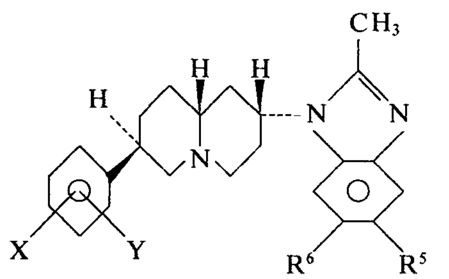

(III-2)

où X, Y, $R^5$ et $R^6$ ont les mêmes significations que dans la revendication 1,

sous la forme du racémate ou des énantiomères, ainsi que les sels d'addition acide de ces composés, caractérisés en ce que l'on utilise des composés de départ à substitution correspondante.

14. Procédés selon la revendication 1 de préparation de composés de formule générale

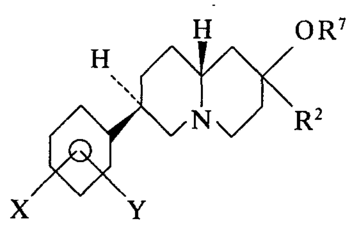

(IV)

où X, Y et $R^2$ ont les mêmes significations que dans la revendication 1 et où $R^7$ représente un alcoyle inférieur ou un acyle,

sous la forme du racémate ou des énantiomères, caractérisés en ce que l'on utilise des composés de départ à substitution correspondante.

15. Procédés selon la revendication 14 de préparation d'un composé où X et Y représentent un hydrogène, $OR^7$ un acétoxy orienté en $\beta$ et $R^2$ un n-butyle, caractérisés en ce que l'on utilise des composés de départ à substitution correspondante.

16. Procédés selon la revendication 14 de préparation d'un composé où X et Y représentent un hydrogène, $OR^7$ un trifluoracétoxy orienté en $\beta$, et $R^2$ un tert-butyle, caractérisés en ce que l'on utilise des composés de départ à substitution correspondante.

17. Procédés selon la revendication 14 de préparation d'un composé où X représente un o-chloro, $OR^7$ un acétoxy orienté en $\alpha$ et $R^2$ un phényle, caractérisés en ce que l'on utilise des composés de départ à substitution correspondante.